(19)

Europäisches Patentamt
European Patent Office
Office européen des brevets

(11)  **EP 4 089 680 B1**

(12)  **EUROPEAN PATENT SPECIFICATION**

(45) Date of publication and mention
of the grant of the patent:
**12.03.2025  Bulletin 2025/11**

(21) Application number: **21822840.1**

(22) Date of filing: **07.06.2021**

(51) International Patent Classification (IPC):
**G16C 60/00** (2019.01)   **C10G 9/00** (2006.01)
**C10G 11/18** (2006.01)   **C10G 35/24** (2006.01)
**C10G 45/72** (2006.01)   **C10G 47/36** (2006.01)
**G16C 20/10** (2019.01)   **G16C 20/30** (2019.01)
**G16C 20/70** (2019.01)

(52) Cooperative Patent Classification (CPC):
**C10G 11/187; C10G 9/005; C10G 35/24;
C10G 45/72; C10G 47/36; G16C 60/00;**
G16C 20/10; G16C 20/30; G16C 20/70

(86) International application number:
**PCT/CN2021/098570**

(87) International publication number:
**WO 2021/249329 (16.12.2021 Gazette 2021/50)**

(54) **METHOD, APPARATUS AND SYSTEM FOR REAL-TIME OPTIMIZATION OF MOLECULAR-LEVEL DEVICE, AND STORAGE MEDIUM**

VERFAHREN, VORRICHTUNG UND SYSTEM ZUR ECHTZEITOPTIMIERUNG EINER VORRICHTUNG AUF MOLEKULARNIVEAU UND SPEICHERMEDIUM

PROCÉDÉ, APPAREIL ET SYSTÈME POUR L'OPTIMISATION EN TEMPS RÉEL D'UN DISPOSITIF DE NIVEAU MOLÉCULAIRE ET SUPPORT D'ENREGISTREMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **12.06.2020  CN 202010533480**

(43) Date of publication of application:
**16.11.2022  Bulletin 2022/46**

(73) Proprietor: **PetroChina Company Limited
Dongcheng District
Beijing 100007 (CN)**

(72) Inventors:
• **WANG, Hangzhou
Beijing 100724 (CN)**
• **JI, Ye
Beijing 100724 (CN)**
• **LIU, Yixin
Beijing 100724 (CN)**
• **SUN, Baowen
Beijing 100724 (CN)**

• **SHI, Zhenmin
Beijing 100724 (CN)**

(74) Representative: **Slingsby Partners LLP
1 Kingsway
London WC2B 6AN (GB)**

(56) References cited:
**CN-A- 104 484 714     CN-A- 109 698 014
CN-A- 109 859 805     CN-A- 109 949 870
CN-A- 111 899 793     US-A1- 2014 262 957**

• **LI J. ET AL:** "Data-driven mathematical modeling and global optimization framework for entire petrochemical planning operations", AICHE JOURNAL, JOHN WILEY & SONS, INC, US, vol. 62, no. 9, 27 March 2016 (2016-03-27), pages 3020 - 3040, XP071061553, ISSN: 0001-1541, DOI: 10.1002/AIC.15220

- KHOR C. S. ET AL: "Petroleum refinery optimization", OPTIMIZATION AND ENGINEERING, SPRINGER NEW YORK LLC, US, vol. 18, no. 4, 3 November 2016 (2016-11-03), pages 943 - 989, XP036352192, ISSN: 1389-4420, [retrieved on 20161103], DOI: 10.1007/S11081-016-9338-X
- GUEDDAR T. ET AL: "Disaggregationaggregation based model reduction for refinery-wide optimization", COMPUTERS & CHEMICAL ENGINEERING, PERGAMON PRESS, OXFORD, GB, vol. 35, no. 9, 28 April 2011 (2011-04-28), pages 1838 - 1856, XP028245865, ISSN: 0098-1354, [retrieved on 20110505], DOI: 10.1016/J.COMPCHEMENG.2011.04.016
- CHERNYSHEVA E. A. ET AL: "Enhancing the Efficiency of Refinery Crude Oil Distillation Process by Optimized Preliminary Feedstock Blending (Review)", PETROLEUM CHEMISTRY, PLEIADES PUBLISHING, MOSCOW, vol. 60, no. 1, 1 January 2020 (2020-01-01), pages 1 - 15, XP037025633, ISSN: 0965-5441, [retrieved on 20200218], DOI: 10.1134/S0965544120010053
- REN Y. ET AL: "Molecular reconstruction: Recent progress toward composition modeling of petroleum fractions", CHEMICAL ENGENEERING JOURNAL, vol. 357, 11 September 2018 (2018-09-11), AMSTERDAM, NL, pages 761 - 775, XP055776630, ISSN: 1385-8947, DOI: 10.1016/j.cej.2018.09.083
- JOO E. ET AL: "CRACKER - a PC based simulator for industrial cracking furnaces", COMPUTERS & CHEMICAL ENGINEERING, PERGAMON PRESS, OXFORD, GB, vol. 24, no. 2-7, 15 July 2000 (2000-07-15), pages 1523 - 1528, XP026981867, ISSN: 0098-1354, [retrieved on 20000715]

# EP 4 089 680 B1

**Description**

<u>Technical Field</u>

The present disclosure relates to the technical field of petroleum processing and, in particularly to a method, an apparatus and a system for real-time optimization of a molecular-level device, and a storage medium.

<u>Background</u>

In the field of petroleum processing, it is necessary to maximize the utilization of the raw material in the production and processing flow in order to achieve efficient conversion of the raw material of the production and processing devices into high value-added products during the working process of the existing production and processing devices.

To achieve maximize the utilization of the raw material in the processing flow, it is necessary to optimize the production and processing process of the production and processing devices in real time; however, due to the complexity of the production and processing process of the production and processing devices, making the optimization relatively difficult, it is difficult to produce a cost-effective product. Li J. et al. (2016) "Data-driven mathematical modeling and global optimization framework for entire petrochemical planning operations", AICHE JOURNAL, vol. 62, no. 9, pages 3020-3040 discloses a modeling and global optimization-based planning formulation, which predicts product yields and properties for all of the production units within a highly integrated refinery-petrochemical complex. Khor C. S. et al. (2016) "Petroleum refinery optimization", OPTIMIZATION AND ENGINEERING, vol. 18, no. 4, pages 943-989 is a review article on the role of optimization methods in refineries for wide-ranging multiscale applications and activities including the traditional planning linear programming. Gueddart T. et al. (2011) "Disaggregatio-naggregation based model reduction for refinery-wide optimization", COMPUTERS & CHEMICAL ENGINEERING, vol. 35, no. 9, pages 1838-1856 describes artificial neural network-based reduced nonlinear refinery optimization models developed by generating and using input-output data from a process simulator. Chernysheva E. A. et al. (2020) "Enhancing the Efficiency of Refinery Crude Oil Distillation Process by Optimized Preliminary Feedstock Blending (Review)", PETROLEUM CHEMISTRY, vol. 60, no. 1, pages 1-15 is a review article on the optimization of crude oil refining processes through optimal blending of components. Ren Y. et al. (2018) "Molecular reconstruction: Recent progress toward composition modeling of petroleum fractions", CHEMICAL ENGENEERING JOURNAL, vol. 357, pages 761-775 is a review article on molecular reconstruction models used in oil refinery modeling.

In view of this, how to optimize the production and processing process of the production and processing device has become one of problems to be solved urgently by those skilled in the art.

<u>Summary</u>

In order to solve the aforementioned technical problems or at least partially solve the aforementioned technical problems, the present disclosure provides a method, an apparatus and a system for real-time optimization of a molecular level device, and a storage medium.

In view of this, according to a first aspect, the present disclosure provides a method for the real-time optimization of a molecular level device, the method comprising the following steps:

acquiring molecular composition of crude oil;
acquiring molecular composition of various fractions obtained by distillation of the crude oil according to physical properties of various single molecules in the molecular composition of the crude oil;
respectively inputting, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition;
acquiring a preset standard set for a preset target product;
determining whether the predicted product meets a preset standard for a target product corresponding to the predicted product in the preset standard set according to the predicted molecular composition of the predicted product and the predicted molecular content of each single molecule in the predicted molecular composition; and
if the predicted product does not meet any preset standard for a target product corresponding to the predicted product in the preset standard set, adjusting an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set.

The method preferably further includes:

acquiring an input flow of petroleum processing feedstocks input to each of the petroleum processing devices;

determining whether each of the input flows meets a preset input flow range of the respective petroleum processing device; and

adjusting the preset feedstock ratio if any one of the input flows does not meet the preset input flow range of the respective petroleum processing device, and respectively re-inputting, according to the adjusted preset feedstock ratio, the corresponding fractions into the pre-trained product prediction model of the respective petroleum processing device as petroleum processing feedstocks, until each of the input flows meets the preset input flow range of the respective petroleum processing device.

In the preferred embodiment, if each of the input flows meets the preset input flow range of the respective petroleum processing device, it is believed that a subsequent step may be carried out, to perform the step of obtaining predicted molecular composition of a corresponding predicted product and predicted molecular content of each single molecule in the predicted molecular composition.

The determining whether the predicted product meets a preset standard for a target product corresponding to the predicted product in the preset standard set preferably includes:

calculating a physical property of each single molecule in the predicted molecular composition according to the predicted molecular composition of the predicted product and the predicted molecular content of each single molecule in the predicted molecular composition;

calculating a predicted physical property of the predicted product according to the physical property of each single molecule in the predicted molecular composition and the predicted molecular content of each single molecule in the predicted molecular composition; and

determining whether the predicted physical property of each of the predicted products meets a preset physical property restriction interval of the corresponding target product in the preset standard set.

In the preferred embodiment, if the predicted physical property of each of the predicted products meets the preset physical property restriction interval, it is believed that a subsequent step may be carried out, to determine that the predicted product meets the preset standard for the target product corresponding to the predicted product in the preset standard set, and to perform the step of obtaining predicted molecular composition of a corresponding predicted product and predicted molecular content of each single molecule in the predicted molecular composition.

The method preferably further includes:

blending each of the predicted products which is used as a product blending feedstock according to a preset rule set, to obtain molecular composition of a plurality of mixed products and content of each single molecule in each of the mixed products; and

respectively calculating product property of each of the mixed products according to the molecular composition of each of the mixed products and the content of each single molecule in each of the mixed products.

The determining whether the predicted product meets a preset standard for a target product corresponding to the predicted product in the preset standard set preferably includes:

determining whether the product property of each of the mixed products meets a preset product property of a target mixed product obtained by blending corresponding each target product in the preset standard set;

if the preset product property is met, obtaining a target parameter according to all of the mixed products and determining whether the target parameter meets a preset condition;

if the target parameter meets the preset condition, determining that the predicted product meets a preset standard for a target product corresponding to the predicted product in the preset standard set, and outputting the preset feedstock ratio, the pre-trained product prediction model and the preset rule set as a production and processing scheme; and

if the target parameter does not meet the preset condition, adjusting the operation parameter in the pre-trained product prediction model and a preset rule in the preset rule set, to re-obtain a plurality of mixed products, until the product property of each of the mixed products meets the preset product property and the target parameters in all of the mixed products meet the preset condition.

The obtaining a target parameter according to all of the mixed products and determining whether the target parameter meets a preset condition preferably includes:

acquiring a product price of each of mixed products and a yield of each of mixed products;

calculating a product benefit of each of mixed products according to the yield of each of mixed products and the product price of each of mixed products;

accumulating the product benefit of each of mixed products to obtain a cumulative benefit;

acquiring a feedstock price of each group of the petroleum processing feedstocks and an operating cost of each of the petroleum processing devices;

subtracting feedstock prices of all of the petroleum processing feedstocks and operating costs of all of the petroleum processing devices from the cumulative benefit to obtain a comprehensive benefit;

serving the comprehensive benefit as the target parameter;

determining whether the comprehensive benefit reaches a maximum value;

determining that the target parameter meets the preset condition if the comprehensive benefit reaches the maximum value; and

determining that the target parameter does not meet the preset condition if the comprehensive benefit does not reach the maximum value.

The operation parameter includes a temperature of an environment where a reaction path in the pre-trained product prediction model is located, and the adjusting an operation parameter in the pre-trained product prediction model preferably includes:

adjusting a temperature of an environment where a reaction path corresponding to the predicted product in the pre-trained product prediction model is located; and

re-obtaining the predicted molecular composition of the predicted product and the predicted molecular content of each single molecule in each of the predicted products according to the adjusted temperature until the predicted product meets the preset standard f the target product corresponding to the predicted product in the preset standard set.

The operation parameter includes a pressure of an environment where a reaction path in the pre-trained product prediction model is located, and the adjusting an operation parameter in the pre-trained product prediction model preferably includes:

adjusting a pressure of an environment where a reaction path corresponding to the predicted product in the pre-trained product prediction model is located; and

re-obtaining the predicted molecular composition of the predicted product and the predicted molecular content of each single molecule in each of the predicted products according to the adjusted pressure until the predicted product meets the preset standard for the target product corresponding to the predicted product in the preset standard set.

The respectively calculating product property of each of the mixed products according to the molecular composition of each of the mixed products and the content of each single molecule in each of the mixed products preferably includes:

acquiring first molecular composition of each group of the product blending feedstocks and first component content of each single molecule in each group of the product blending feedstocks;

based on the preset rule set, obtaining second molecular composition of each of mixed products and second component content of each single molecule in each of mixed products according to the first molecular composition of each group of the product blending feedstocks and the first component content of each single molecule in each group of the product blending feedstocks;

calculating a physical property of each single molecule in each of the mixed products according to the number of groups of each group contained in each single molecule in each of the mixed products and a contribution value of each group to the physical property; and

calculating a product property of each of the mixed products according to the physical property and the second component content of each single molecule in each of the mixed products.

The calculating a physical property of each single molecule preferably includes:

for each single molecule, acquiring the number of groups of each group constituting the single molecule and a contribution value of each group to the physical property; and

inputting the number of groups of each group constituting the single molecule and the contribution value of each group to the physical property into a pre-trained property calculation model, to acquire the physical property of the single molecule outputted by the pre-trained property calculation model; wherein,

the pre-trained property calculation model is used to calculate the physical property of the single molecule according to the number of groups of each group contained in single molecule and a contribution value of each group to the physical property.

Before the inputting the number of groups of each group constituting the single molecule and the contribution value of each group to the physical property into a pre-trained property calculation model, the method preferably further includes:

comparing the number of groups of each group constituting the single molecule with molecular information of a template single molecule with known physical properties pre-stored in a database, the molecular information comprising the number of groups of each group constituting the template single molecule;
determining whether there is a same template single molecule as the single molecule;
if there is a same template single molecule as the single molecule, outputting the physical properties of the template single molecule as a physical property of the single molecule; and
if there is not a same template single molecule as the single molecule, then performing the step of the inputting the number of groups of each group constituting the single molecule and the contribution value of each group to the physical property into a pre-trained property calculation model.

A of training the property calculation model preferably includes:

constructing a property calculation model of a single molecule;
acquiring the number of groups of each group constituting a sample single molecule; wherein the physical property of the sample single molecule is known;
inputting the number of groups of each group constituting the sample single molecule into the property calculation model;
acquiring a predicted physical property of the sample single molecule output by the property calculation model;
if a deviation value between the predicted physical property and the physical property which is known is less than a preset deviation threshold, determining that the property calculation model converges, acquiring a contribution value of each group to the physical property in the property calculation model which is converged, and storing the contribution value of the group to the physical property; and
if the deviation value between the predicted physical property and the physical property which is known is greater than or equal to the deviation threshold, adjusting a contribution value of each group to the physical property in the property calculation model until the property calculation model converges.

The acquiring the number of groups of each group constituting a sample single molecule preferably includes:

determining a primary group, the number of groups of the primary group, a multi-stage group, and the number of groups of the multi-stage group in all groups of the single molecule;
taking all groups constituting the single molecule as the primary group; and
taking various groups which coexist and contribute to a same physical property in common as the multi-stage group, and taking the number of the various groups as a level of the multi-stage group.

The pre-trained property calculation model preferably determines the physical property of the single molecule in the following manner:

obtaining the product of the number of groups of various groups and the contribution values of the various groups to the physical property; and
obtaining the physical property of the single molecule according to the sum of the products of the number of groups of various groups and the contribution values of the various groups to the physical property.

For example, the pre-trained property calculation model is as follows:

$$f = a + \sum n_i \Delta f_i \, ,$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.
The acquiring the number of groups of each group constituting a single molecule includes:

determining a primary group, the number of groups of the primary group, a multi-stage group, and the number of groups of the multi-stage group in all groups of the single molecule;

taking all groups constituting the single molecule as the primary group; and

taking various groups which coexist and contribute to same physical property in common as the multi-stage group, and taking the number of the various groups as a level of the multi-stage group.

The pre-trained property calculation model determines the physical property of the single molecule in the following manner:

for each level of groups, obtaining the product of the number of groups of various groups contained therein and the contribution values of the various groups to the physical property respectively, and then obtaining the sum of the products of the number of groups of various groups contained therein and the contribution values of the various groups to the physical property as a contribution value of this level of groups to physical property; and

obtaining the physical property of the single molecule according to the sum of the contribution values of the groups at all levels to the physical property.

For example, the pre-trained property calculation model is as follows:

$$f = a + \sum_i \mathrm{m}_{1i}\Delta f_{1i} + \sum_j m_{2j}\Delta f_{2j}\ldots\ldots + \sum_l m_{Nl}\Delta f_{Nl}$$

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

The physical property of the single molecule preferably includes a boiling point of a single molecule;

the calculating the physical property of the single molecule includes:

calculating the boiling point of the single molecule according to a property calculation model as follows:

$$T = \frac{SOL \times GROUP_{11} + SOL \times GROUP_{12} + \ldots\ldots + SOL \times GROUP_{1N}}{(SOL \times \mathrm{Numh})^d + b} + c$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

The physical property of the single molecule preferably includes a density of a single molecule;

The pre-trained property calculation model preferably determines the density of the single molecule in the following manner:

obtaining a single molecule vector by converting the number of groups of each group constituting the single molecule;

obtaining, by converting the contribution value of each level group to the density, the contribution value vector of this level group;

obtaining the product of the single molecule vector and the contribution value vector of the groups at all levels respectively, and then obtaining the sum of the products of the single molecule vector and the contribution value vector of the groups at all levels; and

obtaining the density of the single molecule according to the ratio of the product of the single molecule vector and the contribution value vector of the primary group to the sum of the products of the single molecule vector and the contribution value vector of the groups at all levels.

For example, calculating the density of the single molecule according to a property calculation model as follows:

$$D = \frac{SOL \times GROUP_{21}}{(SOL \times GROUP_{22} + \ldots\ldots + SOL \times GROUP_{2N}) \times e} \; ;$$

where, $D$ is the density of the single molecule, $SOL$ is a single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

The physical property of the single molecule preferably includes an octane number of a single molecule;

The pre-trained property calculation model preferably determines the octane number of the single molecule in the following manner:

obtaining a single molecule vector by converting the number of groups of each group constituting the single molecule;
obtaining, by converting the contribution value of each level group to the octane number, the contribution value vector of this level group;
obtaining the product of the single molecule vector and the contribution value vector of the groups at all levels respectively; and
obtaining the octane number of the single molecule according to the sum of the products of the single molecule vector and the contribution value vector of the groups at all levels.

For example, calculating the octane number of the single molecule according to a property calculation model as follows:

$$X = SOL \times GROUP_{31} + SOL \times GROUP_{32} + \ldots\ldots + SOL \times GROUP_{3N} + h \; ;$$

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUP_{32}$ is a 2N+2-th contribution value vector converted according to a contribution value of the secondary group to the octane number, $GROUP_{3N}$ is a 3N-ty contribution value vector converted according to a contribution value of the N-stage group to the octane number; N is a positive integer greater than or equal to 2; and $h$ is the fifth preset constant.

The product property of the mixed products includes a density, a cloud point, a pour point, an aniline point, and an octane number.

When a product property of the mixed product is the density, calculating the product property of each of the mixed products preferably includes:
calculating the density of each of the mixed products in the following manner:

for each mixed product, obtaining the product of the density of each single molecule in the mixed product and the content of the single molecule; and
obtaining the density of the mixed product according to the sum of the products of the density of each single molecule in the mixed product and the content of the single molecule.

For example, calculating the density of each of the mixed products according to a calculation formula as follows:
$density = \Sigma (D_i \times x_{i\_volume})$;
where, $density$ is the density of the mixed product, $D_i$ is the density of the $i$-th single molecule, and $x_{i\_volume}$ is second component content of the $i$-th single molecule.

When a product property of the mixed product is the cloud point, calculating the product property of each of the mixed products preferably includes:

for each mixed product, calculating a cloud point contribution value of each single molecule according to the density and the boiling point of each single molecule in the mixed product; and
calculating the cloud point of the mixed product according to cloud point contribution values of all of the single molecules and content of each single molecule in the mixed product.

When a product property of the mixed product is the pour point, calculating the product property of each of the mixed products preferably includes:

for each mixed product, calculating a pour point contribution value of each single molecule according to the density and molecular weight of each single molecule in the mixed product; and

calculating the pour point of the mixed product according to pour point contribution values of all of the single molecules and content of each single molecule in the mixed product.

When a product property of the mixed product is the aniline point, calculating the product property of each of the mixed products preferably includes:

for each mixed product, calculating an aniline point contribution value of each single molecule according to the density and the boiling point of each single molecule in the mixed product; and

calculating the aniline point of the mixed product according to aniline point contribution values of all of the single molecules and content of each single molecule in the mixed product.

When a product property of the mixed product is the octane number, calculating the product property of each of the mixed products preferably includes:

for each mixed product, acquiring the octane number of each single molecule and content of each single molecule in the mixed product; and

calculating the octane number of the mixed products according to acalculation formula as follows:

$$
\begin{aligned}
\mathrm{ON} = ( & \sum_{i=\mathrm{HISQFG}} \upsilon_i \beta_i \mathrm{ON}_i + \mathrm{C_H} \sum_{i=\mathrm{H}} \upsilon_i \beta_i \mathrm{ON}_i + \mathrm{C_I} \sum_{i=\mathrm{I}} \upsilon_i \beta_i \mathrm{ON}_i + \mathrm{C_S} \sum_{i=\mathrm{S}} \upsilon_i \beta_i \mathrm{ON}_i \\
& + \mathrm{C_Q} \sum_{i=\mathrm{Q}} \upsilon_i \beta_i \mathrm{ON}_i + \mathrm{C_F} \sum_{i=\mathrm{F}} \upsilon_i \beta_i \mathrm{ON}_i + \mathrm{C_G} \sum_{i=\mathrm{G}} \upsilon_i \beta_i \mathrm{ON}_i ) \div \\
( & \sum_{i=\mathrm{HISQFG}} \upsilon_i \beta_i + \mathrm{C_H} (\sum_{i=\mathrm{H}} \upsilon_i \beta_i - \sum_{i=\mathrm{H}} \upsilon_i) + \mathrm{C_I} (\sum_{i=\mathrm{I}} \upsilon_i \beta_i - \sum_{i=\mathrm{I}} \upsilon_i) + \mathrm{C_S} (\sum_{i=\mathrm{S}} \upsilon_i \beta_i - \sum_{i=\mathrm{S}} \upsilon_i) \\
& + \mathrm{C_Q} (\sum_{i=\mathrm{Q}} \upsilon_i \beta_i - \sum_{i=\mathrm{Q}} \upsilon_i) + \mathrm{C_F} (\sum_{i=\mathrm{F}} \upsilon_i \beta_i - \sum_{i=\mathrm{F}} \upsilon_i) + \mathrm{C_G} (\sum_{i=\mathrm{G}} \upsilon_i \beta_i - \sum_{i=\mathrm{G}} \upsilon_i))
\end{aligned} ;
$$

$$
\mathrm{C_H} = \frac{k_{\mathrm{HI}}^{(a)} \upsilon_\mathrm{I} + k_{\mathrm{HS}}^{(a)} \upsilon_\mathrm{S} + k_{\mathrm{HQ}}^{(a)} \upsilon_\mathrm{Q} + k_{\mathrm{HF}}^{(a)} \upsilon_\mathrm{F} + k_{\mathrm{HG}}^{(a)} \upsilon_\mathrm{G}}{1 + k_{\mathrm{HI}}^{(b)} \upsilon_\mathrm{I} + k_{\mathrm{HS}}^{(b)} \upsilon_\mathrm{S} + k_{\mathrm{HQ}}^{(b)} \upsilon_\mathrm{Q} + k_{\mathrm{HF}}^{(b)} \upsilon_\mathrm{F} + k_{\mathrm{HG}}^{(b)} \upsilon_\mathrm{G}} ;
$$

$$
\mathrm{C_I} = \frac{k_{\mathrm{HI}}^{(a)} \upsilon_\mathrm{H} + k_{\mathrm{IS}}^{(a)} \upsilon_\mathrm{S} + k_{\mathrm{IQ}}^{(a)} \upsilon_\mathrm{Q} + k_{\mathrm{IF}}^{(a)} \upsilon_\mathrm{F} + k_{\mathrm{IG}}^{(a)} \upsilon_\mathrm{G}}{1 + k_{\mathrm{HI}}^{(b)} \upsilon_\mathrm{H} + k_{\mathrm{IS}}^{(b)} \upsilon_\mathrm{S} + k_{\mathrm{IQ}}^{(b)} \upsilon_\mathrm{Q} + k_{\mathrm{IF}}^{(b)} \upsilon_\mathrm{F} + k_{\mathrm{IG}}^{(b)} \upsilon_\mathrm{G}} ;
$$

$$
\mathrm{C_S} = \frac{k_{\mathrm{HS}}^{(a)} \upsilon_\mathrm{H} + k_{\mathrm{IS}}^{(a)} \upsilon_\mathrm{I} + k_{\mathit{SQ}}^{(a)} \upsilon_\mathrm{Q} + k_{\mathit{SF}}^{(a)} \upsilon_\mathrm{F} + k_{\mathit{SG}}^{(a)} \upsilon_\mathrm{G}}{1 + k_{\mathrm{HS}}^{(b)} \upsilon_\mathrm{H} + k_{\mathrm{IS}}^{(b)} \upsilon_\mathrm{I} + k_{\mathit{SQ}}^{(b)} \upsilon_\mathrm{Q} + k_{\mathrm{SF}}^{(b)} \upsilon_\mathrm{F} + k_{\mathrm{SG}}^{(b)} \upsilon_\mathrm{G}} ;
$$

$$
\mathrm{C_Q} = \frac{k_{\mathit{HQ}}^{(a)} \upsilon_\mathrm{H} + k_{\mathrm{IQ}}^{(a)} \upsilon_\mathrm{I} + k_{\mathit{SQ}}^{(a)} \upsilon_\mathrm{S} + k_{\mathrm{QF}}^{(a)} \upsilon_\mathrm{F} + k_{\mathrm{QG}}^{(a)} \upsilon_\mathrm{G}}{1 + k_{\mathrm{HQ}}^{(b)} \upsilon_\mathrm{H} + k_{\mathrm{QI}}^{(b)} \upsilon_\mathrm{I} + k_{\mathrm{SQ}}^{(b)} \upsilon_\mathrm{S} + k_{\mathrm{QF}}^{(b)} \upsilon_\mathrm{F} + k_{\mathrm{QG}}^{(b)} \upsilon_\mathrm{G}} ;
$$

$$
\mathrm{C_F} = \frac{k_{\mathrm{HF}}^{(a)} \upsilon_\mathrm{H} + k_{\mathrm{IF}}^{(a)} \upsilon_\mathrm{I} + k_{\mathrm{SF}}^{(a)} \upsilon_\mathrm{S} + k_{\mathrm{QF}}^{(a)} \upsilon_\mathit{Q} + k_{\mathrm{FG}}^{(a)} \upsilon_\mathrm{G}}{1 + k_{\mathrm{HF}}^{(b)} \upsilon_\mathrm{H} + k_{\mathrm{IF}}^{(b)} \upsilon_\mathrm{I} + k_{\mathrm{SF}}^{(b)} \upsilon_\mathrm{S} + k_{\mathrm{QF}}^{(b)} \upsilon_\mathit{Q} + k_{\mathrm{FG}}^{(b)} \upsilon_\mathrm{G}} ;
$$

$$C_{\mathrm{G}} = \frac{k_{\mathrm{HG}}^{(a)}\upsilon_{\mathrm{H}} + k_{\mathrm{IG}}^{(a)}\upsilon_{\mathrm{I}} + k_{\mathrm{SG}}^{(a)}\upsilon_{\mathrm{S}} + k_{\mathrm{QG}}^{(a)}\upsilon_{\mathrm{Q}} + k_{\mathrm{FG}}^{(a)}\upsilon_{\mathrm{F}}}{1 + k_{HG}^{(b)}\upsilon_{\mathrm{H}} + k_{\mathrm{IG}}^{(b)}\upsilon_{\mathrm{I}} + k_{\mathrm{SG}}^{(b)}\upsilon_{\mathrm{S}} + k_{\mathrm{QG}}^{(b)}\upsilon_{\mathrm{Q}} + k_{FG}^{(b)}\upsilon_{\mathrm{F}}};$$

where, ON is the octane number of the mixed product, HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $\upsilon_i$ is content of each molecule in the mixed product, $\upsilon_{\mathrm{H}}, \upsilon_{\mathrm{I}}, \upsilon_{\mathrm{S}}, \upsilon_{\mathrm{Q}}, \upsilon_{\mathrm{F}}$ and $\upsilon_{\mathrm{G}}$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_{\mathrm{H}}$ is an interaction coefficient of n-alkanes with other molecules, $C_{\mathrm{I}}$ is an interaction coefficient of isoalkanes with other molecules; $C_{\mathrm{S}}$ is an interaction coefficient of cycloalkanes with other molecules; $C_{\mathrm{Q}}$ is an interaction coefficient of olefins with other molecules, $C_{\mathrm{F}}$ is an interaction coefficient of aromatic hydrocarbons with other molecules, $C_{\mathrm{G}}$ is an interaction coefficient of oxygenated compounds with other molecules, $k_{\mathrm{HI}}^{(a)}$ is a first constant coefficient between n-alkanes and isoalkanes, $k_{\mathrm{HS}}^{(a)}$ is a first constant coefficient between n-alkanes and cycloalkanes, $k_{\mathrm{HQ}}^{(a)}$ is a first constant coefficient between n-alkanes and olefins, $k_{\mathrm{HF}}^{(a)}$ is a first constant coefficient between n-alkanes and aromatic hydrocarbons, $k_{HG}^{(a)}$ is a first constant coefficient between n-alkanes and oxygenated compounds, $k_{\mathrm{IS}}^{(a)}$ is a first constant coefficient between isoalkanes and cycloalkanes, $k_{\mathrm{IQ}}^{(a)}$ is a first constant coefficient between isoalkanes and olefins, $k_{\mathrm{IF}}^{(a)}$ is a first constant coefficient between isoalkanes and aromatic hydrocarbons, $k_{\mathrm{IG}}^{(a)}$ is a first constant coefficient between isoalkanes and oxygenated compounds, $k_{\mathrm{SQ}}^{(a)}$ is a first constant coefficient between cycloalkanes and olefins, $k_{\mathrm{SF}}^{(a)}$ is a first constant coefficient between cycloalkanes and aromatic hydrocarbons, $k_{\mathrm{SG}}^{(a)}$ is a first constant coefficient between cycloalkanes and oxygenated compounds, $k_{\mathrm{QF}}^{(a)}$ is a first constant coefficient between olefins and aromatic hydrocarbons, $k_{\mathrm{QG}}^{(a)}$ is a second constant coefficient between olefins and oxygenated compounds, $k_{\mathrm{FG}}^{(a)}$ is a first constant coefficient between aromatic hydrocarbons and oxygenated compounds, $k_{\mathrm{HI}}^{(b)}$ is a second constant coefficient between n-alkanes and isoalkanes, $k_{\mathrm{HS}}^{(b)}$ is a second constant coefficient between n-alkanes and cycloalkanes, $k_{\mathrm{HQ}}^{(b)}$ is a second constant coefficient between n-alkanes and olefins, $k_{\mathrm{HF}}^{(b)}$ is a second constant coefficient between n-alkanes and aromatic hydrocarbons, $k_{\mathrm{HG}}^{(b)}$ is a second constant coefficient between n-alkanes and oxygenated compounds, $k_{\mathrm{IS}}^{(b)}$ is a second constant coefficient between isoalkanes and cycloalkanes, $k_{\mathrm{IQ}}^{(b)}$ is a second constant coefficient between isoalkanes and olefins, $k_{\mathrm{IF}}^{(b)}$ is a second constant coefficient between isoalkanes and aromatic hydrocarbons, $k_{\mathrm{IG}}^{(b)}$ is a second constant coefficient between isoalkanes and oxygenated compounds, $k_{\mathrm{SQ}}^{(b)}$ is a second constant coefficient between cycloalkanes and olefins, $k_{\mathrm{SF}}^{(b)}$ is a second constant coefficient between cycloalkanes and aromatic hydrocarbons, $k_{\mathrm{SG}}^{(b)}$ is a second constant coefficient between cycloalkanes and oxygenated compound, $k_{\mathrm{QF}}^{(b)}$ is a second constant coefficient between olefins and aromatic hydrocarbons, $k_{\mathrm{QG}}^{(b)}$ is a second constant coefficient between olefins and oxygenated compound, and $k_{\mathrm{FG}}^{(b)}$ is a second constant coefficient between aromatic hydrocarbons and oxygenated compound; wherein the octane number includes: a research octane number and a motor octane number.

A step of training the product prediction model preferably includes:

establishing a product prediction model; wherein the product prediction model includes: a set of reaction rules comprising a plurality of reaction rules and a reaction rate algorithm;
acquiring sample feedstock information for a sample feedstock;
training the set of reaction rules by using the sample feedstock information, and fixing the set of reaction rules that has been trained; and
training the reaction rate algorithm by using the sample feedstock information, and fixing the reaction rate algorithm that has been trained, to obtain the product prediction model that has been trained.

The sample feedstock information of the sample feedstock preferably includes: molecular composition of the sample feedstock, molecular content of each molecule in the sample feedstock, molecular composition of an actual product corresponding to the sample feedstock, and actual content of each molecule in the actual product.

The training the set of reaction rules by using the sample feedstock information preferably includes:

processing the molecular composition of the sample feedstock according to a preset set of reaction rules, to obtain a reaction pathway corresponding to each molecule in the molecular composition of the sample feedstock;
obtaining first molecule composition of a device output product comprising the sample feedstock, an intermediate product, and a predicted product according to the reaction path corresponding to each molecule in the molecular composition of the sample feedstock; in the device output product, comprising: the sample feedstock, the intermediate product, and the predicted product;
calculating a first relative deviation according to the first molecular composition of the device output product and second molecular composition of the actual product;
if the first relative deviation meets a preset condition, fixing the set of reaction rules; and
if the first relative deviation does not meet the preset condition, adjusting a reaction rule in the set of reaction rules, and recalculating the first relative deviation according to the adjusted set of reaction rules until the first relative deviation meets the preset condition.

The calculating a first relative deviation according to the first molecular composition of the device output product and second molecular composition of the actual product preferably includes:

acquiring species of single molecules in the first molecule composition, to constitute a first set;
acquiring species of single molecules in the second molecule composition, to constitute a second set;
determining whether the second set is a subset of the first set;
if the second set is not a subset of the first set, obtaining a pre-stored relative deviation value that does not meet the preset condition as the first relative deviation; and
if the second set is a subset of the first set, calculating the first relative deviation in the following manner: determining the first relative deviation according to a ratio of the number of species of the portion of single molecules, which is not in the second set, in the molecular composition of the predicted product to the total number of species of single molecules in the molecular composition of the predicted product.

For example, calculating the first relative deviation by a calculating formula as follows:

$$x_1 = \frac{\mathrm{card}\big((M - M_1 - M_2) - M_3\big)}{\mathrm{card}\ (M - M_1 - M_2)};$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card is the number of elements in the sets.

The training the reaction rate algorithm by using the sample feedstock information preferably includes:

calculating a reaction rate of a reaction path corresponding to each molecule in the molecular composition of the sample feedstock, respectively, according to the reaction rate algorithm;
obtaining predicted content of each molecule in a predicted product corresponding to the sample feedstock according to molecular content of each molecule in the sample feedstock and the reaction rate of the reaction path corresponding to the molecule;
calculating a second relative deviation according to the predicted content of each molecule in the predicted product and the actual content of each molecule in the actual product;

if the second relative deviation meets a preset condition, fixing the reaction rate algorithm; and
if the second relative deviation does not meet the preset condition, adjusting a parameter in the reaction rate algorithm, and recalculating the second relative deviation according to the adjusted reaction rate algorithm until the second relative deviation meets the preset condition.

The calculating a reaction rate of a reaction path corresponding to each molecule in the molecular composition of the sample feedstock, respectively, according to the reaction rate algorithm preferably includes:

calculating a reaction rate of each reaction path according to a reaction rate constant in the reaction rate algorithm;
wherein the reaction rate constant is determined based on a transition state theoretical calculation method.

For example, the reaction rate constant is determined according to a calculation formula as follows:

$$k = \frac{k_B E}{h} \exp(\frac{E\Delta S - \Delta E}{RE})\varphi \times P^\alpha$$ ;

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

The acquiring molecular composition of various fractions obtained by distillation of the crude oil preferably includes:

acquiring molecular composition of crude oil; and
acquiring molecular composition of various fractions obtained by distillation of the crude oil according to physical properties of the various single molecules in the molecular composition of the crude oil.

Types of the petroleum processing devices include:
a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit; wherein each petroleum processing device corresponds to a set of reaction rules.

According to a second aspect, the present disclosure provides an apparatus for real-time optimization of a molecular level device, the apparatus for real-time optimization comprising:

a first acquisition unit configured to acquire molecular composition of crude oil;
a first processing unit configured to acquire molecular composition of various fractions obtained by distillation of the crude oil according to physical properties of various single molecules in the molecular composition of the crude oil;
a second processing unit configured to respectively input, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition;
a second acquisition unit configured to acquire a preset standard set for a preset target product; and
a third processing unit configured to determine whether the predicted product meets a preset standard for a target product corresponding to the predicted product in the preset standard set according to the predicted molecular composition of the predicted product and the predicted molecular content of each single molecule in the predicted molecular composition, and if the predicted product does not meet any preset standard for a target product corresponding to the predicted product in the preset standard set, adjust an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set.

The apparatus further includes:
a flow control unit configured to acquire an input flow of petroleum processing feedstocks input to each of the petroleum processing devices, determine whether each of the input flows meets a preset input flow range of the respective petroleum processing device, adjust the preset feedstock ratio if any one of the input flows does not meet the preset input flow range of the respective petroleum processing device, and respectively re-input, according to the adjusted preset feedstock ratio, the corresponding fractions into the pre-trained product prediction model of the respective petroleum processing device as petroleum processing feedstocks, until each of the input flows meets the preset input flow range of the respective petroleum processing device.

The third processing unit, in particular, is configured to calculate a physical property of each single molecule in the predicted molecular composition according to the predicted molecular composition of the predicted product and the predicted molecular content of each single molecule in the predicted molecular composition, calculate a predicted physical property of the predicted product according to the physical property of each single molecule in the predicted molecular composition and the predicted molecular content of each single molecule in the predicted molecular composition, and determine whether the predicted physical property of each of the predicted products meets a preset physical property restriction interval of the corresponding target product in the preset standard set.

The apparatus further includes:

a product blending unit configured to blend each of the predicted products which is used as a product blending feedstock according to a preset rule set, to obtain molecular composition of a plurality of mixed products and content of each single molecule in each of the mixed products, and respectively calculate product property of each of the mixed products according to the molecular composition of each of the mixed products and the content of each single molecule in each of the mixed products.

The third processing unit, in particular, is configured to determine whether the product property of each of the mixed products meets a preset product property of a target mixed product obtained by blending corresponding each target product in the preset standard set;

if the preset product property is met, obtain a target parameter according to all of the mixed products and determining whether the target parameter meets a preset condition;

if the target parameter meets the preset condition, determine that the predicted product meets a preset standard for a target product corresponding to the predicted product in the preset standard set, and output the preset feedstock ratio, the pre-trained product prediction model and the preset rule set as a production and processing scheme; and

if the target parameter does not meet the preset condition, adjust the operation parameter in the pre-trained product prediction model and a preset rule in the preset rule set, to re-obtain a plurality of mixed products, until the product property of each of the mixed products meets the preset product property and the target parameters in all of the mixed products meet the preset condition.

The third processing unit, in particular, is configured to acquire a product price of each of mixed products and a yield of each of mixed products, calculate a product benefit of each of mixed products according to the yield of each of mixed products and the product price of each of mixed products, accumulate the product benefit of each of mixed products to obtain a cumulative benefit, acquire a feedstock price of each group of the petroleum processing feedstocks and an operating cost of each of the petroleum processing devices, subtract feedstock prices of all of the petroleum processing feedstocks and operating costs of all of the petroleum processing devices from the cumulative benefit to obtain a comprehensive benefit, serve the comprehensive benefit as the target parameter, determine whether the comprehensive benefit reaches a maximum value, determine that the target parameter meets the preset condition if the comprehensive benefit reaches the maximum value; and determine that the target parameter does not meet the preset condition if the comprehensive benefit does not reach the maximum value.

The third processing unit, in particular, is configured to adjust a temperature of an environment where a reaction path corresponding to the predicted product in the pre-trained product prediction model is located, and re-obtain the predicted molecular composition of the predicted product and the predicted molecular content of each single molecule in each of the predicted products according to the adjusted temperature until the predicted product meets the preset standard for the target product corresponding to the predicted product in the preset standard set.

The third processing unit, in particular, is configured to adjust a pressure of an environment where a reaction path corresponding to the predicted product in the pre-trained product prediction model is located, and re-obtain the predicted molecular composition of the predicted product and the predicted molecular content of each single molecule in each of the predicted products according to the adjusted pressure until the predicted product meets the preset standard for the target product corresponding to the predicted product in the preset standard set.

The product blending unit, in particular, is configured to acquire first molecular composition of each group of the product blending feedstocks and first component content of each single molecule in each group of the product blending feedstocks, based on the preset rule set, obtain second molecular composition of each of mixed products and second component content of each single molecule in each of mixed products according to the first molecular composition of each group of the product blending feedstocks and the first component content of each single molecule in each group of the product blending feedstocks, calculate a physical property of each single molecule in each of the mixed products according to the number of groups of each group contained in each single molecule in each of the mixed products and a contribution value of each group to the physical property, and calculate a product property of each of the mixed products according to the physical property and the second component content of each single molecule in each of the mixed products.

The product blending unit, in particular, is configured to, for each single molecule, acquire the number of groups of each group constituting the single molecule and a contribution value of each group to the physical property, and input the number of groups of each group constituting the single molecule and the contribution value of each group to the physical property into a pre-trained property calculation model, to acquire the physical property of the single molecule outputted by the pre-trained property calculation model; wherein the pre-trained property calculation model is used to calculate the physical property of the single molecule according to the number of groups of each group contained in a single molecule and a contribution value of each group to the physical property.

The apparatus further includes:
a single molecule property template matching unit configured to compare the number of groups of each group constituting the single molecule with molecular information of a template single molecule with known physical properties pre-stored in a database, the molecular information comprising the number of groups of each group constituting the template single molecule, determine whether there is a same template single molecule as the single molecule, if there is a same template single molecule as the single molecule, output the physical properties of the template single molecule as a physical property of the single molecule, and if there is not a same template single molecule as the single molecule, then, by the product blending unit, perform the step of the inputting the number of groups of each group constituting the single molecule and the contribution value of each group to the physical property into a pre-trained property calculation model.

The apparatus further includes:
a model training unit configured to construct a property calculation model of a single molecule, acquire the number of groups of each group constituting a sample single molecule; wherein the physical property of the sample single molecule is known, input the number of groups of each group constituting the sample single molecule into the property calculation model, acquire a predicted physical property of the sample single molecule output by the property calculation model, if a deviation value between the predicted physical property and the physical property which is known is less than a preset deviation threshold, determine that the property calculation model converges, acquire a contribution value of each group to the physical property in the property calculation model which is converged, and store the contribution value of the group to the physical property, and if the deviation value between the predicted physical property and the physical property which is known is greater than or equal to the deviation threshold, adjust a contribution value of each group to the physical property in the property calculation model until the property calculation model converges.

The model training unit, in particular, is configured to establish the property calculation model as shown below:

$$f = a + \sum n_i \Delta f_i \;$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.

The model training unit, in particular, is configured to determine a primary group, the number of groups of the primary group, a multi-stage group, and the number of groups of the multi-stage group in all groups of the single molecule, take all groups constituting the single molecule as the primary group, and take various groups which coexist and contribute to a same physical property in common as the multi-stage group, and take the number of the various groups as a level of the multi-stage group.

The model training unit, in particular, is configured to establish the property calculation model as shown below:

$$f = a + \sum_i \mathrm{m}_{1i} \Delta f_{1i} + \sum_j m_{2j} \Delta f_{2j} \ldots\ldots + \sum_l m_{Nl} \Delta f_{Nl} \;$$

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and N is a positive integer greater than or equal to 2.

The product blending unit, in particular, is configured to determine a primary group, the number of groups of the primary group, a multi-stage group, and the number of groups of the multi-stage group in all groups of the single molecule, take all groups constituting the single molecule as the primary group, and take various groups which coexist and contribute to a same physical property in common as the multi-stage group, and take the number of the various groups as a level of the multi-stage group.

The product blending unit, in particular, is configured to calculate a boiling point of the single molecule according to a property calculation model as follows:

$$T = \frac{SOL \times GROUP_{11} + SOL \times GROUP_{12} + \ldots\ldots + SOL \times GROUP_{1N}}{(SOL \times Numh)^d + b} + c \; ;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

The product blending unit, in particular, is configured to calculate a density of the single molecule according to a property calculation model as follows:

$$D = \frac{SOL \times GROUP_{21}}{(SOL \times GROUP_{22} + \ldots\ldots + SOL \times GROUP_{2N}) \times e} \; ;$$

where, $D$ is the density of the single molecule, $SOL$ is a single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

The product blending unit, in particular, is configured to calculate an octane number of the single molecule according to a property calculation model as follows:

$$X = SOL \times GROUP_{31} + SOL \times GROUP_{32} + \ldots\ldots + SOL \times GROUP_{3N} + h \; ;$$

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUP_{32}$ is a 2N+2-th contribution value vector converted according to a contribution value of the secondary group to the octane number, $GROUP_{3N}$ is a 3N-ty contribution value vector converted according to a contribution value of the N-stage group to the octane number; N is a positive integer greater than or equal to 2; and $h$ is the fifth preset constant.

The product property of the mixed products includes a density, a cloud point, a pour point, an aniline point, and an octane number.

The product blending unit, in particular, is configured to calculate the density of each of the mixed products according to a calculation formula as follows:

$$density = \sum (D_i \times x_{i\_volume}) \; ;$$

where, $density$ is the density of the mixed product, $D_i$ is the density of the $i$-th single molecule, and $x_{i\_volume}$ is second component content of the $i$-th single molecule.

The product blending unit, in particular, is configured to, for each mixed product, calculate a cloud point contribution value of each single molecule according to the density and the boiling point of each single molecule in the mixed product, and calculate the cloud point of the mixed product according to cloud point contribution values of all of the single molecules and content of each single molecule in the mixed product.

The product blending unit, in particular, is configured to, for each mixed product, calculate a pour point contribution value of each single molecule according to the density and molecular weight of each single molecule in the mixed product, and calculate the pour point of the mixed product according to pour point contribution values of all of the single molecules and content of each single molecule in the mixed product.

The product blending unit, in particular, is configured to, for each mixed product, calculate an aniline point contribution value of each single molecule according to the density and the boiling point of each single molecule in the mixed product, and calculate the aniline point of the mixed product according to aniline point contribution values of all of the single molecules and content of each single molecule in the mixed product.

The product blending unit, in particular, is configured to, for each mixed product, acquire the octane number of each single molecule and content of each single molecule in the mixed product, and calculate the octane number of the mixed products according to a calculation formula as follows:

$$\begin{aligned}
ON = (&\sum_{i=\text{HISQFG}} \upsilon_i\beta_i ON_i + C_H \sum_{i=H} \upsilon_i\beta_i ON_i + C_I \sum_{i=I} \upsilon_i\beta_i ON_i + C_S \sum_{i=S} \upsilon_i\beta_i ON_i \\
&+ C_Q \sum_{i=Q} \upsilon_i\beta_i ON_i + C_F \sum_{i=F} \upsilon_i\beta_i ON_i + C_G \sum_{i=G} \upsilon_i\beta_i ON_i) \div \\
(&\sum_{i=\text{HISQFG}} \upsilon_i\beta_i + C_H(\sum_{i=H}\upsilon_i\beta_i - \sum_{i=H}\upsilon_i) + C_I(\sum_{i=I}\upsilon_i\beta_i - \sum_{i=I}\upsilon_i) + C_S(\sum_{i=S}\upsilon_i\beta_i - \sum_{i=S}\upsilon_i) \\
&+ C_Q(\sum_{i=Q}\upsilon_i\beta_i - \sum_{i=Q}\upsilon_i) + C_F(\sum_{i=F}\upsilon_i\beta_i - \sum_{i=F}\upsilon_i) + C_G(\sum_{i=G}\upsilon_i\beta_i - \sum_{i=G}\upsilon_i))
\end{aligned} \quad ;$$

$$C_H = \frac{k_{HI}^{(a)}\upsilon_I + k_{HS}^{(a)}\upsilon_S + k_{HQ}^{(a)}\upsilon_Q + k_{HF}^{(a)}\upsilon_F + k_{HG}^{(a)}\upsilon_G}{1 + k_{HI}^{(b)}\upsilon_I + k_{HS}^{(b)}\upsilon_S + k_{HQ}^{(b)}\upsilon_Q + k_{HF}^{(b)}\upsilon_F + k_{HG}^{(b)}\upsilon_G} \quad ;$$

$$C_I = \frac{k_{HI}^{(a)}\upsilon_H + k_{IS}^{(a)}\upsilon_S + k_{IQ}^{(a)}\upsilon_Q + k_{IF}^{(a)}\upsilon_F + k_{IG}^{(a)}\upsilon_G}{1 + k_{HI}^{(b)}\upsilon_H + k_{IS}^{(b)}\upsilon_S + k_{IQ}^{(b)}\upsilon_Q + k_{IF}^{(b)}\upsilon_F + k_{IG}^{(b)}\upsilon_G} \quad ;$$

$$C_S = \frac{k_{HS}^{(a)}\upsilon_H + k_{IS}^{(a)}\upsilon_I + k_{SQ}^{(a)}\upsilon_Q + k_{SF}^{(a)}\upsilon_F + k_{SG}^{(a)}\upsilon_G}{1 + k_{HS}^{(b)}\upsilon_H + k_{IS}^{(b)}\upsilon_I + k_{SQ}^{(b)}\upsilon_Q + k_{SF}^{(b)}\upsilon_F + k_{SG}^{(b)}\upsilon_G} \quad ;$$

$$C_Q = \frac{k_{HQ}^{(a)}\upsilon_H + k_{IQ}^{(a)}\upsilon_I + k_{SQ}^{(a)}\upsilon_S + k_{QF}^{(a)}\upsilon_F + k_{QG}^{(a)}\upsilon_G}{1 + k_{HQ}^{(b)}\upsilon_H + k_{QI}^{(b)}\upsilon_I + k_{SQ}^{(b)}\upsilon_S + k_{QF}^{(b)}\upsilon_F + k_{QG}^{(b)}\upsilon_G} \quad ;$$

$$C_F = \frac{k_{HF}^{(a)}\upsilon_H + k_{IF}^{(a)}\upsilon_I + k_{SF}^{(a)}\upsilon_S + k_{QF}^{(a)}\upsilon_Q + k_{FG}^{(a)}\upsilon_G}{1 + k_{HF}^{(b)}\upsilon_H + k_{IF}^{(b)}\upsilon_I + k_{SF}^{(b)}\upsilon_S + k_{QF}^{(b)}\upsilon_Q + k_{FG}^{(b)}\upsilon_G} \quad ;$$

$$C_G = \frac{k_{HG}^{(a)}\upsilon_H + k_{IG}^{(a)}\upsilon_I + k_{SG}^{(a)}\upsilon_S + k_{QG}^{(a)}\upsilon_Q + k_{FG}^{(a)}\upsilon_F}{1 + k_{HG}^{(b)}\upsilon_H + k_{IG}^{(b)}\upsilon_I + k_{SG}^{(b)}\upsilon_S + k_{QG}^{(b)}\upsilon_Q + k_{FG}^{(b)}\upsilon_F} \quad ;$$

where, ON is the octane number of the mixed product, HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $\upsilon_i$ is content of each molecule in the mixed product, $\upsilon_H$, $\upsilon_I$, $\upsilon_S$, $\upsilon_Q$, $\upsilon_F$ and $\upsilon_G$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_H$ is an interaction coefficient of n-alkanes with other molecules, $C_I$ is an interaction coefficient of isoalkanes with other molecules; $C_S$ is an interaction coefficient of cycloalkanes with other molecules; $C_Q$ is an interaction coefficient of olefins with other molecules, $C_F$ is an interaction coefficient of aromatic hydrocarbons with other molecules, $C_G$ is an interaction coefficient of oxygenated compounds with other molecules, $k_{HI}^{(a)}$ is a first constant coefficient between n-alkanes and isoalkanes, $k_{HS}^{(a)}$ is a first constant coefficient between n-alkanes and cycloalkanes, $k_{HQ}^{(a)}$ is a first constant coefficient between n-alkanes and olefins, $k_{HF}^{(a)}$ is a first constant coefficient between n-alkanes and aromatic hydrocarbons, $k_{HG}^{(a)}$ is a first constant coefficient between n-alkanes and oxygenated compounds, $k_{IS}^{(a)}$ is a first constant coefficient between isoalkanes and cycloalkanes, $k_{IQ}^{(a)}$ is a first constant coefficient between isoalkanes and olefins, $k_{IF}^{(a)}$ is a first constant coefficient between isoalkanes and aromatic hydrocarbons, $k_{IG}^{(a)}$ is a first constant coefficient between isoalkanes and oxygenated compounds, $k_{SQ}^{(a)}$ is a first constant coefficient between cycloalkanes and olefins, $k_{SF}^{(a)}$ is a first constant coefficient between cycloalkanes and aromatic hydrocarbons, $k_{SG}^{(a)}$ is a first constant coefficient between cycloalkanes and oxygenated compounds, $k_{QF}^{(a)}$ is a first constant coefficient between olefins and aromatic hydrocarbons, $k_{QG}^{(a)}$ is a second constant coefficient between olefins and oxygenated compounds, $k_{FG}^{(a)}$ is a first constant coefficient between aromatic hydrocarbons and oxygenated compounds, $k_{HI}^{(b)}$ is a second constant coefficient between n-alkanes and isoalkanes, $k_{HS}^{(b)}$ is a second constant coefficient between n-alkanes and cycloalkanes, $k_{HQ}^{(b)}$ is a second constant coefficient between n-alkanes and olefins, $k_{HF}^{(b)}$ is a second constant coefficient between n-alkanes and aromatic hydrocarbons, $k_{HG}^{(b)}$ is a second constant coefficient between n-alkanes and oxygenated compounds, $k_{IS}^{(b)}$ is a second constant coefficient between isoalkanes and cycloalkanes, $k_{IQ}^{(b)}$ is a second constant coefficient between isoalkanes and olefins, $k_{IF}^{(b)}$ is a second constant coefficient between isoalkanes and aromatic hydrocarbons, $k_{IG}^{(b)}$ is a second constant coefficient between isoalkanes and oxygenated compounds, $k_{SQ}^{(b)}$ is a second constant coefficient between cycloalkanes and olefins, $k_{SF}^{(b)}$ is a second constant coefficient between cycloalkanes and aromatic hydrocarbons, $k_{SG}^{(b)}$ is a second constant coefficient between cycloalkanes and oxygenated compound, $k_{QF}^{(b)}$ is a second constant coefficient between olefins and aromatic hydrocarbons, $k_{QG}^{(b)}$ is a second constant coefficient between olefins and oxygenated compound, and $k_{FG}^{(b)}$ is a second constant coefficient between aromatic hydrocarbons and oxygenated compound; wherein the octane number includes: a research octane number and a motor octane number.

The apparatus further includes:
a model training unit configure to establish a product prediction model; wherein the product prediction model includes: a set of reaction rules comprising a plurality of reaction rules and a reaction rate algorithm, acquire sample feedstock information for a sample feedstock, train the set of reaction rules by using the sample feedstock information, and fix the set of reaction rules that has been trained, and train the reaction rate algorithm by using the sample feedstock information, and fix the reaction rate algorithm that has been trained, to obtain the product prediction model that has been trained.

The sample feedstock information of the sample feedstock includes: molecular composition of the sample feedstock, molecular content of each molecule in the sample feedstock, molecular composition of an actual product corresponding to the sample feedstock, and actual content of each molecule in the actual product.

The model training unit, in particular, is configured to process the molecular composition of the sample feedstock according to a preset set of reaction rules, to obtain a reaction pathway corresponding to each molecule in the molecular composition of the sample feedstock, obtain first molecule composition of a device output product comprising the sample feedstock, an intermediate product, and a predicted product according to the reaction path corresponding to each molecule in the molecular composition of the sample feedstock; in the device output product, comprising: the sample feedstock, the intermediate product, and the predicted product, calculate a first relative deviation according to the first molecular composition of the device output product and second molecular composition of the actual product, if the first relative deviation meets a preset condition, fix the set of reaction rules, and if the first relative deviation does not meet the preset condition, adjust a reaction rule in the set of reaction rules, and recalculate the first relative deviation according to the adjusted set of reaction rules until the first relative deviation meets the preset condition.

The model training unit, in particular, is configured to acquire species of single molecules in the first molecule composition, to constitute a first set, acquire species of single molecules in the second molecule composition, to constitute a second set, determine whether the second set is a subset of the first set, if the second set is not a subset of the first set, obtain a pre-stored relative deviation value that does not meet the preset condition as the first relative deviation, and if the second set is a subset of the first set, calculate the first relative deviation by a calculating formula as follows:

$$x_1 = \frac{\text{card}\big((M - M_1 - M_2) - M_3\big)}{\text{card}\,(M - M_1 - M_2)} \;;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card is the number of elements in the sets.

The model training unit, in particular, is configured to calculate a reaction rate of a reaction path corresponding to each molecule in the molecular composition of the sample feedstock, respectively, according to the reaction rate algorithm, obtain predicted content of each molecule in the predicted product corresponding to the sample feedstock according to molecular content of each molecule in the sample feedstock and the reaction rate of the reaction path corresponding to the molecule, calculate a second relative deviation according to the predicted content of each molecule in the predicted product and the actual content of each molecule in the actual product, if the second relative deviation meets a preset condition, fix the reaction rate algorithm, and if the second relative deviation does not meet the preset condition, adjust a parameter in the reaction rate algorithm, and recalculate the second relative deviation according to the adjusted reaction rate algorithm until the second relative deviation meets the preset condition.

The model training unit, in particular, is configured to calculate a reaction rate of each reaction path according to a reaction rate constant in the reaction rate algorithm;

wherein the reaction rate constant is determined according to a calculation formula as follows:

$$k = \frac{k_B E}{h} \exp\!\left(\frac{E\Delta S - \Delta E}{RE}\right)\varphi \times P^{\alpha} \;;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

Types of the petroleum processing devices include: a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit; wherein each petroleum processing device corresponds to a set of reaction rules.

According to a third aspect, the present disclosure provides a system for real-time optimization of a molecular level device, the system for real-time optimization of a molecular level device includes a processor and a memory, wherein the processor is configured to execute a real-time optimization program of the molecular level device stored in the memory, to implement a method for real-time optimization of the molecular level device described in the first aspect.

According to a fourth aspect, the present disclosure provides a computer-readable storage medium, wherein the computer-readable storage medium has stored therein one or more programs, the one or more programs being executable by one or more processors, to implement a method for real-time optimization of the molecular level device described in the first aspect.

The above-described technical solutions provided by embodiments of the present disclosure have the following advantages over the related art:

the method provided in the present disclosure is performed by acquiring molecular composition of crude oil; acquiring molecular composition of various fractions obtained by distillation of the crude oil according to physical properties of various single molecules in the molecular composition of the crude oil; respectively inputting, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition; acquiring a preset standard set for a preset target product; determining whether the predicted product meets a preset standard for a target product corresponding to the predicted product in the preset standard set according to the predicted molecular composition of the predicted product and the predicted molecular content of each single molecule in the predicted molecular composition; and if the predicted product does not meet any preset standard for a target product corresponding to the predicted product in the preset standard set, adjusting an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set. By the above method, the embodiments of the present disclosure realize the molecular-level overall simulation and real-time optimization of the molecular-level device from the feedstocks to the product processing process, and improves the accuracy and production efficiency.

Brief Description of the Drawings

FIG. 1 is a flowchart illustrating a method for real-time optimization of a molecular level device according to an embodiment of the present disclosure;
FIG. 2 is a schematic structural diagram of an apparatus for real-time optimization of a molecular level device according to an embodiment of the present disclosure; and
FIG. 3 is a structural diagram of a system for real-time optimization of a molecular level device according to another embodiment of the present disclosure.

Detailed Description

In order to make the objects, technical solutions, and advantages of the embodiments of the disclosure more fully apparent, it will be clear, fully described, and fully described in connection with the accompanying drawings, which are incorporated in and constitute a part of this specification, which illustrate some, but not all embodiments of the disclosure. Based on the embodiments in this disclosure, those of ordinary skill in the art, with no creative effort, are within the scope of the disclosure.

A server implementing various embodiments of the present disclosure will now be described with reference to the attached figures. In the following description, the use of terms, such as "modules", "components" or "units", to represent elements is only intended to facilitate the description of the disclosure, which itself is not to be taken in a limiting sense. Thus, "module" and "component" may be used interchangeably.

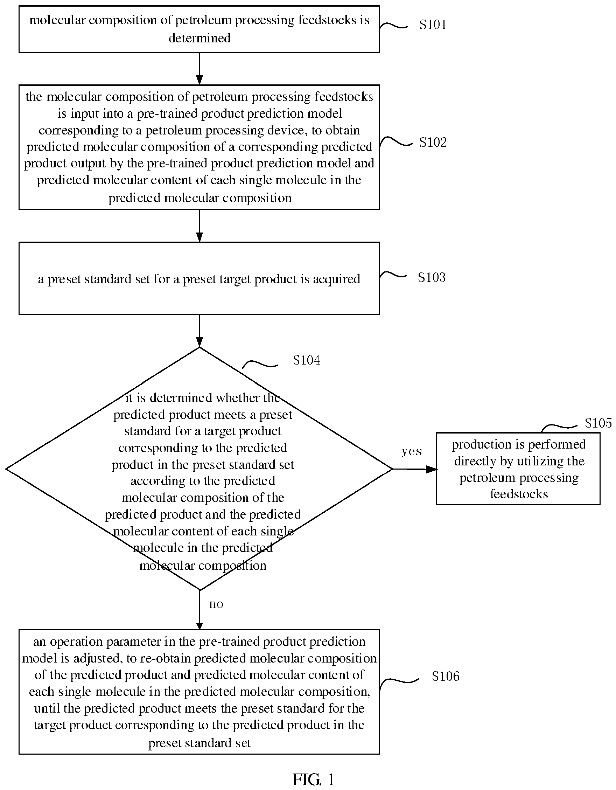

An embodiment of the present disclosure provides a method of the real-time optimization of a molecular level device, as shown in FIG. 1, which may include the following steps:
S101, molecular composition of petroleum processing feedstocks is determined.

In this embodiment, the molecular composition of petroleum processing feedstocks namely is information of the various molecules (single molecule) included in the petroleum processing feedstocks, such as species of various single molecules, content of various single molecules, and concentration of various single molecules. The molecular composition of petroleum processing feedstocks is SOL-based molecular composition.

In this embodiment, the species of the single molecule include, but are not limited to, olefins, alkanes, cycloalkanes, and aromatic hydrocarbons.

S 102, the molecular composition of petroleum processing feedstocks is inputted into a pre-trained product prediction model corresponding to a petroleum processing device, to obtain predicted molecular composition of a corresponding predicted product output by the pre-trained product prediction model and predicted molecular content of each single molecule in the predicted molecular composition.

In the embodiment of the present disclosure, the types of petroleum processing device include:
a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit; wherein each petroleum processing device corresponds to a set of reaction rules.

S103, a preset standard set for a preset target product is acquired.

In the embodiment of the present disclosure, the preset standard set includes one or more preset standard, wherein the preset standard include, but are not limited to, a comprehensive benefit of the product to be generated, a proportion of production of the predicted product in the mixed product, a predicted physical property corresponding to the predicted product. Examples for various preset standard will be described later, and are not described herein redundantly.

S104, it is determined whether the predicted product meets a preset standard for a target product corresponding to the predicted product in the preset standard set according to the predicted molecular composition of the predicted product and the predicted molecular content of each single molecule in the predicted molecular composition, and if yes, step S105 is performed; if the predicted product does not meet any preset standard for a target product corresponding to the predicted product in the preset standard set , step S106 is performed.

S105, production is performed directly by utilizing the petroleum processing feedstocks.

S106, an operation parameter in the pre-trained product prediction model is adjusted, to re-obtain predicted molecular composition of the predicted product and predicted molecular content of each single molecule in the predicted molecular composition, until the predicted product meets the preset standard for the target product corresponding to the predicted product in the preset standard set.

In the embodiments of the present disclosure, the operation parameter includes a temperature of an environment where a reaction path in the pre-trained product prediction model is located and a pressure of an environment where a reaction path in the pre-trained product prediction model is located. Adjustments for the operating parameters will be described later, and are not described herein redundantly.

In another embodiment of the present disclosure, the method further includes:

acquiring molecular composition of crude oil;
acquiring molecular composition of various fractions obtained by distillation of the crude oil according to physical properties of various single molecules in the molecular composition of the crude oil; and
according to a preset feedstock ratio, serving the corresponding fractions as the petroleum processing feedstocks.

In this embodiment, there are many species of molecules in crude oil, different single molecules have different boiling points, and they need to be separated by distillation at different temperatures. Generally, a single molecule with a larger molecular weight in crude oil has a higher boiling point and is more difficult to separate. In the process of crude oil separation, the distillation range is divided according to the type of distilled oil and boiling points of molecules, each distillation range corresponding to a type of distilled oil, to complete the separation of crude oil. In this step, single molecules in crude oil and content corresponding to each single molecule are acquired.

In embodiments of this disclosure, the molecular composition of the petroleum processing feedstocks may be determined by one or more of a comprehensive two-dimensional gas chromatography method, a quaternary rod gas chromatography-mass spectrometer detection method, a gas chromatography/field ionization-time-of-flight mass spectrometry detection method, a gas chromatography method, a near-infrared spectroscopy method, a nuclear magnetic resonance spectroscopy method, a Raman spectroscopy method, a Fourier transform ion cyclotron resonance mass spectrometry method, an electrostatic field rail trap mass spectrometry method, and an ion mobility mass spectrometry method. In addition, the molecular composition of the petroleum processing feedstocks may also be determined in other ways, such as ASTM D2425, SH/T 0606, and ASTM D8144-18.

The molecular detection method described above may detect the structure of the molecule and thereby obtaining the species of the molecule. However, due to the large number of molecular species in crude oil, although the crude oil can no longer be detected when the crude oil is reused after the crude oil is detected once, the workload of detecting each single molecule is large and time-consuming. Therefore, in this scheme, single molecules may also be constructed based on the structure-oriented lumped molecular characterization method. The structure-oriented lumped molecular characterization method namely is the SOL molecular characterization method, which uses 24 structural increment fragments to characterize the basic structure of complex hydrocarbon molecules. Any single petroleum molecule may be represented by a specific set of structural increment fragments. The SOL molecular characterization method is lumped at the molecular scale, reducing the number of molecules in the actual system from millions to thousands, greatly reducing the complexity of the simulation. This characterization method may represent not only alkanes, cycloalkanes, up to complex aromatic structures containing 50-60 carbon atoms, but also olefins or cycloolefins as intermediate products or secondary reaction products, and also consider sulfur, nitrogen, oxygen and other heteroatom compounds.

In this embodiment, the molecular composition of crude oil is information of various molecules (single molecules) in crude oil, such as single molecules contained in the feedstock, a species of a single molecule, a volume and content of each single molecule.

In this embodiment, the boiling point of each single molecule in the crude oil can be calculated separately, the fractional distillation range can be determined based on the boiling point and content of each single molecule, and the crude oil can be distilled and cut according to the fractional distillation range to obtain multiple fractions. **In** this step, since the crude oil is distilled based on the physical property of a single molecule, the molecular composition of each fraction obtained after crude oil distillation can be known.

In this embodiment, the corresponding fractions are used as petroleum processing feedstocks for secondary processing, wherein the preset feedstock ratio namely is the ratio of each fraction input into different petroleum processing devices, respectively. Through the pre-trained product prediction model of each petroleum processing device, combined with the molecular composition of the fraction input to the petroleum processing device, the molecular composition in the predicted product and the content of each single molecule in the predicted product are obtained.

In this embodiment, the fractions obtained after distillation of crude oil include light oil products and heavy oil products. Among them, light oil products, such as naphtha, do not need secondary processing, while heavy oil products generally require different secondary processing, so that heavy oil products are converted into light oil products to improve the properties of oil products. In this solution, the corresponding fractions are inputted into the petroleum processing device for processing according to the preset feedstock ratio. The preset feedstock ratio includes: the type and amount of the fractions input to the petroleum processing device, and the fraction that does not require secondary processing is not included in the preset feedstock ratio.

In this embodiment, the pre-trained product prediction model has been trained and optimized. Through the pre-trained product prediction model, it is possible to adjust the reaction conditions in the petroleum processing device, such as pressure, temperature and space velocity after the petroleum processing feedstocks are inputted into the petroleum processing device, so as to suppress the progress of certain reactions or improve the progress of certain reactions to control the formation of products. **In** this step, the product situation under a certain set condition may be obtained.

In another embodiment of the present disclosure, the method further includes:

acquiring a preset input flow range of petroleum processing feedstocks input to each of the petroleum processing devices;

determining whether each of the input flows meets the preset input flow range of the respective petroleum processing device;

adjusting the preset feedstock ratio if any one of the input flows does not meet the preset input flow range of the respective petroleum processing device, and respectively re-inputting, according to the adjusted preset feedstock ratio, the corresponding fractions into the pre-trained product prediction model of the respective petroleum processing device as petroleum processing feedstocks, until each of the input flows meets the preset input flow range of the respective petroleum processing device; and

if each of the input flows meets the preset input flow range of the respective petroleum processing device, then performing the step of obtaining predicted molecular composition of a corresponding predicted product and predicted molecular content of each single molecule in the predicted molecular composition. **In** this embodiment, the input flow of the feedstock meets the preset input flow range of the respective petroleum processing device, the subsequent steps of the scheme are directly performed.

In this embodiment, each group of petroleum processing devices has a corresponding processing capacity, to avoid the situation where the processing time of the feedstocks in the petroleum processing device is too short and the feedstocks do not react completely due to the input of the feedstocks exceeding the processing capacity of the petroleum processing unit, and the worse situation may cause damage to the petroleum processing device. In this embodiment, a preset input flow range is set, and the maximum value of the range can be between 80% and 95% of the maximum processing capacity of the petroleum processing device, and thus by limiting the amount of feedstocks entering the petroleum processing device, damage to the petroleum processing device is avoided.

In this embodiment, when the feedstock input flow of any of the petroleum processing devices is greater than the preset input flow range, the preset feedstock ratio is adjusted and the amount of petroleum processing feedstocks input to the petroleum processing device is re-planed, such that the input flow of the feedstocks of each petroleum processing device meets the preset input flow rate range of the respective petroleum processing device.

In another embodiment of the present disclosure, the determining whether the predicted product meets a preset standard for a target product corresponding to the predicted product in the preset standard set in the step S104 further includes:

calculating a predicted physical property of the predicted product according to the physical property of each single molecule in the predicted molecular composition and the predicted molecular content of each single molecule in the predicted molecular composition; the predicted physical property of the predicted product including, but not limited to, a density, a cloud point, a pour point, an aniline point, and an octane number;

determining whether the predicted physical property of each of the predicted products meets a preset physical property restriction interval of the corresponding target product in the preset standard set; and

if the predicted physical property of each of the predicted products meets the preset physical property restriction interval, determining that the predicted product meets the preset standard for the target product corresponding to the predicted product in the preset standard set, and performing the step of obtaining predicted molecular composition of a corresponding predicted product and predicted molecular content of each single molecule in the predicted molecular composition.

In another embodiment of the present disclosure, the method further includes:

blending each of the predicted products which is used as a product blending feedstock according to a preset rule set, to obtain molecular composition of a plurality of mixed products and content of each single molecule in each of the mixed products; and

respectively calculating product property of each of the mixed products according to the molecular composition of each of the mixed products and the content of each single molecule in each of the mixed products.

In this embodiment, the predicted products inputted by each petroleum processing device are blended as a product blending feedstock, wherein each set of preset rules in the preset rule set includes the type and amount of the predicted product used. Corresponding mixed products are obtained by mixing the predicted products output by different petroleum processing devices, wherein the mixed products include but are not limited to gasoline products such as automotive oil, lubricating oil, hydraulic oil, gear oil, cutting oil and so on for vehicles. The production planning may be completed by blending the product blending feedstocks so that the obtained mixed products meet the national standards of the corresponding products.

In this embodiment, according to the molecular composition of the predicted product and the content of each single molecule in the predicted product, combined with the preset rule set, the molecular composition of the different mixed products and the content of each single molecule in the different mixed products are obtained.

In another embodiment of the present disclosure, the determining whether the predicted product meets a preset standard for a target product corresponding to the predicted product in the preset standard set in step S104 further includes:

determining whether the product property of each of the mixed products meets a preset product property of a target mixed product obtained by blending corresponding each target product in the preset standard set;

if the preset product property is met, obtaining a target parameter according to all of the mixed products and determining whether the target parameter meets a preset condition;

if the target parameter meets the preset condition, determining that the predicted product meets a preset standard for a target product corresponding to the predicted product in the preset standard set, and outputting the preset feedstock ratio, the pre-trained product prediction model and the preset rule set as a production and processing scheme; and

if the target parameter does not meet the preset condition, adjusting the operation parameter in the pre-trained product prediction model and a preset rule in the preset rule set, to re-obtain a plurality of mixed products, until the product property of each of the mixed products meets the preset product property and the target parameters in all of the mixed products meet the preset condition.

In this embodiment, the product property of each of mixed products is calculated separately. The various single molecules included in each of mixed products are determined, that is, the molecular composition of the mixed product is determined, the physical property of each single molecule in the mixed product is calculated separately, then the physical property of the mixed gasoline product is calculated according to the physical property and content of each single molecule in the mixed gasoline product. The physical property of single molecule includes, but not limited to, a density, a boiling point, a density, and an octane number. For example, the physical property of single molecule may also include viscosity, solubility parameters, cetane number, degree of unsaturation, etc.

In this embodiment, if the product property of each of mixed products meets a preset product property, it means that each of the mixed products blended at this time is an eligible product. The relevant target parameters are obtained according to the mixed products, and it is determined whether the target parameters meet the preset conditions. The target parameters may be the economic benefits of the product, the content of substances in the product that will cause harm to the environment, and the proportion of products, which meet a preset standard, in all mixed products to all of the mixed products. In this step, the ultimate goal of the refinery's refining is to pursue benefits. A gross profit value may be calculated according to the price of each mixed product and the amount of the mixed product, and the gross profit value may be used to confirm whether the final benefit has reached the maximum, so as to confirm whether the target parameters meet the preset conditions, among which, confirming whether the final benefit has reached the maximum may be calculated by random algorithm. Meanwhile, with the gradual strengthening of people's environmental protection awareness, the content of substances that will cause harm to the environment in the mixed product will also affect the sales of the mixed product, even if the calculated benefit value is large, which cannot be sold at the sales end and cannot be converted into benefits. Therefore, in order to increase the competitiveness of oil products, it is possible to limit the content of substances that are harmful to the environment in mixed products. Moreover, when different mixed products are sold, the market will have different demand. For example, the price of No. 98 motor gasoline is higher than the price of No. 95 motor gasoline, but the consumption of No. 95 motor gasoline is larger, and if the refinery produces a large amount of No. 98 motor gasoline, the market will take longer to digest it, resulting in a backlog of No. 98 motor gasoline inventory, resulting in more labor and other costs, resulting in the final benefits are not as good as that of No. 95 motor gasoline. Therefore, in this step, the proportion of the production volume of mixed products that meet a certain preset standard in all mixed products may be calculated to avoid product backlog.

For instance, in another embodiment of the present disclosure, the determining whether the predicted product meets a preset standard for a target product corresponding to the predicted product in the preset standard set in step S104 further includes:

acquiring a product price of each of mixed products and a yield of each of mixed products;

calculating a product benefit of each of mixed products according to the yield of each of mixed products and the product price of each of mixed products;

accumulating the product benefit of each of mixed products to obtain a cumulative benefit;

acquiring a feedstock price of each group of the petroleum processing feedstocks and an operating cost of each of the petroleum processing devices;

subtracting feedstock prices of all of the petroleum processing feedstocks and operating costs of all of the petroleum processing devices from the cumulative benefit to obtain a comprehensive benefit;

serving the comprehensive benefit as the target parameter; determining whether the comprehensive benefit reaches a maximum value;

determining that the target parameter meets the preset condition if the comprehensive benefit reaches the maximum value; and

determining that the target parameter does not meet the preset condition if the comprehensive benefit does not reach the maximum value.

In this embodiment, the comprehensive benefit is taken as the target parameter to ensure the production benefit, which may be determined whether the comprehensive benefit reaches the maximum value through a global optimization algorithm of random search with multiple starting points.

In this embodiment, when the target parameter also meets the corresponding preset condition, it means that the overall production process has met the production requirements at this time, and sustainable production may be carried out. At this time, the preset feedstock ratios for different fractions input into different petroleum processing devices in the output scheme, the pre-trained product prediction model used to calculate the molecular composition of the predicted product produced by each petroleum processing device and the content of each single molecule, and the preset rule set for blending predicted products output from petroleum processing devices are taken as a production and processing scheme. In the actual production process, the production and processing scheme is used for production, and the real-time optimization of the device is realized at the molecular level.

In this embodiment, when the target parameter does not meet the preset condition, it means that the economic benefits of the final blended mixed product may not reach the maximum value, or that the amount of substances with environmental impact in the mixed product exceeds the set value, or that the proportion of mixed products that meet a preset standard in all mixed products does not reach the set value. At this time, by adjusting the operation parameter in the pre-trained product prediction model and the preset rule in the preset rule set, a plurality of mixed products in another situation may be obtained, until the product property of each of mixed products output in this scheme meets the preset rule and the target parameters in all mixed products meet the preset conditions, that is, the real-time optimization of the molecular-level device is completed.

In another embodiment of the present disclosure, the operation parameter includes a temperature of an environment where a reaction path in the pre-trained product prediction model is located, and the adjusting an operation parameter in the pre-trained product prediction model in the step S106 further includes:

adjusting a temperature of an environment where a reaction path corresponding to the predicted product in the pre-trained product prediction model is located; and

re-obtaining the predicted molecular composition of the predicted product and the predicted molecular content of each single molecule in each of the predicted products according to the adjusted temperature until the predicted product meets the preset standard for the target product corresponding to the predicted product in the preset standard set.

In another embodiment of the present disclosure, the operation parameter includes a pressure of an environment where a reaction path in the pre-trained product prediction model is located, and the adjusting an operation parameter in the pre-trained product prediction model further includes:

adjusting a pressure of an environment where a reaction path corresponding to the predicted product in the pre-trained product prediction model is located; and

re-obtaining the predicted molecular composition of the predicted product and the predicted molecular content of each single molecule in each of the predicted products according to the adjusted pressure until the predicted product meets the preset standard for the target product corresponding to the predicted product in the preset standard set.

Further description of calculating the product property for each of mixed products. The respectively calculating product property of each of the mixed products according to the molecular composition of each of the mixed products and the content of each single molecule in each of the mixed products includes:

first molecular composition of each group of the product blending feedstocks and first component content of each single molecule in each group of the product blending feedstock are acquired; Since the product blending feedstocks are the predicted product of each group of petroleum processing devices, the first molecular composition of the product blending feedstocks and the first component content of each single molecule may be obtained based on the predicted product.

Based on the preset rule set, second molecular composition of each of mixed products and second component content of each single molecule in each of mixed products are obtained according to the first molecular composition of each group of the product blending feedstocks and the first component content of each single molecule in each group of the product blending feedstocks; in this embodiment, the preset rules in the preset rule set set the type and quantity of the required product blending feedstocks, therefore, according to the molecular composition and the first component content of each single molecule in the product blending feedstocks, the second molecular composition of the mixed product and the second component content of each single molecule may be obtained.

A physical property of each single molecule is calculated according to the number of groups of each group contained in each single molecule in each of the mixed products and a contribution value of each group to the physical property; in this embodiment, for each single molecule, the number of groups of each group constituting the single molecule and a contribution value of each group to the physical property are acquired, and the number of groups of each group constituting the single molecule and the contribution value of each group to the physical property are inputted into a pre-trained property calculation model, to acquire the physical property of the single molecule outputted by the pre-trained property calculation model.

A physical property of each of the mixed products is calculated according to the physical property and the second component content of each single molecule in each of the mixed products.

The properties of the mixed gasoline product include: Research Octane Number, Motor Octane Number, Reid vapor pressure, Enn's distillation range, density, benzene volume fraction, aromatics volume fraction, olefin volume fraction, oxygen mass fraction, and sulfur quality fraction.

Calculation of the physical property of each single molecule includes: for each single molecule, acquiring the number of groups of each group constituting the single molecule and a contribution value of each group to the physical property; and

> inputting the number of groups of each group constituting the single molecule and the contribution value of each group to the physical property into a pre-trained property calculation model, to acquire the physical property of the single molecule outputted by the pre-trained property calculation model;
> wherein the pre-trained property calculation model is used to calculate the physical property of the single molecule according to the number of groups of each group contained in single molecule and a contribution value of each group to the physical property.

In another embodiment of the present disclosure, before the inputting the number of groups of each group constituting the single molecule and the contribution value of each group to the physical property into a pre-trained property calculation model, the method further includes:

> comparing the number of groups of each group constituting the single molecule with molecular information of a template single molecule with known physical properties pre-stored in a database, the molecular information comprising the number of groups of each group constituting the template single molecule;
> determining whether there is a same template single molecule as the single molecule;
> if there is a same template single molecule as the single molecule, outputting the physical properties of the template single molecule as a physical property of the single molecule; and
> if there is not a same template single molecule as the single molecule, then performing the step of the inputting the number of groups of each group constituting the single molecule and the contribution value of each group to the physical property into a pre-trained property calculation model.

In another embodiment of the present disclosure, a step of training the property calculation model includes:

> constructing a property calculation model of a single molecule;
> acquiring the number of groups of each group constituting a sample single molecule; wherein the physical property of the sample single molecule is known;
> inputting the number of groups of each group constituting the sample single molecule into the property calculation model;
> acquiring a predicted physical property of the sample single molecule output by the property calculation model;
> if a deviation value between the predicted physical property and the physical property which is known is less than a preset deviation threshold, determining that the property calculation model converges, acquiring a contribution value of each group to the physical property in the property calculation model which is converged, and storing the contribution value of the group to the physical property; and

if the deviation value between the predicted physical property and the physical property which is known is greater than or equal to the deviation threshold, adjusting a contribution value of each group to the physical property in the property calculation model until the property calculation model converges.

In this embodiment, in the property calculation model, a contribution value of each group to physical properties is included. The contribution value is an adjustable value, and the contribution value is the initial value during the first training. Further, in the property calculation model, a contribution value of each group to each physical property is included.

In this embodiment, a training sample set is preset. A plurality of sample single molecule information is included in the training sample set. The sample single molecule information includes, but not limited to, the number of groups of each group constituting a sample single molecule, as well as the physical property of the sample single molecule.

In this embodiment, since the physical property of the single molecule may be various, the contribution value of each group to each physical property may be obtained from the converged property calculation model.

For each group, the contribution value of the group to each physical property is stored, so that when the physical property of the single molecule is subsequently calculated, the contribution value of each group in the single molecule to the physical property that needs to be known may be obtained, and the number of groups of each group in the single molecule and the contribution value of each group to the physical properties that need to be known are used as the input of the property calculation model. The property calculation model takes the number of groups of each group in the single molecule as the model variable, and the contribution value of each group to the physical property that needs to be known as the model parameter (replace the adjustable contribution value of each group in the property calculation model to the physical property), and calculate the physical properties that need to be known.

In this embodiment, if there are multiple physical properties of the sample single molecule, the predicted physical property of the sample single molecule output by the property calculation model will also be multiple. At this time, the deviation value between each predicted physical property and the corresponding physical property which is known is calculated, it is determined if the deviation values between all the predicted physical property and the corresponding physical property which is known are less than the preset deviation threshold, and if so, it is determined that the property calculation model converges, and the contribution value of each group corresponding to the physical property may be obtained according to the property calculation model which is converged. The contribution value of each group to different physical properties may be obtained through the above scheme.

Two property calculation models that may be used for different physical properties are given below. It should be appreciated by those skilled in the art that the following two property calculation models are only illustrative of the present embodiments and are not intended to limit the present embodiments.

Model one, a property calculation model as follows is established:

$$f = a + \sum n_i \Delta f_i \, ;$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.

For example: for the boiling point, in the SOL-based molecular characterization method, 24 groups are present as a primary group; among the 24 groups, one or more of N6, N5, N4, N3, me, AA, NN, RN, NO, RO, and KO also contribute to the boiling point, while for different physical properties, the contribution value of the group to the physical property is inconsistent, but the contribution value of the same group in different molecules to the same physical property is consistent. Based on this scheme, the above-mentioned property calculation model is established in this embodiment, and by training the established property calculation model, it makes the property calculation model convergent, that is, the contribution value of each group in the model to the physical property is trained, and the contribution value of each group to the physical property is finally obtained.

In this embodiment, for a group that constitutes a single molecule, the group may be further divided into multi-stage groups. Further, a primary group and a multi-stage group are determined in all groups of a single molecule; wherein all groups constituting the single molecule are taken as the primary group, and various groups which coexist and contribute to the same physical property in common are taken as the multi-stage group, and the number of the various groups is taken as a level of the multi-stage group; it can be used as a multi-stage group according to the simultaneous existence of multiple groups that will act together on the same physical property. Specifically, for example, when N6 and N4 groups exist separately in different molecules, they will have a certain impact on the physical properties, and when they exist in one molecule at the same time, on the basis of the original contribution to the physical properties, they make the contribution value of the physical properties fluctuated to a certain extent. The method of dividing the above-mentioned multi-stage groups may also be divided according to the chemical bond force between the groups according to the preset bond force interval. For different physical properties, the various chemical bond forces have different effects, which can be divided according to the impact of molecular stability on physical properties.

In another embodiment of the present disclosure, the acquiring the number of groups of each group constituting a sample single molecule includes:

determining a primary group, the number of groups of the primary group, a multi-stage group, and the number of groups of the multi-stage group in all groups of the single molecule;

taking all groups constituting the single molecule as the primary group; and

taking various groups which coexist and contribute to same physical property in common as the multi-stage group, and taking the number of the various groups as a level of the multi-stage group.

Model two: based on the divided multi-stage group, a property calculation model may be established as follows:

$$f = a + \sum_i \mathrm{m}_{1i} \Delta f_{1i} + \sum_j m_{2j} \Delta f_{2j} \ldots\ldots + \sum_l m_{Nl} \Delta f_{Nl} \quad ;$$

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

In another embodiment of the present disclosure, the acquiring the number of groups of each group constituting a single molecule includes:

determining a primary group, the number of groups of the primary group, a multi-stage group, and the number of groups of the multi-stage group in all groups of the single molecule;

taking all groups constituting the single molecule as the primary group; and

taking various groups which coexist and contribute to same physical property in common as the multi-stage group, and taking the number of the various groups as a level of the multi-stage group.

In addition to the general property calculation model described above, a property calculation model may be established for each property, respectively, depending on the type of property.

For example, the boiling point of a single molecule is calculated according to a property calculation model as follows:

$$T = \frac{SOL \times GROUP_1 + SOL \times GROUP_2 + \ldots\ldots + SOL \times GROUP_N}{(SOL \times Numh)^d + b} + c \quad ;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

Converting the single molecule vector according to the number of groups of each group constituting the single molecule includes: taking the number of species of all groups constituting a single molecule as a dimension of the single molecule vector; and taking the number of groups of each group as an element value of the corresponding dimension in the single molecule vector.

Converting the first contribution value vector according to a contribution value of each primary group of the single molecule to the boiling point includes: taking the number of types of primary groups as a dimension of the first contribution value vector; and taking the contribution value of each primary group to the boiling point as an element value of the corresponding dimension in the first contribution value vector. Converting the second contribution value vector according to a contribution value of each secondary group of the single molecule to the boiling point includes: taking the number of types of secondary groups as a dimension of the second contribution value vector; and taking the contribution value of each secondary group to the boiling point as an element value of the corresponding dimension in the second contribution value vector. By analogy, converting the N-th contribution value vector according to a contribution value of each N-stage group of the single molecule to the boiling point includes: taking the number of types of N-stage groups as a dimension of the N-th contribution value vector; and taking the contribution value of each N-stage group to the boiling point as an element value of the corresponding dimension in the N-th contribution value vector.

As another example, the density of the single molecule is calculated according to a property calculation model as follows:

$$D = \frac{SOL \times GROUP_{21}}{(SOL \times GROUP_{22} + \ldots\ldots + SOL \times GROUP_{2N}) \times e} ;$$

where, $D$ is the density of the single molecule, $SOL$ is a single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

Converting the single molecule vector converted according to the number of groups of each group constituting the single molecule includes: taking the number of species of all groups constituting a single molecule as a dimension of the single molecule vector; and taking the number of groups of each group as an element value of the corresponding dimension in the single molecule vector.

Converting the N+1-th contribution value vector according to a contribution value of each primary group of the single molecule to the density includes: taking the number of types of primary groups as a dimension of the N+1-th contribution value vector; and taking the contribution value of each primary group to the density as an element value of the corresponding dimension in the N+1-th contribution value vector. Converting the N+2-th contribution value vector according to a contribution value of each secondary group of the single molecule to the density includes: taking the number of types of secondary groups as a dimension of the N+2-th contribution value vector; and taking the contribution value of each secondary group to the density as an element value of the corresponding dimension in the N+2-th contribution value vector. By analogy, converting the 2N-th contribution value vector according to a contribution value of each N-stage group of the single molecule to the density includes: taking the number of types of N-stage groups as a dimension of the 2N contribution value vector; and taking the contribution value of each N-stage group to the density as an element value of the corresponding dimension in the 2N contribution value vector.

For example, the octane number of the single molecule is calculated according to a property calculation model as follows:

$$X = SOL \times GROUP_{31} + SOL \times GROUP_{32} + \ldots\ldots + SOL \times GROUP_{3N} + h ;$$

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUP_{32}$ is a 2N+2-th contribution value vector converted according to a contribution value of the secondary group to the octane number, $GROUP_{3N}$ is a 3N-ty contribution value vector converted according to a contribution value of the N-stage group to the octane number; $N$ is a positive integer greater than or equal to 2; and $h$ is the fifth preset constant.

Converting the single molecule vector according to the number of groups of each group constituting the single molecule includes: taking the number of species of all groups constituting a single molecule as a dimension of the single molecule vector; and taking the number of groups of each group as an element value of the corresponding dimension in the single molecule vector.

Converting the 2N+1-th contribution value vector according to a contribution value of each primary group of the single molecule to the octane number includes: taking the number of types of primary groups as a dimension of the 2N+1-th contribution value vector; and taking the contribution value of each primary group to the octane number as an element value of the corresponding dimension in the 2N+1-th contribution value vector. Converting the 2N+2-th contribution value vector according to a contribution value of each secondary group of the single molecule to the octane number includes: taking the number of types of secondary groups as a dimension of the 2N+2-th contribution value vector; and taking the contribution value of each secondary group to the octane number as an element value of the corresponding dimension in the 2N+2-th contribution value vector. By analogy, converting the 3N contribution value vector according to a contribution value of each N-stage group of the single molecule to the octane number includes: taking the number of types of N-stage groups as a dimension of the 3N contribution value vector; and taking the contribution value of each N-stage group to the octane number as an element value of the corresponding dimension in the 3N contribution value vector.

After the physical property of the corresponding single molecule is obtained by calculation in the above steps, the single molecule is taken as a template single molecule, and the number of groups and corresponding physical properties of each group constituting a single molecule are stored into the database.

The product property of the mixed product includes: Research Octane Number, Motor Octane Number, Reid vapor pressure, Enn's distillation range, density, benzene volume fraction, aromatics volume fraction, olefin volume fraction, oxygen mass fraction, and sulfur quality fraction.

Five manners to calculate the physical properties of a mixed product are provided below, but those skilled in the art should be appreciated that the following several manners are only used to illustrate the present embodiments and are not intended to limit the present embodiments.

Method one, when a product property of the mixed product is the density, the density of each of the mixed products is calculated according to a calculation formula as follows:

$$density = \sum (D_i \times x_{i\_volume}) ;$$

where, *density* is the density of the mixed product, $D_i$ is the density of the *i*-th single molecule, and $x_{i\_volume}$ is second component content of the *i*-th single molecule.

Method two, when a product property of the mixed product is the cloud point, calculating the product property of the mixed product includes:

calculating a cloud point contribution value of each single molecule according to the density and the boiling point of each single molecule; and
calculating the cloud point of the mixed product according to cloud point contribution values and content of all of the single molecules in the mixed product.

Method three, when a product property of the mixed product is the pour point, calculating the product property of the mixed product includes:

calculating a pour point contribution value of each single molecule according to the density and molecular weight of each single molecule; and
calculating the pour point of the mixed product according to pour point contribution values and content of all of the single molecules in the mixed product.

Method four, when a product property of the mixed product is the aniline point, calculating the product property of the mixed product includes:

calculating an aniline point contribution value of the single molecule according to the density and the boiling point of the single molecule; and
calculating the aniline point of the mixed product according to the aniline point contribution values and content of all of the single molecules in the mixed product.

Method five, when a product property of the mixed product is the octane number, a calculation method includes:

acquiring the octane number and content of each single molecule in the mixed product; and
calculating the octane number of the mixed product according to a calculation formula as follows:

$$\begin{aligned} ON = (&\sum_{i=HISQFG} \upsilon_i \beta_i ON_i + C_H \sum_{i=H} \upsilon_i \beta_i ON_i + C_I \sum_{i=I} \upsilon_i \beta_i ON_i + C_S \sum_{i=S} \upsilon_i \beta_i ON_i \\ &+ C_Q \sum_{i=Q} \upsilon_i \beta_i ON_i + C_F \sum_{i=F} \upsilon_i \beta_i ON_i + C_G \sum_{i=G} \upsilon_i \beta_i ON_i) \div \\ (&\sum_{i=HISQFG} \upsilon_i \beta_i + C_H (\sum_{i=H} \upsilon_i \beta_i - \sum_{i=H} \upsilon_i) + C_I (\sum_{i=I} \upsilon_i \beta_i - \sum_{i=I} \upsilon_i) + C_S (\sum_{i=S} \upsilon_i \beta_i - \sum_{i=S} \upsilon_i) \\ &+ C_Q (\sum_{i=Q} \upsilon_i \beta_i - \sum_{i=Q} \upsilon_i) + C_F (\sum_{i=F} \upsilon_i \beta_i - \sum_{i=F} \upsilon_i) + C_G (\sum_{i=G} \upsilon_i \beta_i - \sum_{i=G} \upsilon_i))) \end{aligned}$$

;

$$C_{\text{H}} = \frac{k_{\text{HI}}^{(a)}v_{\text{I}} + k_{\text{HS}}^{(a)}v_{\text{S}} + k_{\text{HQ}}^{(a)}v_{\text{Q}} + k_{\text{HF}}^{(a)}v_{\text{F}} + k_{\text{HG}}^{(a)}v_{\text{G}}}{1 + k_{\text{HI}}^{(b)}v_{\text{I}} + k_{\text{HS}}^{(b)}v_{\text{S}} + k_{\text{HQ}}^{(b)}v_{\text{Q}} + k_{\text{HF}}^{(b)}v_{\text{F}} + k_{\text{HG}}^{(b)}v_{\text{G}}} \, ;$$

$$C_{\text{I}} = \frac{k_{\text{HI}}^{(a)}v_{\text{H}} + k_{\text{IS}}^{(a)}v_{\text{S}} + k_{\text{IQ}}^{(a)}v_{\text{Q}} + k_{\text{IF}}^{(a)}v_{\text{F}} + k_{\text{IG}}^{(a)}v_{\text{G}}}{1 + k_{\text{HI}}^{(b)}v_{\text{H}} + k_{\text{IS}}^{(b)}v_{\text{S}} + k_{\text{IQ}}^{(b)}v_{\text{Q}} + k_{\text{IF}}^{(b)}v_{\text{F}} + k_{\text{IG}}^{(b)}v_{\text{G}}} \, ;$$

$$C_{\text{S}} = \frac{k_{\text{HS}}^{(a)}v_{\text{H}} + k_{\text{IS}}^{(a)}v_{\text{I}} + k_{SQ}^{(a)}v_{\text{Q}} + k_{SF}^{(a)}v_{\text{F}} + k_{SG}^{(a)}v_{\text{G}}}{1 + k_{\text{HS}}^{(b)}v_{\text{H}} + k_{\text{IS}}^{(b)}v_{\text{I}} + k_{\text{SQ}}^{(b)}v_{\text{Q}} + k_{\text{SF}}^{(b)}v_{\text{F}} + k_{\text{SG}}^{(b)}v_{\text{G}}} \, ;$$

$$C_{\text{Q}} = \frac{k_{HQ}^{(a)}v_{\text{H}} + k_{\text{IQ}}^{(a)}v_{\text{I}} + k_{SQ}^{(a)}v_{S} + k_{QF}^{(a)}v_{\text{F}} + k_{QG}^{(a)}v_{\text{G}}}{1 + k_{HQ}^{(b)}v_{\text{H}} + k_{\text{QI}}^{(b)}v_{\text{I}} + k_{\text{SQ}}^{(b)}v_{\text{S}} + k_{\text{QF}}^{(b)}v_{\text{F}} + k_{\text{QG}}^{(b)}v_{\text{G}}} \, ;$$

$$C_{\text{F}} = \frac{k_{\text{HF}}^{(a)}v_{\text{H}} + k_{\text{IF}}^{(a)}v_{\text{I}} + k_{\text{SF}}^{(a)}v_{\text{S}} + k_{QF}^{(a)}v_{Q} + k_{FG}^{(a)}v_{\text{G}}}{1 + k_{\text{HF}}^{(b)}v_{\text{H}} + k_{\text{IF}}^{(b)}v_{\text{I}} + k_{\text{SF}}^{(b)}v_{\text{S}} + k_{\text{QF}}^{(b)}v_{\text{Q}} + k_{\text{FG}}^{(b)}v_{\text{G}}} \, ;$$

$$C_{\text{G}} = \frac{k_{\text{HG}}^{(a)}v_{\text{H}} + k_{\text{IG}}^{(a)}v_{\text{I}} + k_{\text{SG}}^{(a)}v_{\text{S}} + k_{QG}^{(a)}v_{\text{Q}} + k_{FG}^{(a)}v_{\text{F}}}{1 + k_{HG}^{(b)}v_{\text{H}} + k_{\text{IG}}^{(b)}v_{\text{I}} + k_{\text{SG}}^{(b)}v_{\text{S}} + k_{\text{QG}}^{(b)}v_{\text{Q}} + k_{FG}^{(b)}v_{\text{F}}} \, ;$$

where, the ON is the octane number of the mixed products, the HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $v_i$ is content of each molecule in the mixed product, $v_{\text{H}}$, $v_{\text{I}}$, $v_{\text{S}}$, $v_{\text{Q}}$, $v_{\text{F}}$ and $v_{\text{G}}$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_{\text{H}}$ represents an interaction coefficient of n-alkanes with other molecules, $C_{\text{I}}$ represents an interaction coefficient of isoalkanes with other molecules; $C_{\text{S}}$ represents an interaction coefficient of cycloalkanes with other molecules; $C_{\text{Q}}$ represents an interaction coefficient of olefins with other molecules, $C_{\text{F}}$ represents an interaction coefficient of aromatic hydrocarbons with other molecules, $C_{\text{G}}$ represents an interaction coefficient of oxygenated compounds with other molecules, $k_{\text{HI}}^{(a)}$ represents a first constant coefficient between n-alkanes and isoalkanes, $k_{\text{HS}}^{(a)}$ represents a first constant coefficient between n-alkanes and cycloalkanes, $k_{\text{HQ}}^{(a)}$ represents a first constant coefficient between n-alkanes and olefins, $k_{\text{HF}}^{(a)}$ represents a first constant coefficient between n-alkanes and aromatic hydrocarbons, $k_{HG}^{(a)}$ represents a first constant coefficient between n-alkanes and oxygenated compounds, $k_{\text{IS}}^{(a)}$ represents a first constant coefficient between isoalkanes and cycloalkanes, $k_{\text{IQ}}^{(a)}$ represents a first constant coefficient between isoalkanes and olefins, $k_{\text{IF}}^{(a)}$ represents a first constant coefficient between isoalkanes and aromatic hydrocarbons, $k_{\text{IG}}^{(a)}$ represents a first constant coefficient between isoalkanes and oxygenated compounds, $k_{SQ}^{(a)}$ represents a first constant coefficient between cycloalkanes and olefins, $k_{\text{SF}}^{(a)}$ represents a first constant coefficient between cycloalkanes and aromatic hydrocarbons, $k_{\text{SG}}^{(a)}$ represents a first constant coefficient between cycloalkanes and oxygenated compounds,

$k_{QF}^{(a)}$ represents a first constant coefficient between olefins and aromatic hydrocarbons, $k_{QG}^{(a)}$ represents a second constant coefficient between olefins and oxygenated compounds, $k_{FG}^{(a)}$ represents a first constant coefficient between aromatic hydrocarbons and oxygenated compounds, $k_{HI}^{(b)}$ represents a second constant coefficient between n-alkanes and isoalkanes, $k_{HS}^{(b)}$ represents a second constant coefficient between n-alkanes and cycloalkanes, $k_{HQ}^{(b)}$ represents a second constant coefficient between n-alkanes and olefins, $k_{HF}^{(b)}$ represents a second constant coefficient between n-alkanes and aromatic hydrocarbons, $k_{HG}^{(b)}$ represents a second constant coefficient between n-alkanes and oxygenated compounds, $k_{IS}^{(b)}$ represents a second constant coefficient between isoalkanes and cycloalkanes, $k_{IQ}^{(b)}$ represents a second constant coefficient between isoalkanes and olefins, $k_{IF}^{(b)}$ represents a second constant coefficient between isoalkanes and aromatic hydrocarbons, $k_{IG}^{(b)}$ represents a second constant coefficient between isoalkanes and oxygenated compounds, $k_{SQ}^{(b)}$ represents a second constant coefficient between cycloalkanes and olefins, $k_{SF}^{(b)}$ represents a second constant coefficient between cycloalkanes and aromatic hydrocarbons, $k_{SG}^{(b)}$ represents a second constant coefficient between cycloalkanes and oxygenated compound, $k_{QF}^{(b)}$ represents a second constant coefficient between olefins and aromatic hydrocarbons, $k_{QG}^{(b)}$ represents a second constant coefficient between olefins and oxygenated compound, and $k_{FG}^{(b)}$ represents a second constant coefficient between aromatic hydrocarbons and oxygenated compound; wherein the octane number includes: a research octane number and a motor octane number.

A steps of training the product prediction model are described further below:

establishing a product prediction model; wherein the product prediction model includes: a set of reaction rules comprising a plurality of reaction rules and a reaction rate algorithm;
acquiring sample feedstock information for a sample feedstock;
training the set of reaction rules by using the sample feedstock information, and fixing the set of reaction rules that has been trained; and
training the reaction rate algorithm by using the sample feedstock information, and fixing the reaction rate algorithm that has been trained, to obtain the product prediction model that has been trained.

In the embodiments of the present disclosure, a corresponding product prediction model is established according to the type of petroleum processing devices.

The product prediction model corresponding to the petroleum processing device includes: a set of reaction rules and a reaction rate algorithm corresponding to the petroleum processing device. The set of reaction rules includes: a plurality of reaction rules corresponding to the petroleum processing devices.

The sample feedstock information of the sample feedstock includes: molecular composition of the sample feedstock, molecular content of each molecule in the sample feedstock, molecular composition of an actual product corresponding to the sample feedstock, and actual content of each molecule in the actual product. The actual product refers to the product obtained after the sample feedstock is processed by the petroleum processing device.

A way to train a set of reaction rules is given below.

The molecular composition of the sample feedstock according to a preset set of reaction rules, to obtain a reaction pathway corresponding to each molecule in the molecular composition of the sample feedstock; when the reaction pathway is firstly calculated, the molecular composition of the sample feedstock is processed in a set of preset reaction rules, to obtain a reaction pathway corresponding to each molecule in the molecular composition of the sample feedstock.

Each molecule in the sample feedstock is reacted according to reaction rules in a set of reaction rules, to obtain a reaction path corresponding to each molecule.

First molecule composition of a device output product comprising the sample feedstock, an intermediate product, and a predicted product are obtained according to the reaction path corresponding to each molecule in the molecular composition of the sample feedstock, in the device output product, comprising: the sample feedstock, the intermediate product, and the predicted product.

A first relative deviation is calculated according to the first molecular composition of the device output product and second molecular composition of the actual product.

If the first relative deviation meets a preset condition, the set of reaction rules is fixed.

If the first relative deviation does not meet the preset condition, a reaction rule in the set of reaction rules is adjusted, and the first relative deviation is recalculated according to the adjusted set of reaction rules until the first relative deviation meets the preset condition.

The calculating a first relative deviation according to the first molecular composition of the device output product and second molecular composition of the actual product specifically includes:

acquiring species of single molecules in the first molecule composition, to constitute a first set;
acquiring species of singles molecule in the second molecule composition, to constitute a second set;
determining whether the second set is a subset of the first set;
if the second set is not a subset of the first set, obtaining a pre-stored relative deviation value that does not meet the preset condition as the first relative deviation; and
if the second set is a subset of the first set, calculating the first relative deviation by a calculating formula as follows:

$$x_1 = \frac{\mathrm{card}\big((M - M_1 - M_2) - M_3\big)}{\mathrm{card}\ (M - M_1 - M_2)};$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card is the number of elements in the sets.

A way to train the reaction rate algorithm is given below. Those skilled in the art should be appreciated that this way is only used to illustrate the present embodiments and is not intended to limit the present embodiments.

Calculating a reaction rate of a reaction path corresponding to each molecule in the molecular composition of the sample feedstock, respectively, according to the reaction rate algorithm;

obtaining predicted content of each molecule in the predicted product corresponding to the sample feedstock according to molecular content of each molecule in the sample feedstock and the reaction rate of the reaction path corresponding to the molecule;
calculating a second relative deviation according to the predicted content of each molecule in the predicted product and the actual content of each molecule in the actual product;
if the second relative deviation meets a preset condition, fixing the reaction rate algorithm; and
if the second relative deviation does not meet the preset condition, adjusting a parameter in the reaction rate algorithm, and recalculating the second relative deviation according to the adjusted reaction rate algorithm until the second relative deviation meets the preset condition.

Specifically, a reaction rate of each reaction path is calculated according to a reaction rate constant in the reaction rate algorithm.

The reaction rate constant is determined according to a calculation formula as follows:

$$k = \frac{k_B E}{h} \exp\left(\frac{E \Delta S - \Delta E}{RE}\right) \varphi \times P^{\alpha};$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

Specifically, the reaction rate of the reaction path is obtained according to the reaction rate constant and the reaction concentration corresponding to the reaction path. For example, under the condition that the reaction rate constant has been determined, the larger the space velocity, the shorter the contact time between the feedstocks and the catalyst, the shorter the reaction time of the feedstocks, the higher the concentration of the reactant in the feedstocks, and the higher the reaction rate of the reaction path; on the contrary, the smaller the space velocity, the longer the contact time between the feedstocks and the catalyst, the longer the reaction time of the feedstocks, the lower the concentration of reactants in the feedstocks, and the lower the reaction rate of this reaction path.

In this embodiment, the reaction rate corresponding to each reaction path is calculated by the reaction rate calculation method in the pre-trained product prediction model, in combination with the single molecule content of each single molecule in the feedstock, the predicted content of each single molecule in the predicted product. For example, for the single molecule A in the feedstock, it is assumed that the single molecule A corresponds to three reaction paths, and the reaction rates corresponding to the three reaction paths are known; as the reaction proceeds, the concentration of the single molecule A decreases, and the reaction rates corresponding to the three reaction paths will decrease in proportion to the decrease in concentration, and thus single molecule A will generate products in proportion to the reaction rates of the three paths. According to the above steps, the product obtained by the reaction of each molecule may be obtained, and the predicted product may be obtained. When the single molecule content of each single molecule in the catalytic reforming feedstock is known, the content of each single molecule in the predicted product may be obtained.

In this embodiment, calculating the second relative deviation is, for example:

$$\text{The second relative deviation} = (\text{actual content} - \text{predicted content}) \div \text{actual content}.$$

The method provided in the present disclosure is performed by acquiring molecular composition of crude oil; acquiring molecular composition of various fractions obtained by distillation of the crude oil according to physical properties of various single molecules in the molecular composition of the crude oil; respectively inputting, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition; acquiring a preset standard set for a preset target product; determining whether the predicted product meets a preset standard for a target product corresponding to the predicted product in the preset standard set according to the predicted molecular composition of the predicted product and the predicted molecular content of each single molecule in the predicted molecular composition; and if the predicted product does not meet any preset standard for a target product corresponding to the predicted product in the preset standard set, adjusting an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set. By the above method, the embodiments of the present disclosure realize the molecular-level overall simulation and real-time optimization of the molecular-level device from the feedstocks to the product processing process, and improves the accuracy and production efficiency.

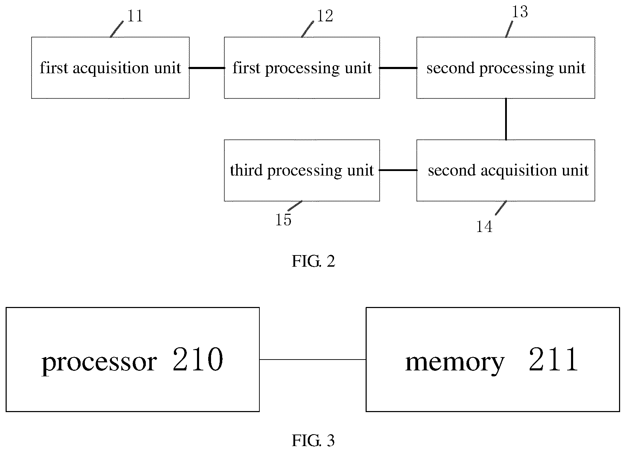

The embodiments of the present disclosure also provide an apparatus for real-time optimization of a molecular level device. As shown in FIG. 2, it is a schematic structural diagram of an apparatus for real-time optimization of a molecular level device according to an embodiment of the present disclosure. The apparatus for real-time optimization includes a first acquisition unit 11, a first processing unit 12, a second processing unit 13, a second acquisition unit 14 and a third processing unit 15.

In this embodiment, the first processing unit 12 is configured to acquire molecular composition of various fractions obtained by distillation of the crude oil according to physical properties of the various single molecules in the molecular composition of crude oil.

In this embodiment, the second processing unit 13 is configured to respectively input, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition.

In this embodiment, the second acquisition unit 14 is configured to acquire a preset standard set for a preset target product.

In this embodiment, the third processing unit 15 is configured to determine whether the predicted product meets a preset standard for a target product corresponding to the predicted product in the preset standard set according to the predicted molecular composition of the predicted product and the predicted molecular content of each single molecule in the predicted molecular composition, and if the predicted product does not meet any preset standard for a target product corresponding to the predicted product in the preset standard set, adjust an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set.

In this embodiment, the apparatus further includes: a flow control unit.

The flow control unit is configured to acquire an input flow of petroleum processing feedstocks input to each of the petroleum processing devices, determine whether each of the input flows meets a preset input flow range of the respective petroleum processing device, adjust the preset feedstock ratio if any one of the input flows does not meet the preset input flow range of the respective petroleum processing device, and respectively re-input, according to the adjusted preset feedstock ratio, the corresponding fractions into the pre-trained product prediction model of the respective petroleum processing device as petroleum processing feedstocks, until each of the input flows meets the preset input flow range of the respective petroleum processing device.

In this embodiment, the third processing unit 15, in particular, is configured to calculate a physical property of each single molecule in the predicted molecular composition according to the predicted molecular composition of the predicted product and the predicted molecular content of each single molecule in the predicted molecular composition, calculate a predicted physical property of the predicted product according to the physical property of each single molecule in the predicted molecular composition and the predicted molecular content of each single molecule in the predicted molecular composition, and determine whether the predicted physical property of each of the predicted products meet a preset physical property restriction interval of the corresponding target product in the preset standard set.

In this embodiment, the apparatus further includes a product blending unit.

The product blending unit is configured to blend each of the predicted products which is used as a product blending feedstock according to a preset rule set, to obtain molecular composition of a plurality of mixed products and content of each single molecule in each of the mixed products, and respectively calculate product property of each of the mixed products according to the molecular composition of each of the mixed products and the content of each single molecule in each of the mixed products.

In this embodiment, the third processing unit 15, in particular, is configured to determine whether the product property of each of the mixed products meets a preset product property of a target mixed product obtained by blending corresponding each target product in the preset standard set; if the preset product property is met, obtain a target parameter according to all of the mixed products and determining whether the target parameter meets a preset condition; if the target parameter meets the preset condition, determine that the predicted product meets a preset standard for a target product corresponding to the predicted product in the preset standard set, and output the preset feedstock ratio, the pre-trained product prediction model and the preset rule set as a production and processing scheme; and if the target parameter does not meet the preset condition, adjust the operation parameter in the pre-trained product prediction model and a preset rule in the preset rule set, to re-obtain a plurality of mixed products, until the product property of each of the mixed products meets the preset product property and the target parameters in all of the mixed products meet the preset condition.

In this embodiment, the third processing unit 15, in particular, is configured to acquire a product price of each of mixed products and a yield of each of mixed products, calculate a product benefit of each of mixed products according to the yield of each of mixed products and the product price of each of mixed products, accumulate the product benefit of each of mixed products to obtain a cumulative benefit, acquire a feedstock price of each group of the petroleum processing feedstocks and an operating cost of each of the petroleum processing devices, subtract feedstock prices of all of the petroleum processing feedstocks and operating costs of all of the petroleum processing devices from the cumulative benefit to obtain a comprehensive benefit, serve the comprehensive benefit as the target parameter, determine whether the comprehensive benefit reaches a maximum value, determine that the target parameter meets the preset condition if the comprehensive benefit reaches the maximum value; and determine that the target parameter does not meet the preset condition if the comprehensive benefit does not reach the maximum value.

In this embodiment, the third processing unit 15, in particular, is configured to adjust a temperature of an environment where a reaction path corresponding to the predicted product in the pre-trained product prediction model is located, and re-obtain the predicted molecular composition of the predicted product and the predicted molecular content of each single molecule in each of the predicted products according to the adjusted temperature until the predicted product meets the preset standard for the target product corresponding to the predicted product in the preset standard set.

In this embodiment, the third processing unit 15, in particular, is configured to adjust a pressure of an environment where a reaction path corresponding to the predicted product in the pre-trained product prediction model is located, and re-obtain the predicted molecular composition of the predicted product and the predicted molecular content of each single molecule in each of the predicted products according to the adjusted pressure until the predicted product meets the preset standard for the target product corresponding to the predicted product in the preset standard set.

In this embodiment, a product blending unit, in particular, is configured to acquire first molecular composition of each group of the product blending feedstocks and first component content of each single molecule in each group of the product blending feedstocks, based on the preset rule set, obtain second molecular composition of each of mixed products and second component content of each single molecule in each of mixed products according to the first molecular composition of each group of the product blending feedstocks and the first component content of each single molecule in each group of the product blending feedstocks, calculate a physical property of each single molecule in each of the mixed products according to the number of groups of each group contained in each single molecule in each of the mixed products and a contribution value of each group to the physical property, and calculate a product property of each of the mixed products according to the physical property and the second component content of each single molecule in each of the mixed products.

In this embodiment, the product blending unit, in particular, is configured to, for each single molecule, acquire the number of groups of each group constituting the single molecule and a contribution value of each group to the physical property, and input the number of groups of each group constituting the single molecule and the contribution value of each group to the physical property into a pre-trained property calculation model, to acquire the physical property of the single molecule outputted by the pre-trained property calculation model; wherein the pre-trained property calculation model is used to calculate the physical property of the single molecule according to the number of groups of each group contained in a single molecule and a contribution value of each group to the physical property.

In this embodiment, the apparatus further includes:

a single molecule property template matching unit configured to compare the number of groups of each group constituting the single molecule with molecular information of a template single molecule with known physical properties pre-stored in a database, the molecular information comprising the number of groups of each group constituting the template single molecule, determine whether there is a same template single molecule as the single molecule, if there is a same template single molecule as the single molecule, output the physical properties of the template single molecule as a physical property of the single molecule, and if there is not a same template single molecule as the single molecule, then, by the product blending unit, perform the step of the inputting the number of groups of each group constituting the single molecule and the contribution value of each group to the physical property into a pre-trained property calculation model.

In this embodiment, the apparatus further includes: a model training unit.

The model training unit is configured to construct a property calculation model of a single molecule, acquire the number of groups of each group constituting a sample single molecule; wherein the physical property of the sample single molecule is known, input the number of groups of each group constituting the sample single molecule into the property calculation model, acquire a predicted physical property of the sample single molecule output by the property calculation model, if a deviation value between the predicted physical property and the physical property which is known is less than a preset deviation threshold, determine that the property calculation model converges, acquire a contribution value of each group to the physical property in the property calculation model which is converged, and store the contribution value of the group to the physical property, and if the deviation value between the predicted physical property and the physical property which is known is greater than or equal to the deviation threshold, adjust a contribution value of each group to the physical property in the property calculation model until the property calculation model converges.

In this embodiment, the model training unit, in particular, is configured to establish the property calculation model as shown below:

$$f = a + \sum n_i \Delta f_i \; ;$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.

In this embodiment, the model training unit, in particular, is configured to determine a primary group, the number of groups of the primary group, a multi-stage group, and the number of groups of the multi-stage group in all groups of the single molecule, take all groups constituting the single molecule as the primary group, and take various groups which coexist and contribute to a same physical property in common as the multi-stage group, and taking the number of the various groups as a level of the multi-stage group.

In this embodiment, the model training unit, in particular, is configured to establish the property calculation model as shown below:

$$f = a + \sum_i \mathrm{m}_{1i} \Delta f_{1i} + \sum_j m_{2j} \Delta f_{2j} \ldots\ldots + \sum_l m_{Nl} \Delta f_{Nl} \; ;$$

where, $f$ is the physical property of the single molecule, $m_{1i}$ is the number of groups of the $l$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

In this embodiment, the product blending unit, in particular, is configured to determine a primary group, the number of groups of the primary group, a multi-stage group, and the number of groups of the multi-stage group in all groups of the single molecule, take all groups constituting the single molecule as the primary group, and take various groups which coexist and contribute to a same physical property in common as the multi-stage group, and taking the number of the various groups as a level of the multi-stage group.

In this embodiment, the product blending unit, in particular, is configured to calculate a boiling point of the single molecule according to a property calculation model as follows:

$$T = \frac{SOL \times GROUP_{11} + SOL \times GROUP_{12} + \ldots\ldots + SOL \times GROUP_{1N}}{(SOL \times Numh)^d + b} + c \quad ;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

In this embodiment, the product blending unit, in particular, is configured to calculate a density of the single molecule according to a property calculation model as follows:

$$D = \frac{SOL \times GROUP_{21}}{(SOL \times GROUP_{22} + \ldots\ldots + SOL \times GROUP_{2N}) \times e} \quad ,$$

where, $D$ is the density of the single molecule, $SOL$ is the single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

In this embodiment, the product blending unit, in particular, is configured to calculate an octane number of the single molecule according to a property calculation model as follows: $X = SOL \times GROUP_{31} + SOL \times GROUP_{32} + \ldots\ldots + SOL \times GROUP_{3N} + h$ ;

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUP_{32}$ is a 2N+2-th contribution value vector converted according to a contribution value of the secondary group to the octane number, $GROUP_{3N}$ is a 3N-ty contribution value vector converted according to a contribution value of the N-stage group to the octane number; $N$ is a positive integer greater than or equal to 2; and $h$ is the fifth preset constant.

In this embodiment, the product property of the mixed products includes a density, a cloud point, a pour point, an aniline point, and an octane number, and also includes other product property, and the other product property is not described herein redundantly.

In this embodiment, the product blending unit, in particular, is configured to calculate the density of each of the mixed products according to a calculation formula as follows: $density = \Sigma (D_i \times x_{i\_volume})$;

where, $density$ is the density of the mixed product, $D_i$ is the density of the $I$-th single molecule, and $x_{i\_volume}$ is second component content of the $i$-th single molecule.

In this embodiment, the product blending unit, in particular, is configured to, for each mixed product, calculate a cloud point contribution value of each single molecule according to the density and the boiling point of each single molecule in the mixed product, and calculate the cloud point of the mixed product according to cloud point contribution values of all of the single molecules and content of each single molecule in the mixed product.

In this embodiment, a product blending unit, in particular, is configured to, for each mixed product, calculate a pour point contribution value of each single molecule according to the density and molecular weight of each single molecule in the mixed product, and calculate the pour point of the mixed product according to pour point contribution values of all of the single molecules and content of each single molecule in the mixed product.

In this embodiment, the product blending unit, in particular, is configured to, for each mixed product, calculate an aniline point contribution value of each single molecule according to the density and the boiling point of each single molecule in the mixed product, and calculate the aniline point of the mixed product according to aniline point contribution values of all of the single molecules and content of each single molecule in the mixed product.

In this embodiment, the product blending unit, in particular, is configured to, for each mixed product, acquire the octane number of each single molecule and content of each single molecule in the mixed product, and calculate the octane number of the mixed products according to a calculation formula as follows:

$$
\mathrm{ON} = (\sum_{i=\mathrm{HISQFG}} \upsilon_i \beta_i \mathrm{ON}_i + \mathrm{C_H} \sum_{i=\mathrm{H}} \upsilon_i \beta_i \mathrm{ON}_i + \mathrm{C_I} \sum_{i=\mathrm{I}} \upsilon_i \beta_i \mathrm{ON}_i + \mathrm{C_S} \sum_{i=\mathrm{S}} \upsilon_i \beta_i \mathrm{ON}_i
$$

$$
+ \mathrm{C_Q} \sum_{i=\mathrm{Q}} \upsilon_i \beta_i \mathrm{ON}_i + \mathrm{C_F} \sum_{i=\mathrm{F}} \upsilon_i \beta_i \mathrm{ON}_i + \mathrm{C_G} \sum_{i=\mathrm{G}} \upsilon_i \beta_i \mathrm{ON}_i) \div
$$

$$
( \sum_{i=\mathrm{HISQFG}} \upsilon_i \beta_i + \mathrm{C_H}(\sum_{i=\mathrm{H}} \upsilon_i \beta_i - \sum_{i=\mathrm{H}} \upsilon_i) + \mathrm{C_I}(\sum_{i=\mathrm{I}} \upsilon_i \beta_i - \sum_{i=\mathrm{I}} \upsilon_i) + \mathrm{C_S}(\sum_{i=\mathrm{S}} \upsilon_i \beta_i - \sum_{i=\mathrm{S}} \upsilon_i)
$$

$$
+ \mathrm{C_Q}(\sum_{i=\mathrm{Q}} \upsilon_i \beta_i - \sum_{i=\mathrm{Q}} \upsilon_i) + \mathrm{C_F}(\sum_{i=\mathrm{F}} \upsilon_i \beta_i - \sum_{i=\mathrm{F}} \upsilon_i) + \mathrm{C_G}(\sum_{i=\mathrm{G}} \upsilon_i \beta_i - \sum_{i=\mathrm{G}} \upsilon_i))
$$
;

$$
\mathrm{C_H} = \frac{k_{\mathrm{HI}}^{(a)} \upsilon_\mathrm{I} + k_{\mathrm{HS}}^{(a)} \upsilon_\mathrm{S} + k_{\mathrm{HQ}}^{(a)} \upsilon_\mathrm{Q} + k_{\mathrm{HF}}^{(a)} \upsilon_\mathrm{F} + k_{\mathrm{HG}}^{(a)} \upsilon_\mathrm{G}}{1 + k_{\mathrm{HI}}^{(b)} \upsilon_\mathrm{I} + k_{\mathrm{HS}}^{(b)} \upsilon_\mathrm{S} + k_{\mathrm{HQ}}^{(b)} \upsilon_\mathrm{Q} + k_{\mathrm{HF}}^{(b)} \upsilon_\mathrm{F} + k_{\mathrm{HG}}^{(b)} \upsilon_\mathrm{G}} ;
$$

$$
\mathrm{C_I} = \frac{k_{\mathrm{HI}}^{(a)} \upsilon_\mathrm{H} + k_{\mathrm{IS}}^{(a)} \upsilon_\mathrm{S} + k_{\mathrm{IQ}}^{(a)} \upsilon_\mathrm{Q} + k_{\mathrm{IF}}^{(a)} \upsilon_\mathrm{F} + k_{\mathrm{IG}}^{(a)} \upsilon_\mathrm{G}}{1 + k_{\mathrm{HI}}^{(b)} \upsilon_\mathrm{H} + k_{\mathrm{IS}}^{(b)} \upsilon_\mathrm{S} + k_{\mathrm{IQ}}^{(b)} \upsilon_\mathrm{Q} + k_{\mathrm{IF}}^{(b)} \upsilon_\mathrm{F} + k_{\mathrm{IG}}^{(b)} \upsilon_\mathrm{G}} ;
$$

$$
\mathrm{C_S} = \frac{k_{\mathrm{HS}}^{(a)} \upsilon_\mathrm{H} + k_{\mathrm{IS}}^{(a)} \upsilon_\mathrm{I} + k_{SQ}^{(a)} \upsilon_\mathrm{Q} + k_{SF}^{(a)} \upsilon_\mathrm{F} + k_{SG}^{(a)} \upsilon_\mathrm{G}}{1 + k_{\mathrm{HS}}^{(b)} \upsilon_\mathrm{H} + k_{\mathrm{IS}}^{(b)} \upsilon_\mathrm{I} + k_{SQ}^{(b)} \upsilon_\mathrm{Q} + k_{SF}^{(b)} \upsilon_\mathrm{F} + k_{SG}^{(b)} \upsilon_\mathrm{G}} ;
$$

$$
\mathrm{C_Q} = \frac{k_{HQ}^{(a)} \upsilon_\mathrm{H} + k_{IQ}^{(a)} \upsilon_\mathrm{I} + k_{SQ}^{(a)} \upsilon_S + k_{QF}^{(a)} \upsilon_\mathrm{F} + k_{QG}^{(a)} \upsilon_\mathrm{G}}{1 + k_{HQ}^{(b)} \upsilon_\mathrm{H} + k_{QI}^{(b)} \upsilon_\mathrm{I} + k_{SQ}^{(b)} \upsilon_S + k_{QF}^{(b)} \upsilon_\mathrm{F} + k_{QG}^{(b)} \upsilon_\mathrm{G}} ;
$$

$$
\mathrm{C_F} = \frac{k_{\mathrm{HF}}^{(a)} \upsilon_\mathrm{H} + k_{\mathrm{IF}}^{(a)} \upsilon_\mathrm{I} + k_{SF}^{(a)} \upsilon_\mathrm{S} + k_{QF}^{(a)} \upsilon_Q + k_{FG}^{(a)} \upsilon_\mathrm{G}}{1 + k_{\mathrm{HF}}^{(b)} \upsilon_\mathrm{H} + k_{\mathrm{IF}}^{(b)} \upsilon_\mathrm{I} + k_{SF}^{(b)} \upsilon_\mathrm{S} + k_{QF}^{(b)} \upsilon_Q + k_{FG}^{(b)} \upsilon_\mathrm{G}} ;
$$

$$
\mathrm{C_G} = \frac{k_{\mathrm{HG}}^{(a)} \upsilon_\mathrm{H} + k_{\mathrm{IG}}^{(a)} \upsilon_\mathrm{I} + k_{SG}^{(a)} \upsilon_\mathrm{S} + k_{QG}^{(a)} \upsilon_Q + k_{FG}^{(a)} \upsilon_\mathrm{F}}{1 + k_{HG}^{(b)} \upsilon_\mathrm{H} + k_{IG}^{(b)} \upsilon_\mathrm{I} + k_{SG}^{(b)} \upsilon_\mathrm{S} + k_{QG}^{(b)} \upsilon_Q + k_{FG}^{(b)} \upsilon_\mathrm{F}} ;
$$

where, ON is the octane number of the mixed product, HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $\upsilon_i$ is content of each molecule in the mixed product, $\upsilon_\mathrm{H}$, $\upsilon_I$, $\upsilon_\mathrm{S}$, $\upsilon_\mathrm{Q}$, $\upsilon_\mathrm{F}$ and $\upsilon_\mathrm{G}$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_\mathrm{H}$ is an interaction coefficient of n-alkanes with other molecules, $C_\mathrm{I}$ is an interaction coefficient of isoalkanes with other molecules; $C_\mathrm{S}$ is an interaction coefficient of cycloalkanes with other molecules; $C_\mathrm{Q}$ is an interaction coefficient of olefins with other molecules, $C_\mathrm{F}$ is an interaction coefficient of aromatic hydrocarbons with other molecules, $C_\mathrm{G}$ is an interaction coefficient of oxygenated compounds with other molecules, $k_{\mathrm{HI}}^{(a)}$ is a first constant coefficient between n-alkanes and isoalkanes, $k_{\mathrm{HS}}^{(a)}$ is a first constant coefficient between n-alkanes and cycloalkanes, $k_{\mathrm{HQ}}^{(a)}$ is a first constant coefficient between n-alkanes and olefins, $k_{\mathrm{HF}}^{(a)}$ is a first constant coefficient between n-alkanes and aromatic hydrocarbons, $k_{HG}^{(a)}$ is a first constant coefficient between n-alkanes and oxygenated compounds, $k_{IS}^{(a)}$ is a first constant coefficient between isoalkanes and cycloalkanes, $k_{IQ}^{(a)}$ is a first constant coefficient between isoalkanes and olefins, $k_{IF}^{(a)}$ is a first constant coefficient between isoalkanes and aromatic hydrocarbons, $k_{IG}^{(a)}$ is a first constant coefficient between isoalkanes and oxygenated compounds, $k_{SQ}^{(a)}$ is a first constant coefficient between cycloalkanes and olefins, $k_{SF}^{(a)}$ is a first constant coefficient between cycloalkanes and aromatic hydrocarbons, $k_{SG}^{(a)}$ is a first constant coefficient between cycloalkanes and oxygenated compounds, $k_{QF}^{(a)}$ is a first constant coefficient between olefins and aromatic hydrocarbons, $k_{QG}^{(a)}$ is a second constant coefficient between olefins and oxygenated compounds, $k_{FG}^{(a)}$ is a first constant coefficient between aromatic hydrocarbons and oxygenated compounds, $k_{HI}^{(b)}$ is a second constant coefficient between n-alkanes and isoalkanes, $k_{HS}^{(b)}$ is a second constant coefficient between n-alkanes and cycloalkanes, $k_{HQ}^{(b)}$ is a second constant coefficient between n-alkanes and olefins, $k_{HF}^{(b)}$ is a second constant coefficient between n-alkanes and aromatic hydrocarbons, $k_{HG}^{(b)}$ is a second constant coefficient between n-alkanes and oxygenated compounds, $k_{IS}^{(b)}$ is a second constant coefficient between isoalkanes and cycloalkanes, $k_{IQ}^{(b)}$ is a second constant coefficient between isoalkanes and olefins, $k_{IF}^{(b)}$ is a second constant coefficient between isoalkanes and aromatic hydrocarbons, $k_{IG}^{(b)}$ is a second constant coefficient between isoalkanes and oxygenated compounds, $k_{SQ}^{(b)}$ is a second constant coefficient between cycloalkanes and olefins, $k_{SF}^{(b)}$ is a second constant coefficient between cycloalkanes and aromatic hydrocarbons, $k_{SG}^{(b)}$ is a second constant coefficient between cycloalkanes and oxygenated compound, $k_{QF}^{(b)}$ is a second constant coefficient between olefins and aromatic hydrocarbons, $k_{QG}^{(b)}$ is a second constant coefficient between olefins and oxygenated compound, and $k_{FG}^{(b)}$ is a second constant coefficient between aromatic hydrocarbons and oxygenated compound; wherein the octane number includes: a research octane number and a motor octane number.

In this embodiment, the apparatus further includes: a model training unit.

The model training unit is configure to establish a product prediction model; wherein the product prediction model includes: a set of reaction rules comprising a plurality of reaction rules and a reaction rate algorithm, acquire sample feedstock information for a sample feedstock, train the set of reaction rules by using the sample feedstock information, and fix the set of reaction rules that has been trained, and train the reaction rate algorithm by using the sample feedstock information, and fix the reaction rate algorithm that has been trained, to obtain the product prediction model that has been trained.

In this embodiment, the sample feedstock information of the sample feedstock includes: molecular composition of the sample feedstock, molecular content of each molecule in the sample feedstock, molecular composition of an actual product corresponding to the sample feedstock, and actual content of each molecule in the actual product.

In this embodiment, the model training unit, in particular, is configured to process the molecular composition of the sample feedstock according to a preset set of reaction rules, to obtain a reaction pathway corresponding to each molecule in the molecular composition of the sample feedstock, obtain first molecule composition of a device output product comprising the sample feedstock, an intermediate product, and a predicted product according to the reaction path corresponding to each molecule in the molecular composition of the sample feedstock; in the device output product, comprising: the sample feedstock, the intermediate product, and the predicted product, calculate a first relative deviation according to the first molecular composition of the device output product and second molecular composition of the actual product, if the first relative deviation meets a preset condition, fix the set of reaction rules, and if the first relative deviation does not meet the preset condition, adjust a reaction rule in the set of reaction rules, and recalculate the first relative deviation according to the adjusted set of reaction rules until the first relative deviation meets the preset condition.

**In** this embodiment, the model training unit, in particular, is configured to acquire species of single molecules in the first molecule composition, to constitute a first set, acquire species of single molecules in the second molecule composition, to constitute a second set, determine whether the second set is a subset of the first set, if the second set is not a subset of the first set, obtain a pre-stored relative deviation value that does not meet the preset condition as the first relative deviation, and if the second set is a subset of the first set, calculate the first relative deviation by a calculating formula as follows:

$$x_1 = \frac{\mathrm{card}\big((M - M_1 - M_2) - M_3\big)}{\mathrm{card}\ (M - M_1 - M_2)} \ ;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card is the number of elements in the sets.

In this embodiment, the model training unit, in particular, is configured to calculate a reaction rate of a reaction path corresponding to each molecule in the molecular composition of the sample feedstock, respectively, according to the reaction rate algorithm, obtain predicted content of each molecule in the predicted product corresponding to the sample feedstock according to molecular content of each molecule in the sample feedstock and the reaction rate of the reaction path corresponding to the molecule, calculate a second relative deviation according to the predicted content of each molecule in the predicted product and the actual content of each molecule in the actual product, if the second relative deviation meets a preset condition, fix the reaction rate algorithm, and if the second relative deviation does not meet the preset condition, adjust a parameter in the reaction rate algorithm, and recalculate the second relative deviation according to the adjusted reaction rate algorithm until the second relative deviation meets the preset condition.

In this embodiment, the model training unit, in particular, is configured to calculate a reaction rate of each reaction path according to a reaction rate constant in the reaction rate algorithm;

wherein the reaction rate constant is determined according to a calculation formula as follows:

$$k = \frac{k_B E}{h} \exp(\frac{E\Delta S - \Delta E}{RE})\varphi \times P^\alpha \ ;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

In this embodiment, types of the petroleum processing devices include: a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit; wherein each petroleum processing device corresponds to a set of reaction rules.

The embodiments of the present disclosure also provide a system for real-time optimization of a molecular level device. As shown in FIG. 3, it is a structural diagram of a system for real-time optimization of a molecular level device according to another embodiment of the present disclosure.

In the embodiment of the present disclosure, the system for real-time optimization of the molecular level device includes a processor 210 and a memory 211, wherein the processor 210 is configured to execute a real-time optimization program of the molecular level device stored in the memory 211 to implement a method for real-time optimization of the molecular level device described in method embodiments; for example, the method includes the following steps: acquiring molecular composition of crude oil; acquiring molecular composition of various fractions obtained by distillation of the crude oil according to physical properties of various single molecules in the molecular composition of the crude oil; respectively inputting, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition; acquiring a preset standard set for a preset target product; determining whether the predicted product meets a preset standard for a target product corresponding to the predicted product in the preset standard set according to the predicted molecular composition of the predicted product and the predicted molecular content of each single molecule in the predicted molecular composition; and if the predicted product does not meet any preset standard for a target product corresponding to the predicted product in the preset standard set, adjusting an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set.

The embodiments of the present disclosure also provide a computer-readable storage medium having stored thereon one or more programs, wherein the storage medium may include volatile memory, such as random access memory; the memory may also include non-volatile memory, such as read-only memory, flash memory, hard disk, or solid-state memory; the memory may also include a combination of the above types of memory.

When the one or more programs in the storage medium is/are executable by one or more processors to implement a method for real-time optimization of the molecular level device described in method embodiments; for example, the method includes the following steps:

acquiring molecular composition of crude oil; acquiring molecular composition of various fractions obtained by distillation of the crude oil according to physical properties of various single molecules in the molecular composition of the crude oil; respectively inputting, according to a preset feedstock ratio, the corresponding fractions into a pre-trained product prediction model corresponding to a petroleum processing device as petroleum processing feedstocks, to obtain predicted molecular composition of a corresponding predicted product output by the pre-trained product prediction model and predicted molecular content of each single molecule in the predicted molecular composition; acquiring a preset standard for a preset target product; determining whether the predicted product meets a preset standard for a target product corresponding to the predicted product in the preset standard set according to the predicted molecular composition of the predicted product and the predicted molecular content of each single molecule in the predicted molecular composition; and if the predicted product does not meet any preset standard for a target product corresponding to the predicted product in the preset standard set, adjusting an operation parameter in the pre-trained product prediction model, to re-obtain predicted molecular composition of the predicted product and predicted molecular content of each single molecule in the predicted molecular composition, until the predicted product meets the preset standard for the target product corresponding to the predicted product in the preset standard set.

For ease of description, the above devices are described as being functionally divided into various units. Of course, the functionality of the various units may be implemented in the same or multiple software and/or hardware when implementing the present disclosure.

The various embodiments in this specification have been described in a recursive manner, and similar parts between the various embodiments are described with reference to each other, with each being illustrated as being distinct from the other embodiments. **In** particular, for the purpose of device or system embodiments, since they are substantially similar to the method embodiments, portions of the description that are relatively simple and related to the method embodiments are described. The apparatus and system embodiments described above are illustrative only, wherein the elements illustrated as discrete components may or may not be physically separate, components shown as units may or may not be physical units, i.e., may be located in one place or may be distributed across multiple network elements. Some or all of the modules therein may be selected according to the needs of the target to accomplish the objectives of the present embodiment scheme. One of ordinary skill in the art will understand and implement without the exercise of inventive faculty.

It is noted that relational terms such as "first" and "second" and the like, herein are used solely to distinguish one entity or operation from another entity or operation without necessarily requiring or implying any such relationship or order between such entities or operations. Furthermore, the terms "comprise", "include" or any other variation thereof, are intended to cover a non-exclusive inclusion, such that a process, method, article, or apparatus that comprises a list of elements does not include only those elements but may include other elements not expressly listed or inherent to such process, method, article, or apparatus. An element defined by a statement "including one ..." does not, without more limitation, preclude the existence of additional identical elements in the process, method, article, or apparatus that comprises the element.

**Claims**

1. A computer-implemented method for obtaining a production and processing scheme of petroleum processing, wherein the method comprises:

    acquiring molecular composition of crude oil;
    acquiring molecular composition of various fractions obtained by distillation of the crude oil according to physical properties of various single molecules in the molecular composition of the crude oil;

serving the corresponding fractions as petroleum processing feedstocks, and respectively inputting, according to a preset feedstock ratio, the molecular composition of the corresponding fractions into a pre-trained product prediction model corresponding to a petroleum processing device, to obtain a predicted molecular composition of a corresponding predicted product output by the pre-trained product prediction model and a predicted molecular content of each single molecule in the predicted molecular composition;

blending each of the predicted products, which is used as a product blending feedstock according to a preset rule set, to obtain a molecular composition of a plurality of mixed products and content of each single molecule in each of the mixed products, wherein each set of preset rules in the preset rule set includes the type and amount of the predicted product used;

respectively calculating a product property of each of the mixed products according to the molecular composition of each of the mixed products and the content of each single molecule in each of the mixed products;

acquiring a preset standard set for a preset target product;

determining whether the product property of each of the mixed products meets a preset product property of a target mixed product obtained by blending each corresponding target product in the preset standard set;

if the preset product property is met:

obtaining a target parameter according to all of the mixed products and determining whether the target parameter meets a preset condition, wherein the target parameter represents: a content of substances in the product that will cause harm to the environment; or a proportion of products, which meet a preset standard, in all mixed products to all of the mixed products;

if the target parameter meets the preset condition, determining that the predicted product meets a preset standard for a target product corresponding to the predicted product in the preset standard set, and outputting the preset feedstock ratio, the pre-trained product prediction model and the preset rule set as a production and processing scheme, and using the production and processing scheme to conduct petroleum processing in an actual production process; and

if the target parameter does not meet the preset condition adjusting an operation parameter in the pre-trained product prediction model and a preset rule in the preset rule set, to re-obtain a plurality of mixed products until the product property of each of the mixed products meets the preset product property and the target parameters in all of the mixed products meet the preset condition;

wherein the operation parameter in the pre-trained product prediction model comprises: a temperature of an environment in which a reaction path in the pre-trained product prediction model is located, a pressure of an environment in which a reaction path in the pre-trained product prediction model is located, and space velocity.

2. The method according to claim 1, wherein the method further comprises:

acquiring an input flow of petroleum processing feedstocks input to each of the petroleum processing devices;

determining whether each of the input flows meets a preset input flow range of the respective petroleum processing device; and

adjusting the preset feedstock ratio if any one of the input flows does not meet the preset input flow range of the respective petroleum processing device, and respectively re-inputting, according to the adjusted preset feedstock ratio, the corresponding fractions into the pre-trained product prediction model of the respective petroleum processing device as petroleum processing feedstocks, until each of the input flows meets the preset input flow range of the respective petroleum processing device.

3. The method according to claim 1, wherein the adjusting an operation parameter in the pre-trained product prediction model comprises:

adjusting a temperature of an environment where a reaction path corresponding to the predicted product in the pre-trained product prediction model is located; and

re-obtaining the predicted molecular composition of the predicted product and the predicted molecular content of each single molecule in each of the predicted products according to the adjusted temperature.

4. The method according to claim 1, wherein the adjusting an operation parameter in the pre-trained product prediction model comprises:

adjusting a pressure of an environment where a reaction path corresponding to the predicted product in the pre-trained product prediction model is located; and

re-obtaining the predicted molecular composition of the predicted product and the predicted molecular content of each single molecule in each of the predicted products according to the adjusted pressure.

5. The method according to claim 1, wherein the respectively calculating product property of each of the mixed products according to the molecular composition of each of the mixed products and the content of each single molecule in each of the mixed products comprises:

acquiring first molecular composition of each set of the product blending feedstocks and first component content of each single molecule in each set of the product blending feedstocks;

based on the preset rule set, obtaining second molecular composition of each of mixed products and second component content of each single molecule in each of mixed products according to the first molecular composition of each set of the product blending feedstocks and the first component content of each single molecule in each set of the product blending feedstocks;

calculating a physical property of each single molecule in each of the mixed products according to the number of groups of each group contained in each single molecule in each of the mixed products and a contribution value of each group to the physical property; and

calculating a product property of each of the mixed products according to the physical property and the second component content of each single molecule in each of the mixed products.

6. The method according to claim 5, wherein calculation of the physical property of each single molecule comprises:

for each single molecule, acquiring the number of groups of each group constituting the single molecule and a contribution value of each group to the physical property; and

inputting the number of groups of each group constituting the single molecule and the contribution value of each group to the physical property into a pre-trained property calculation model, to acquire the physical property of the single molecule outputted by the pre-trained property calculation model;

wherein the pre-trained property calculation model is used to calculate the physical property of the single molecule according to the number of groups of each group contained in a single molecule and a contribution value of each group to the physical property.

7. The method according to claim 6, wherein, before the inputting the number of groups of each group constituting the single molecule and the contribution value of each group to the physical property into a pre-trained property calculation model, the method further comprises:

comparing the number of groups of each group constituting the single molecule with molecular information of a template single molecule with known physical properties pre-stored in a database, the molecular information comprising the number of groups of each group constituting the template single molecule;

determining whether there is a same template single molecule as the single molecule;

if there is a same template single molecule as the single molecule, outputting the physical properties of the template single molecule as a physical property of the single molecule; and

if there is not a same template single molecule as the single molecule, then performing the step of the inputting the number of groups of each group constituting the single molecule and the contribution value of each group to the physical property into a pre-trained property calculation model.

8. The method according to claim 6 or 7, wherein a step of training the property calculation model comprises:

constructing a property calculation model of a single molecule;

acquiring the number of groups of each group constituting a sample single molecule; wherein the physical property of the sample single molecule is known;

inputting the number of groups of each group constituting the sample single molecule into the property calculation model;

acquiring a predicted physical property of the sample single molecule output by the property calculation model;

if a deviation value between the predicted physical property and the physical property which is known is less than a preset deviation threshold, determining that the property calculation model converges, acquiring a contribution value of each group to the physical property in the property calculation model which is converged, and storing the contribution value of the group to the physical property; and

if the deviation value between the predicted physical property and the physical property which is known is greater than or equal to the deviation threshold, adjusting a contribution value of each group to the physical property in the property calculation model until the property calculation model converges.

9. The method according to claim 8, wherein the property calculation model is established as shown below:

$$f = a + \sum n_i \Delta f_i \,;$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.

10. The method according to claim 8, wherein the acquiring the number of groups of each group constituting a sample single molecule comprises:

determining a primary group, the number of groups of the primary group, a multi-stage group, and the number of groups of the multi-stage group in all groups of the single molecule;
taking all groups constituting the single molecule as the primary group; and
taking various groups which coexist and contribute to a same physical property in common as the multi-stage group, and taking the number of the various groups as a level of the multi-stage group.

11. The method according to claim 10, wherein,

the property calculation model is established as shown below:

$$f = a + \sum_i \mathrm{m}_{1i} \Delta f_{1i} + \sum_j m_{2j} \Delta f_{2j} \ldots\ldots + \sum_l m_{Nl} \Delta f_{Nl} \,;$$

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

12. The method according to claim 7, wherein the acquiring the number of groups of each group constituting the single molecule comprises:

determining a primary group, the number of groups of the primary group, a multi-stage group, and the number of groups of the multi-stage group in all groups of the single molecule;
taking all groups constituting the single molecule as the primary group; and
taking various groups which coexist and contribute to a same physical property in common as the multi-stage group, and taking the number of the various groups as a level of the multi-stage group.

13. The method according to claim 12, wherein,

the physical property of the single molecule comprises a boiling point of a single molecule;
the calculating the physical property of the single molecule comprises: calculating the boiling point of the single molecule according to a property calculation model as follows:

$$T = \frac{SOL \times GROUP_{11} + SOL \times GROUP_{12} + \ldots\ldots + SOL \times GROUP_{1N}}{(SOL \times \mathrm{Numh})^d + b} + c \,;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point,

$GROUP_{1N}$ is an N-th contribution value vector converted according to a contribution value of the N-stage group to the boiling point, $N$umh is the number of atoms other than the hydrogen atom in the single molecule, $d$ is a first preset constant, $b$ is a second preset constant, $c$ is a third preset constant, and $N$ is a positive integer greater than or equal to 2.

**14.** The method according to claim 12, wherein,

the physical property of the single molecule comprises a density of a single molecule;
the calculating the physical property of the single molecule comprises:

calculating the density of the single molecule according to a property calculation model as follows:

$$D = \frac{SOL \times GROUP_{21}}{(SOL \times GROUP_{22} + \ldots\ldots + SOL \times GROUP_{2N}) \times e} \;,$$

where, $D$ is the density of the single molecule, $SOL$ is a single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

**15.** The method according to claim 12, wherein,

the physical property of the single molecule comprises an octane number of a single molecule;
the calculating the physical property of the single molecule comprises:

calculating the octane number of the single molecule according to a property calculation model as follows:

$$X = SOL \times GROUP_{31} + SOL \times GROUP_{32} + \ldots\ldots + SOL \times GROUP_{3N} + h \;,$$

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUP_{32}$ is a 2N+2-th contribution value vector converted according to a contribution value of the secondary group to the octane number, $GROUP_{3N}$ is a 3N-ty contribution value vector converted according to a contribution value of the N-stage group to the octane number; $N$ is a positive integer greater than or equal to 2; and $h$ is the fifth preset constant.

**16.** The method according to claim 1, wherein,
the product property of the mixed products comprises a density, a cloud point, a pour point, an aniline point, and an octane number.

**17.** The method according to claim 16, wherein, when a product property of the mixed product is the density, calculating the product property of each of the mixed products comprises:

calculating the density of each of the mixed products according to a calculation formula as follows:

$$density = \sum \left( D_i \times x_{i\_volume} \right) \;;$$

where, $density$ is the density of the mixed product, $D_i$ is the density of the $i$-th single molecule, and $x_{i\_volume}$ is second component content of the $i$-th single molecule.

**18.** The method according to claim 16, wherein, when a product property of the mixed product is the cloud point, calculating the product property of each of the mixed products comprises:

for each mixed product, calculating a cloud point contribution value of each single molecule according to the density and the boiling point of each single molecule in the mixed product; and

calculating the cloud point of the mixed product according to cloud point contribution values of all of the single molecules and content of each single molecule in the mixed product.

19. The method according to claim 16, wherein, when a product property of the mixed product is the pour point, calculating the product property of each of the mixed products comprises:

for each mixed product, calculating a pour point contribution value of each single molecule according to the density and molecular weight of each single molecule in the mixed product; and

calculating the pour point of the mixed product according to pour point contribution values of all of the single molecules and content of each single molecule in the mixed product.

20. The method according to claim 16, wherein, when a product property of the mixed product is the aniline point, calculating the product property of each of the mixed products comprises:

for each mixed product, calculating an aniline point contribution value of each single molecule according to the density and the boiling point of each single molecule in the mixed product; and

calculating the aniline point of the mixed product according to aniline point contribution values of all of the single molecules and content of each single molecule in the mixed product.

21. The method according to claim 16, wherein, when a product property of the mixed product is the octane number, calculating the product property of each of the mixed products comprises:

for each mixed product, acquiring the octane number of each single molecule and content of each single molecule in the mixed product; and

calculating the octane number of the mixed products according to a calculation formula as follows:

$$
\begin{aligned}
\mathrm{ON} = ( &\sum_{i=\mathrm{HISQFG}} \upsilon_i \beta_i \mathrm{ON}_i + \mathrm{C}_\mathrm{H} \sum_{i=\mathrm{H}} \upsilon_i \beta_i \mathrm{ON}_i + \mathrm{C}_\mathrm{I} \sum_{i=\mathrm{I}} \upsilon_i \beta_i \mathrm{ON}_i + \mathrm{C}_\mathrm{S} \sum_{i=\mathrm{S}} \upsilon_i \beta_i \mathrm{ON}_i \\
&+ \mathrm{C}_\mathrm{Q} \sum_{i=\mathrm{Q}} \upsilon_i \beta_i \mathrm{ON}_i + \mathrm{C}_\mathrm{F} \sum_{i=\mathrm{F}} \upsilon_i \beta_i \mathrm{ON}_i + \mathrm{C}_\mathrm{G} \sum_{i=\mathrm{G}} \upsilon_i \beta_i \mathrm{ON}_i ) \div \\
( &\sum_{i=\mathrm{HISQFG}} \upsilon_i \beta_i + \mathrm{C}_\mathrm{H} (\sum_{i=\mathrm{H}} \upsilon_i \beta_i - \sum_{i=\mathrm{H}} \upsilon_i) + \mathrm{C}_\mathrm{I} (\sum_{i=\mathrm{I}} \upsilon_i \beta_i - \sum_{i=\mathrm{I}} \upsilon_i) + \mathrm{C}_\mathrm{S} (\sum_{i=\mathrm{S}} \upsilon_i \beta_i - \sum_{i=\mathrm{S}} \upsilon_i) \\
&+ \mathrm{C}_\mathrm{Q} (\sum_{i=\mathrm{Q}} \upsilon_i \beta_i - \sum_{i=\mathrm{Q}} \upsilon_i) + \mathrm{C}_\mathrm{F} (\sum_{i=\mathrm{F}} \upsilon_i \beta_i - \sum_{i=\mathrm{F}} \upsilon_i) + \mathrm{C}_\mathrm{G} (\sum_{i=\mathrm{G}} \upsilon_i \beta_i - \sum_{i=\mathrm{G}} \upsilon_i))
\end{aligned} ;
$$

$$
\mathrm{C}_\mathrm{H} = \frac{k_{\mathrm{HI}}^{(a)} \upsilon_\mathrm{I} + k_{\mathrm{HS}}^{(a)} \upsilon_\mathrm{S} + k_{\mathrm{HQ}}^{(a)} \upsilon_\mathrm{Q} + k_{\mathrm{HF}}^{(a)} \upsilon_\mathrm{F} + k_{\mathrm{HG}}^{(a)} \upsilon_\mathrm{G}}{1 + k_{\mathrm{HI}}^{(b)} \upsilon_\mathrm{I} + k_{\mathrm{HS}}^{(b)} \upsilon_\mathrm{S} + k_{\mathrm{HQ}}^{(b)} \upsilon_\mathrm{Q} + k_{\mathrm{HF}}^{(b)} \upsilon_\mathrm{F} + k_{\mathrm{HG}}^{(b)} \upsilon_\mathrm{G}} ;
$$

$$
\mathrm{C}_\mathrm{I} = \frac{k_{\mathrm{HI}}^{(a)} \upsilon_\mathrm{H} + k_{\mathrm{IS}}^{(a)} \upsilon_\mathrm{S} + k_{\mathrm{IQ}}^{(a)} \upsilon_\mathrm{Q} + k_{\mathrm{IF}}^{(a)} \upsilon_\mathrm{F} + k_{\mathrm{IG}}^{(a)} \upsilon_\mathrm{G}}{1 + k_{\mathrm{HI}}^{(b)} \upsilon_\mathrm{H} + k_{\mathrm{IS}}^{(b)} \upsilon_\mathrm{S} + k_{\mathrm{IQ}}^{(b)} \upsilon_\mathrm{Q} + k_{\mathrm{IF}}^{(b)} \upsilon_\mathrm{F} + k_{\mathrm{IG}}^{(b)} \upsilon_\mathrm{G}} ;
$$

$$
\mathrm{C}_\mathrm{S} = \frac{k_{\mathrm{HS}}^{(a)} \upsilon_\mathrm{H} + k_{\mathrm{IS}}^{(a)} \upsilon_\mathrm{I} + k_{SQ}^{(a)} \upsilon_\mathrm{Q} + k_{SF}^{(a)} \upsilon_\mathrm{F} + k_{\mathrm{SG}}^{(a)} \upsilon_\mathrm{G}}{1 + k_{\mathrm{HS}}^{(b)} \upsilon_\mathrm{H} + k_{\mathrm{IS}}^{(b)} \upsilon_\mathrm{I} + k_{\mathrm{SQ}}^{(b)} \upsilon_\mathrm{Q} + k_{\mathrm{SF}}^{(b)} \upsilon_\mathrm{F} + k_{\mathrm{SG}}^{(b)} \upsilon_\mathrm{G}} ;
$$

$$
\mathrm{C}_\mathrm{Q} = \frac{k_{HQ}^{(a)} \upsilon_\mathrm{H} + k_{\mathrm{IQ}}^{(a)} \upsilon_\mathrm{I} + k_{\mathrm{SQ}}^{(a)} \upsilon_\mathrm{S} + k_{\mathrm{QF}}^{(a)} \upsilon_\mathrm{F} + k_{\mathrm{QG}}^{(a)} \upsilon_\mathrm{G}}{1 + k_{\mathrm{HQ}}^{(b)} \upsilon_\mathrm{H} + k_{\mathrm{IQ}}^{(b)} \upsilon_\mathrm{I} + k_{\mathrm{SQ}}^{(b)} \upsilon_\mathrm{S} + k_{\mathrm{QF}}^{(b)} \upsilon_\mathrm{F} + k_{\mathrm{QG}}^{(b)} \upsilon_\mathrm{G}} ;
$$

$$C_F = \frac{k_{HF}^{(a)}\upsilon_H + k_{IF}^{(a)}\upsilon_I + k_{SF}^{(a)}\upsilon_S + k_{QF}^{(a)}\upsilon_Q + k_{FG}^{(a)}\upsilon_G}{1 + k_{HF}^{(b)}\upsilon_H + k_{IF}^{(b)}\upsilon_I + k_{SF}^{(b)}\upsilon_S + k_{QF}^{(b)}\upsilon_Q + k_{FG}^{(b)}\upsilon_G};$$

$$C_G = \frac{k_{HG}^{(a)}\upsilon_H + k_{IG}^{(a)}\upsilon_I + k_{SG}^{(a)}\upsilon_S + k_{QG}^{(a)}\upsilon_Q + k_{FG}^{(a)}\upsilon_F}{1 + k_{HG}^{(b)}\upsilon_H + k_{IG}^{(b)}\upsilon_I + k_{SG}^{(b)}\upsilon_S + k_{QG}^{(b)}\upsilon_Q + k_{FG}^{(b)}\upsilon_F};$$

where, ON is the octane number of the mixed product, HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $\upsilon_i$ is content of each molecule in the mixed product, $\upsilon_H$, $\upsilon_I$, $\upsilon_S$, $\upsilon_Q$, $\upsilon_F$ and $\upsilon_G$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_H$ is an interaction coefficient of n-alkanes with other molecules, $C_I$ is an interaction coefficient of isoalkanes with other molecules; $C_S$ is an interaction coefficient of cycloalkanes with other molecules; $C_Q$ is an interaction coefficient of olefins with other molecules, $C_F$ is an interaction coefficient of aromatic hydrocarbons with other molecules, $C_G$ is an interaction coefficient of oxygenated compounds with other molecules, $k_{HI}^{(a)}$ is a first constant coefficient between n-alkanes and isoalkanes, $k_{HS}^{(a)}$ is a first constant coefficient between n-alkanes and cycloalkanes, $k_{HQ}^{(a)}$ is a first constant coefficient between n-alkanes and olefins, $k_{HF}^{(a)}$ is a first constant coefficient between n-alkanes and aromatic hydrocarbons, $k_{HG}^{(a)}$ is a first constant coefficient between n-alkanes and oxygenated compounds, $k_{IS}^{(a)}$ is a first constant coefficient between isoalkanes and cycloalkanes, $k_{IQ}^{(a)}$ is a first constant coefficient between isoalkanes and olefins, $k_{IF}^{(a)}$ is a first constant coefficient between isoalkanes and aromatic hydrocarbons, $k_{IG}^{(a)}$ is a first constant coefficient between isoalkanes and oxygenated compounds, $k_{SQ}^{(a)}$ is a first constant coefficient between cycloalkanes and olefins, $k_{SF}^{(a)}$ is a first constant coefficient between cycloalkanes and aromatic hydrocarbons, $k_{SG}^{(a)}$ is a first constant coefficient between cycloalkanes and oxygenated compounds, $k_{QF}^{(a)}$ is a first constant coefficient between olefins and aromatic hydrocarbons, $k_{QG}^{(a)}$ is a second constant coefficient between olefins and oxygenated compounds, $k_{FG}^{(a)}$ is a first constant coefficient between aromatic hydrocarbons and oxygenated compounds, $k_{HI}^{(b)}$ is a second constant coefficient between n-alkanes and isoalkanes, $k_{HS}^{(b)}$ is a second constant coefficient between n-alkanes and cycloalkanes, $k_{HQ}^{(b)}$ is a second constant coefficient between n-alkanes and olefins, $k_{HF}^{(b)}$ is a second constant coefficient between n-alkanes and aromatic hydrocarbons, $k_{HG}^{(b)}$ is a second constant coefficient between n-alkanes and oxygenated compounds, $k_{IS}^{(b)}$ is a second constant coefficient between isoalkanes and cycloalkanes, $k_{IQ}^{(b)}$ is a second constant coefficient between isoalkanes and olefins, $k_{IF}^{(b)}$ is a second constant coefficient between isoalkanes and aromatic hydrocarbons, $k_{IG}^{(b)}$ is a second constant coefficient between isoalkanes and oxygenated compounds, $k_{SQ}^{(b)}$ is a second constant coefficient between cycloalkanes and olefins, $k_{SF}^{(b)}$ is a

second constant coefficient between cycloalkanes and aromatic hydrocarbons, $k_{SG}^{(b)}$ ris a second constant coefficient between cycloalkanes and oxygenated compound, $k_{QF}^{(b)}$ is a second constant coefficient between olefins and aromatic hydrocarbons, $k_{QG}^{(b)}$ is a second constant coefficient between olefins and oxygenated compound, and $k_{FG}^{(b)}$ is a second constant coefficient between aromatic hydrocarbons and oxygenated compound; wherein the octane number comprises: a research octane number and a motor octane number.

22. The method according to claim 1, wherein, a step of training the product prediction model comprises:

establishing a product prediction model; wherein the product prediction model comprises: a set of reaction rules comprising a plurality of reaction rules and a reaction rate algorithm;
acquiring sample feedstock information for a sample feedstock;
training the set of reaction rules by using the sample feedstock information, and fixing the set of reaction rules that has been trained; and
training the reaction rate algorithm by using the sample feedstock information, and fixing the reaction rate algorithm that has been trained, to obtain the product prediction model that has been trained.

23. The method according to claim 22, wherein the sample feedstock information of the sample feedstock comprises: molecular composition of the sample feedstock, molecular content of each molecule in the sample feedstock, molecular composition of an actual product corresponding to the sample feedstock, and actual content of each molecule in the actual product.

24. The method according to claim 23, wherein the training the set of reaction rules by using the sample feedstock information comprises:

processing the molecular composition of the sample feedstock according to a preset set of reaction rules, to obtain a reaction pathway corresponding to each molecule in the molecular composition of the sample feedstock;
obtaining first molecule composition of a device output product comprising the sample feedstock, an intermediate product, and a predicted product according to the reaction path corresponding to each molecule in the molecular composition of the sample feedstock; in the device output product, comprising: the sample feedstock, the intermediate product, and the predicted product;
calculating a first relative deviation according to the first molecular composition of the device output product and second molecular composition of the actual product;
if the first relative deviation meets a preset condition, fixing the set of reaction rules; and
if the first relative deviation does not meet the preset condition, adjusting a reaction rule in the set of reaction rules, and recalculating the first relative deviation according to the adjusted set of reaction rules until the first relative deviation meets the preset condition.

25. The method according to claim 24, wherein the calculating a first relative deviation according to the first molecular composition of the device output product and second molecular composition of the actual product comprises:

acquiring species of single molecules in the first molecule composition, to constitute a first set;
acquiring species of single molecules in the second molecule composition, to constitute a second set;
determining whether the second set is a subset of the first set;
if the second set is not a subset of the first set, obtaining a pre-stored relative deviation value that does not meet the preset condition as the first relative deviation; and
if the second set is a subset of the first set, calculating the first relative deviation by a calculating formula as follows:

$$x_1 = \frac{\mathrm{card}\left((M - M_1 - M_2) - M_3\right)}{\mathrm{card}\ (M - M_1 - M_2)};$$

where, $x_1$ is the first relative deviation, $M$ is the first set, $M_1$ is a set of species of single molecules in the molecular composition of the sample feedstock, $M_2$ is a set of species of single molecules in the molecular composition of the

intermediate product, $M_3$ is the second set, and card is the number of elements in the sets.

26. The method according to claim 23, wherein the training the reaction rate algorithm by using the sample feedstock information comprises:

calculating a reaction rate of a reaction path corresponding to each molecule in the molecular composition of the sample feedstock, respectively, according to the reaction rate algorithm;
obtaining predicted content of each molecule in a predicted product corresponding to the sample feedstock according to molecular content of each molecule in the sample feedstock and the reaction rate of the reaction path corresponding to the molecule;
calculating a second relative deviation according to the predicted content of each molecule in the predicted product and the actual content of each molecule in the actual product;
if the second relative deviation meets a preset condition, fixing the reaction rate algorithm; and
if the second relative deviation does not meet the preset condition, adjusting a parameter in the reaction rate algorithm, and recalculating the second relative deviation according to the adjusted reaction rate algorithm until the second relative deviation meets the preset condition.

27. The method according to claim 26, wherein the calculating a reaction rate of a reaction path corresponding to each molecule in the molecular composition of the sample feedstock, respectively, according to the reaction rate algorithm comprises:

calculating a reaction rate of each reaction path according to a reaction rate constant in the reaction rate algorithm;
wherein the reaction rate constant is determined according to a calculation formula as follows:

$$ k = \frac{k_B E}{h} \exp(\frac{E\Delta S - \Delta E}{RE})\varphi \times P^\alpha ; $$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

28. The method according to any one of claims 1 to 27, wherein types of the pre-trained product prediction model corresponding to a petroleum processing device comprise:
a pre-trained product prediction model corresponding to a catalytic cracking unit, a pre-trained product prediction model corresponding to a delayed coking unit, a pre-trained product prediction model corresponding to a residue hydrotreating unit, a pre-trained product prediction model corresponding to a hydrocracking unit, a pre-trained product prediction model corresponding to a diesel hydro-upgrading unit, a pre-trained product prediction model corresponding to a diesel hydro-refining unit, a pre-trained product prediction model corresponding to a gasoline hydro-refining unit, a pre-trained product prediction model corresponding to a catalytic reforming unit and an alkylation unit; a pre-trained product prediction model corresponding to wherein each petroleum processing device corresponds to a set of reaction rules.

29. An apparatus for obtaining a production and processing scheme of petroleum processing, wherein the apparatus comprises:

a first acquisition unit configured to acquire molecular composition of crude oil;
a first processing unit configured to acquire molecular composition of various fractions obtained by distillation of the crude oil according to physical properties of various single molecules in the molecular composition of the crude oil;
a second processing unit configured to serve the corresponding fractions as petroleum processing feedstocks, and respectively input, according to a preset feedstock ratio, the the molecular composition of corresponding fractions into a pre-trained product prediction model corresponding to a petroleum processing device, to obtain a predicted molecular composition of a corresponding predicted product output by the pre-trained product prediction model and predicted molecular content of each single molecule in the predicted molecular composition;

a product blending unit configured to blend each of the predicted products which is used as a product blending feedstock according to a preset rule set, to obtain molecular composition of a plurality of mixed products and content of each single molecule in each of the mixed products, and respectively calculate a product property of each of the mixed products according to the molecular composition of each of the mixed products and the content of each single molecule in each of the mixed products, wherein each set of preset rules in the preset rule set includes the type and amount of the predicted product used;

a second acquisition unit configured to acquire a preset standard set for a preset target product; and

a third processing unit configured to determine whether the product property of each of the mixed products meets a preset product property of a target mixed product obtained by blending corresponding each target product in the preset standard set, and, if the preset product property is met:

obtain a target parameter according to all of the mixed products and determining whether the target parameter meets a preset condition;

if the target parameter meets the preset condition, determine that the predicted product meets a preset standard for a target product corresponding to the predicted product in the preset standard set, and output the preset feedstock ratio, the pre-trained product prediction model and the preset rule set as a production and processing scheme, and use the production and processing scheme to conduct petroleum processing in the actual production process;

if the target parameter does not meet the preset condition, adjust the operation parameter in the pre-trained product prediction model and a preset rule in the preset rule set, to re-obtain a plurality of mixed products, until the product property of each of the mixed products meets the preset product property and the target parameters in all of the mixed products meet the preset condition, wherein the target parameter represents: or a content of substances in the product that will cause harm to the environment; or a proportion of products, which meet a preset standard, in all mixed products to all of the mixed products, wherein the operation parameter in the pre-trained product prediction model comprises a temperature of an environment in which a reaction path in the pre-trained product prediction model is located, a pressure of an environment in which a reaction path in the pre-trained product prediction model is located, and space velocity.

30. A system for obtaining a production and processing scheme of petroleum processing, wherein the system for obtaining a production and processing scheme of petroleum processing comprises a processor and a memory, wherein the processor is configured to execute a real-time optimization program of the molecular level device stored in the memory to implement a computer-implemented method for obtaining a production and processing scheme of petroleum processing of any one of claims 1 to 28.

31. A computer-readable storage medium, wherein the computer-readable storage medium has stored therein one or more programs, the one or more programs being executable by one or more processors to implement a computer-implemented method for obtaining a production and processing scheme of petroleum processing of any one of claims 1 to 28.

**Patentansprüche**

1. Computerimplementiertes Verfahren für ein Erhalten eines Produktions- und Verarbeitungsschemas der Erdölverarbeitung, das Verfahren umfassend:

Erfassen einer molekularen Zusammensetzung von Rohöl;

Erfassen einer molekularen Zusammensetzung verschiedener Fraktionen, die durch Destillation des Rohöls erhalten werden, entsprechend den physikalischen Eigenschaften verschiedener Einzelmoleküle in der molekularen Zusammensetzung des Rohöls;

Bereitstellen der entsprechenden Fraktionen als Erdölverarbeitungs-Einsatzstoffe und jeweiliges Eingeben der molekularen Zusammensetzung der entsprechenden Fraktionen gemäß einem voreingestellten Einsatzstoffverhältnis in ein vortrainiertes Produktvorhersagemodell, das einer Erdölverarbeitungsvorrichtung entspricht, um eine vorhergesagte molekulare Zusammensetzung eines entsprechenden vorhergesagten Produkts, das von dem vortrainierten Produktvorhersagemodell ausgegeben wird, und einen vorhergesagten molekularen Gehalt jedes Einzelmoleküls in der vorhergesagten molekularen Zusammensetzung zu erhalten;

Mischen jedes der vorhergesagten Produkts, das als Produktvermischungseinsatzstoff gemäß einem voreingestellten Regelsatz verwendet wird, um eine molekulare Zusammensetzung einer Mehrzahl von Mischprodukten und den Gehalt jedes Einzelmoleküls in jedem der Mischprodukte zu erhalten, wobei jeder Satz von voreingestellten Regeln in dem voreingestellten Regelsatz die Art und die Menge des verwendeten vorhergesagten Produkts umfasst;

jeweiliges Berechnen einer Produkteigenschaft jedes der Mischprodukte gemäß der molekularen Zusammensetzung jedes der Mischprodukte und dem Gehalt jedes Einzelmoleküls in jedem der Mischprodukte;

Erfassen eines voreingestellten Standardsatzes für ein voreingestelltes Zielprodukt;

Bestimmen, ob die Produkteigenschaft jedes der Mischprodukte eine voreingestellte Produkteigenschaft eines Zielmischprodukts erfüllt, das durch Vermischen jedes entsprechenden Zielprodukts in dem voreingestellten Standardsatz erhalten wird;

wenn die voreingestellte Produkteigenschaft erfüllt ist:

Erhalten eines Zielparameters gemäß allen Mischprodukten und Bestimmen, ob der Zielparameter eine voreingestellte Bedingung erfüllt, wobei der Zielparameter repräsentiert: einen Gehalt an Substanzen in dem Produkt, die die Umwelt schädigen; oder einen Anteil von Produkten, die einen voreingestellten Standard erfüllen, in allen Mischprodukten zu allen Mischprodukten;

wenn der Zielparameter die voreingestellte Bedingung erfüllt, Bestimmen, dass das vorhergesagte Produkt einen voreingestellten Standard für ein Zielprodukt **erfüllt,** das dem vorhergesagten Produkt in dem voreingestellten Standardsatz entspricht, und Ausgeben des voreingestellten Einsatzstoffverhältnisses, des vortrainierten Produktvorhersagemodells und des voreingestellten Regelsatzes als ein Produktions- und Verarbeitungsschema, und Verwenden des Produktions- und Verarbeitungsschemas, um die Erdölverarbeitung in einem tatsächlichen Produktionsprozess durchzuführen; und

wenn der Zielparameter die voreingestellte Bedingung nicht erfüllt, Anpassen eines Betriebsparameters in dem vortrainierten Produktvorhersagemodell und einer voreingestellten Regel in dem voreingestellten Regelsatz, um eine Mehrzahl von Mischprodukten erneut zu erhalten, bis die Produkteigenschaft von jedem der Mischprodukte die voreingestellte Produkteigenschaft erfüllt und die Zielparameter in allen Mischprodukten die voreingestellte Bedingung erfüllen;

wobei der Betriebsparameter in dem vortrainierten Produktvorhersagemodell umfasst: eine Temperatur einer Umgebung, in der ein Reaktionsweg in dem vortrainierten Produktvorhersagemodell angeordnet ist, einen Druck einer Umgebung, in der ein Reaktionsweg in dem vortrainierten Produktvorhersagemodell angeordnet ist, und eine Raumgeschwindigkeit.

2. Verfahren nach Anspruch 1, wobei das Verfahren ferner umfasst:

Erfassen eines Eingangsstroms von Erdölverarbeitungs-Einsatzstoffen, die in jede der Erdölverarbeitungsvorrichtungen eingegeben werden;

Bestimmen, ob jeder der Eingangsströme einem voreingestellten Eingangsstrombereich der jeweiligen Erdölverarbeitungsvorrichtung entspricht; und

Anpassen des voreingestellten Einsatzstoffverhältnisses, wenn einer der Eingangsströme nicht dem voreingestellten Eingangsstrombereich der jeweiligen Erdölverarbeitungsvorrichtung entspricht, und jeweiliges erneutes Eingeben, gemäß dem angepassten voreingestellten Einsatzstoffverhältnis, der entsprechenden Fraktionen in das vortrainierte Produktvorhersagemodell der jeweiligen Erdölverarbeitungsvorrichtung als Erdölverarbeitungs-Einsatzstoffe, bis jeder der Eingangsströme dem voreingestellten Eingangsstrombereich der jeweiligen Erdölverarbeitungsvorrichtung entspricht.

3. Verfahren nach Anspruch 1, wobei das Anpassen eines Betriebsparameters in dem vortrainierten Produktvorhersagemodell umfasst:

Anpassen einer Temperatur einer Umgebung, in der ein Reaktionsweg angeordnet ist, der dem vorhergesagten Produkt in dem vortrainierten Produktvorhersagemodell entspricht; und

erneutes Erhalten der vorhergesagten molekularen Zusammensetzung des vorhergesagten Produkts und des vorhergesagten molekularen Gehalts jedes Einzelmoleküls in jedem der vorhergesagten Produkte gemäß der angepassten Temperatur.

4. Verfahren nach Anspruch 1, wobei das Anpassen eines Betriebsparameters in dem vortrainierten Produktvorhersagemodell umfasst:

Anpassen eines Drucks einer Umgebung, in der ein Reaktionsweg angeordnet ist, der dem vorhergesagten Produkt in dem vortrainierten Produktvorhersagemodell entspricht; und
erneutes Erhalten der vorhergesagten molekularen Zusammensetzung des vorhergesagten Produkts und des vorhergesagten molekularen Gehalts jedes Einzelmoleküls in jedem der vorhergesagten Produkte gemäß dem angepassten Druck.

5. Verfahren nach Anspruch 1, wobei das jeweilige Berechnen der Produkteigenschaft jedes der Mischprodukte gemäß der molekularen Zusammensetzung jedes der Mischprodukte und dem Gehalt jedes Einzelmoleküls in jedem der Mischprodukte umfasst:

Erfassen der ersten molekularen Zusammensetzung jedes Satzes der Produktvermischungseinsatzstoffe und des ersten Bestandteilgehalts jedes Einzelmoleküls in jedem Satz der Produktvermischungseinsatzstoffe;
basierend auf dem voreingestellten Regelsatz, Erhalten der zweiten molekularen Zusammensetzung jedes Mischprodukts und des zweiten Bestandteilgehalts jedes Einzelmoleküls in jedem Mischprodukt gemäß der ersten molekularen Zusammensetzung jedes Satzes der Produktvermischungseinsatzstoffe und des ersten Bestandteilgehalts jedes Einzelmoleküls in jedem Satz der Produktvermischungseinsatzstoffe;
Berechnen einer physikalischen Eigenschaft jedes Einzelmoleküls in jedem der Mischprodukte gemäß der Anzahl der Gruppen jeder Gruppe, die in jedem Einzelmolekül in jedem der Mischprodukte umfasst ist, und eines Beitragswertes jeder Gruppe zu der physikalischen Eigenschaft; und
Berechnen einer Produkteigenschaft für jedes der Mischprodukte gemäß der physikalischen Eigenschaft und des Bestandteilgehalts jedes Einzelmoleküls in jedem der Mischprodukte.

6. Verfahren nach Anspruch 5, wobei ein Berechnen der physikalischen Eigenschaft jedes Einzelmoleküls umfasst:

für jedes Einzelmolekül, Erfassen der Anzahl der Gruppen jeder Gruppe, die das Einzelmolekül bilden, und eines Beitragswertes jeder Gruppe zu der physikalischen Eigenschaft; und
Eingeben der Anzahl der Gruppen jeder Gruppe, die das Einzelmolekül bilden, und des Beitragswertes jeder Gruppe zu der physikalischen Eigenschaft in ein vortrainiertes Eigenschaftsberechnungsmodell, um die physikalische Eigenschaft des Einzelmoleküls zu erfassen, die durch das vortrainierte Eigenschaftsberechnungsmodell ausgegeben wird;
wobei das vortrainierte Eigenschaftsberechnungsmodell verwendet wird, um die physikalische Eigenschaft des Einzelmoleküls gemäß der Anzahl der Gruppen jeder Gruppe, die in einem Einzelmolekül umfasst ist, und einem Beitragswert jeder Gruppe zu der physikalischen Eigenschaft zu berechnen.

7. Verfahren nach Anspruch 6, wobei das Verfahren vor dem Eingeben der Anzahl der Gruppen jeder Gruppe, die das Einzelmolekül bilden, und des Beitragswertes jeder Gruppe zu der physikalischen Eigenschaft in ein vortrainiertes Eigenschaftsberechnungsmodell ferner umfasst:

Vergleich der Anzahl der Gruppen jeder Gruppe, die das Einzelmolekül bilden, mit molekularen Informationen eines Vorlage-Einzelmoleküls mit bekannten physikalischen Eigenschaften, die zuvor in einer Datenbank gespeichert wurden, wobei die molekularen Informationen die Anzahl der Gruppen jeder Gruppe umfassen, die das Vorlage-Einzelmolekül bilden;
Bestimmen, ob es ein gleiches Vorlage-Einzelmolekül wie das Einzelmolekül gibt;
wenn es ein gleiches Vorlage-Einzelmolekül wie das Einzelmolekül gibt, Ausgeben der physikalischen Eigenschaften des Vorlage-Einzelmoleküls als eine physikalische Eigenschaft des Einzelmoleküls; und
wenn es kein gleiches Vorlage-Einzelmolekül wie das Einzelmolekül gibt, dann Durchführung des Schritts des Eingebens der Anzahl von Gruppen jeder Gruppe, die das Einzelmolekül bilden, und des Beitragswerts jeder Gruppe zu der physikalischen Eigenschaft in ein vortrainiertes Eigenschaftsberechnungsmodell.

8. Verfahren nach Anspruch 6 oder 7, wobei ein Schritt des Trainierens des Eigenschaftsberechnungsmodells umfasst:

Konstruieren eines Eigenschaftsberechnungsmodells eines Einzelmoleküls;
Erfassen der Anzahl der Gruppen jeder Gruppe, die ein Probeneinzelmolekül bilden; wobei die physikalische Eigenschaft des Probeneinzelmoleküls bekannt ist;
Eingeben der Anzahl von Gruppen jeder Gruppe, die das Probeneinzelmolekül bilden, in das Eigenschaftsberechnungsmodell;

Erfassen einer vorhergesagten physikalischen Eigenschaft des Probeneinzelmoleküls, die von dem Eigenschaftsberechnungsmodell ausgegeben wird;

wenn ein Abweichungswert zwischen der vorhergesagten physikalischen Eigenschaft und der bekannten physikalischen Eigenschaft kleiner als ein voreingestellter Abweichungsschwellenwert ist, Bestimmen, dass das Eigenschaftsberechnungsmodell konvergiert, Erfassen eines Beitragswerts jeder Gruppe zu der physikalischen Eigenschaft in dem konvergierten Eigenschaftsberechnungsmodell, und Speichern des Beitragswerts der Gruppe zu der physikalischen Eigenschaft; und

wenn der Abweichungswert zwischen der vorhergesagten physikalischen Eigenschaft und der bekannten physikalischen Eigenschaft größer als oder gleich dem Abweichungsschwellenwert ist, Anpassen eines Beitragswerts jeder Gruppe zu der physikalischen Eigenschaft in dem Eigenschaftsberechnungsmodell, bis das Eigenschaftsberechnungsmodell konvergiert.

9. Verfahren nach Anspruch 8, wobei das Eigenschaftsberechnungsmodell wie nachstehend dargestellt aufgebaut ist:

$$f = a + \sum n_i \Delta f_i \quad ;$$

wobei $f$ die physikalische Eigenschaft des Einzelmoleküls ist, $n_i$ die Anzahl der Gruppen der $i$-ten Gruppe in dem Einzelmolekül ist, $\Delta f_i$ der Beitragswert der $i$-ten Gruppe in dem Einzelmolekül zu der physikalischen Eigenschaft ist und $a$ eine assoziierte Konstante ist.

10. Verfahren nach Anspruch 8, wobei das Erfassen der Anzahl der Gruppen jeder Gruppe, die ein Probeneinzelmolekül bilden, umfasst:

Bestimmen einer Primärgruppe, der Anzahl der Gruppen der Primärgruppe, einer Mehrstufengruppe und der Anzahl der Gruppen der Mehrstufengruppe in allen Gruppen des Einzelmoleküls;

Verwenden aller das Einzelmolekül bildenden Gruppen als Primärgruppe; und

Verwendend verschiedene Gruppen, die koexistieren und zu einer gleichen physikalischen Eigenschaft gemeinsam beitragen, als die Mehrstufengruppe, und Verwenden der Anzahl der verschiedenen Gruppen als eine Ebene der Mehrstufengruppe.

11. Verfahren nach Anspruch 10, wobei

das Eigenschaftsberechnungsmodell ist wie nachstehend dargestellt aufgebaut ist:

$$f = a + \sum_i m_{1i} \Delta f_{1i} + \sum_j m_{2j} \Delta f_{2j} \dots \dots + \sum_l m_{Nl} \Delta f_{Nl} \quad ;$$

wobei $f$ die physikalische Eigenschaft des Einzelmoleküls ist, $m_{1i}$ die Anzahl der Gruppen der $i$-ten Gruppe in der Primärgruppe ist, $\Delta f_{1i}$ der Beitragswert der $i$-ten Gruppe in der Primärgruppe zu der physikalischen Eigenschaft ist, $m_{2j}$ die Anzahl der Gruppen der $j$-ten Gruppe in einer Sekundärgruppe ist, $\Delta f_{2j}$ der Beitragswert der $j$-ten Gruppe in der sekundären Gruppe zu der physikalischen Eigenschaft ist, $m_{Nl}$ die Anzahl der Gruppen der $l$-ten Gruppe in einer N-Stufengruppe ist, $\Delta f_{Nl}$ der Beitragswert der $l$-ten Gruppe in der N-Stufengruppe zu der physikalischen Eigenschaft ist, $a$ eine zugehörige Konstante ist und $N$ eine positive ganze Zahl größer oder gleich 2 ist.

12. Verfahren nach Anspruch 7, wobei das Erfassen der Anzahl der Gruppen jeder Gruppe, die das Einzelmolekül bilden, umfasst:

Bestimmen einer Primärgruppe, der Anzahl der Gruppen der Primärgruppe, einer Mehrstufengruppe und der Anzahl der Gruppen der Mehrstufengruppe in allen Gruppen des Einzelmoleküls;

Verwenden aller das Einzelmolekül bildenden Gruppen als Primärgruppe; und

Verwendend verschiedene Gruppen, die koexistieren und zu einer gleichen physikalischen Eigenschaft gemeinsam beitragen, als die Mehrstufengruppe, und Verwenden der Anzahl der verschiedenen Gruppen als eine Ebene der Mehrstufengruppe.

13. Verfahren nach Anspruch 12, wobei

die physikalische Eigenschaft des Einzelmoleküls einen Siedepunkt eines Einzelmoleküls umfasst;

das Berechnen der physikalischen Eigenschaft des Einzelmoleküls umfasst: Berechnen des Siedepunkts des Einzelmoleküls gemäß einem Eigenschaftsberechnungsmodell wie folgt:

$$T = \frac{SOL \times GRUPPE_{11} + SOL \times GRUPPE_{12} + \ldots + SOL \times GRUPPE_{1N}}{(SOL \times \text{Numh})^d + b} + c \; ;$$

wobei **T** der Siedepunkt des Einzelmoleküls ist, **SOL** ein Einzelmolekülvektor ist, der gemäß der Anzahl der Gruppen jeder das Einzelmolekül bildenden Gruppe umgewandelt ist, **GRUPPE₁₁** ein erster Beitragswertvektor ist, der gemäß einem Beitragswert der Primärgruppe zu dem Siedepunkt umgewandelt ist, **GRUPPE₁₂** ein zweiter Beitragswertvektor ist, der gemäß einem Beitragswert der Sekundärgruppe zu dem Siedepunkt umgewandelt ist, **GRUPPE₁ₙ** ein N-ter Beitragswertvektor ist, der gemäß einem Beitragswert der N-Stufengruppe zu dem Siedepunkt umgewandelt ist, **Numh** die Anzahl der Atome außer dem Wasserstoffatom in dem Einzelmolekül ist, **d** eine erste voreingestellte Konstante ist, **b** eine zweite voreingestellte Konstante ist, **c** eine dritte voreingestellte Konstante ist und **N** eine positive ganze Zahl größer oder gleich 2 ist.

**14.** Verfahren nach Anspruch 12, wobei

die physikalische Eigenschaft des Einzelmoleküls eine Dichte eines Einzelmoleküls umfasst;
das Berechnen der physikalischen Eigenschaft des Einzelmoleküls umfasst:

Berechnen der Dichte des Einzelmoleküls gemäß einem Eigenschaftsberechnungsmodell wie folgt:

$$D = \frac{SOL \times GRUPPE_{21}}{(SOL \times GRUPPE_{22} + \ldots + SOL \times GRUPPE_{2N}) \times e} \; ;$$

wobei **D** die Dichte des Einzelmoleküls ist, **SOL** ein Einzelmolekülvektor ist, der gemäß der Anzahl der Gruppen jeder das Einzelmolekül bildenden Gruppe umgewandelt ist, **GRUPPE₂₁** ein N+1-ter Beitragswertvektor ist, der gemäß einem Beitragswert der Primärgruppe in die Dichte umgewandelt ist, **GRUPPE₂₂** ein N+2-ter Beitragswertvektor ist, der gemäß einem Beitragswert der Sekundärgruppe in die Dichte umgewandelt ist, **GRUPPE₂ₙ** ein 2N-ter Beitragswertvektor ist, der gemäß einem Beitragswert der N-Stufengruppe in die Dichte umgewandelt ist, **e** die vierte voreingestellte Konstante ist; und **N** eine positive ganze Zahl größer oder gleich 2 ist.

**15.** Verfahren nach Anspruch 12, wobei

die physikalische Eigenschaft des Einzelmoleküls eine Oktanzahl eines Einzelmoleküls umfasst;
das Berechnen der physikalischen Eigenschaft des Einzelmoleküls umfasst:

Berechnen der Oktanzahl des Einzelmoleküls gemäß einem Eigenschaftsberechnungsmodell wie folgt:

$$X = SOL \times GRUPPE_{31} + SOL \times GRUPPE_{32} + \ldots\ldots + SOL \times GRUPPE_{3N} + h;$$

wobei **X** die Oktanzahl des Einzelmoleküls ist, **SOL** ein Einzelmolekülvektor ist, der gemäß der Anzahl der Gruppen jeder das Einzelmolekül bildenden Gruppe umgewandelt ist, **GRUPPE₃₁** ein 2N+1-ter Beitragswertvektor ist, der gemäß einem Beitragswert der Primärgruppe zu der Oktanzahl umgewandelt ist, **GRUPPE₃₂** ein 2N+2-ter Beitragswertvektor ist, der gemäß einem Beitragswert der Sekundärgruppe zu der Oktanzahl umgewandelt ist, **GRUPPE₃ₙ** ein 3N-ter Beitragswertvektor ist, der gemäß einem Beitragswert der N-Stufengruppe zu der Oktanzahl umgewandelt ist; **N** ist eine positive ganze Zahl größer oder gleich 2; und **h** die fünfte voreingestellte Konstante ist.

**16.** Verfahren nach Anspruch 1, wobei
die Produkteigenschaft der Mischprodukte eine Dichte, einen Trübungspunkt, einen Stockpunkt, einen Anilinpunkt und eine Oktanzahl umfasst.

**17.** Verfahren nach Anspruch 16, wobei, wenn eine Produkteigenschaft des Mischprodukts die Dichte ist, ein Berechnen der Produkteigenschaft eines jeden der Mischprodukte umfasst:

Berechnen der Dichte jedes der Mischprodukte gemäß einer Berechnungsformel wie folgt:

$$Dichte = \sum(D_i \times x_{i\_Volumen});$$

wobei *Dichte* die Dichte des Mischprodukts ist, $D_i$ die Dichte des *i*-ten Einzelmoleküls ist und $x_{i\_Volumen}$ der zweite Bestandteilgehalt des *i*-ten Einzelmoleküls ist.

18. Verfahren nach Anspruch 16, wobei, wenn eine Produkteigenschaft des Mischprodukts der Trübungspunkt ist, ein Berechnen der Produkteigenschaft jedes der Mischprodukte umfasst:

   für jedes Mischprodukt, Berechnen eines Trübungspunkt-Beitragswertes jedes Einzelmoleküls gemäß der Dichte und dem Siedepunkt jedes Einzelmoleküls in dem Mischprodukt; und
   Berechnen des Trübungspunkts des Mischprodukts gemäß den Trübungspunkt-Beitragswerten aller Einzelmoleküle und dem Gehalt jedes Einzelmoleküls in dem Mischprodukt.

19. Verfahren nach Anspruch 16, wobei, wenn eine Produkteigenschaft des Mischprodukts der Stockpunkt ist, ein Berechnen der Produkteigenschaft jedes der Mischprodukte umfasst:

   für jedes Mischprodukt, Berechnen eines Stockpunkt-Beitragswertes jedes Einzelmoleküls gemäß der Dichte und dem Molekulargewicht jedes Einzelmoleküls in dem Mischprodukt; und
   Berechnen des Stockpunkts des Mischprodukts gemäß den Stockpunkt-Beitragswerten aller Einzelmoleküle und dem Gehalt jedes Einzelmoleküls in dem Mischprodukt.

20. Verfahren nach Anspruch 16, wobei, wenn eine Produkteigenschaft des Mischprodukts der Anilinpunkt ist, ein Berechnen der Produkteigenschaft jedes der Mischprodukte umfasst:

   für jedes Mischprodukt, Berechnen eines Anilinpunkt-Beitragswertes jedes Einzelmoleküls gemäß der Dichte und dem Siedepunkt jedes Einzelmoleküls in dem Mischprodukt; und
   Berechnen des Anilinpunkts des Mischprodukts gemäß den Anilinpunkt-Beitragswerten aller Einzelmoleküle und dem Gehalt jedes Einzelmoleküls in dem Mischprodukt.

21. Verfahren nach Anspruch 16, wobei, wenn eine Produkteigenschaft des Mischprodukts die Oktanzahl ist, ein Berechnen der Produkteigenschaft jedes der Mischprodukte umfasst:

   für jedes Mischprodukt, Erfassen der Oktanzahl jedes Einzelmoleküls und des Gehalts jedes Einzelmoleküls in dem Mischprodukt; und
   Berechnen der Oktanzahl der Mischprodukte gemäß einer Berechnungsformel wie folgt:

$$\begin{aligned}
\mathrm{ON} = \Big(&\sum_{i=\mathrm{HISQFG}} v_i\beta_i\mathrm{ON}_i + \mathrm{C_H}\sum_{i=\mathrm{H}} v_i\beta_i\mathrm{ON}_i + \mathrm{C_I}\sum_{i=\mathrm{I}} v_i\beta_i\mathrm{ON}_i + \\
&\mathrm{C_S}\sum_{i=\mathrm{S}} v_i\beta_i\mathrm{ON}_i + \mathrm{C_Q}\sum_{i=\mathrm{Q}} v_i\beta_i\mathrm{ON}_i + \mathrm{C_F}\sum_{i=\mathrm{F}} v_i\beta_i\mathrm{ON}_i + \mathrm{C_G}\sum_{i=\mathrm{G}} v_i\beta_i\mathrm{ON}_i\Big) \div \\
\Big(&\sum_{i=\mathrm{HISQFG}} v_i\beta_i + \mathrm{C_H}(\sum_{i=\mathrm{H}} v_i\beta_i - \sum_{i=\mathrm{H}} v_i) + \mathrm{C_I}(\sum_{i=\mathrm{I}} v_i\beta_i - \sum_{i=\mathrm{I}} v_i) + \\
&\mathrm{C_S}(\sum_{i=\mathrm{S}} v_i\beta_i - \sum_{i=\mathrm{S}} v_i) + \mathrm{C_Q}(\sum_{i=\mathrm{Q}} v_i\beta_i - \sum_{i=\mathrm{Q}} v_i) + \mathrm{C_F}(\sum_{i=\mathrm{F}} v_i\beta_i - \\
&\sum_{i=\mathrm{F}} v_i) + \mathrm{C_G}(\sum_{i=\mathrm{G}} v_i\beta_i - \sum_{i=\mathrm{G}} v_i)\Big) ;
\end{aligned}$$

$$\mathrm{C_H} = \frac{k_{\mathrm{HI}}^{(a)}v_\mathrm{I} + k_{\mathrm{HS}}^{(a)}v_\mathrm{S} + k_{\mathrm{HQ}}^{(a)}v_\mathrm{Q} + k_{\mathrm{HF}}^{(a)}v_\mathrm{F} + k_{\mathrm{HG}}^{(a)}v_\mathrm{G}}{1 + k_{\mathrm{HI}}^{(b)}v_\mathrm{I} + k_{\mathrm{HS}}^{(b)}v_\mathrm{S} + k_{\mathrm{HQ}}^{(b)}v_\mathrm{Q} + k_{\mathrm{HF}}^{(b)}v_\mathrm{F} + k_{\mathrm{HG}}^{(b)}v_\mathrm{G}} ;$$

$$\mathrm{C_I} = \frac{k_{\mathrm{HI}}^{(a)}v_\mathrm{H} + k_{\mathrm{IS}}^{(a)}v_\mathrm{S} + k_{\mathrm{IQ}}^{(a)}v_\mathrm{Q} + k_{\mathrm{IF}}^{(a)}v_\mathrm{F} + k_{\mathrm{IG}}^{(a)}v_\mathrm{G}}{1 + k_{\mathrm{HI}}^{(b)}v_\mathrm{H} + k_{\mathrm{IS}}^{(b)}v_\mathrm{S} + k_{\mathrm{IQ}}^{(b)}v_\mathrm{Q} + k_{\mathrm{IF}}^{(b)}v_\mathrm{F} + k_{\mathrm{IG}}^{(b)}v_\mathrm{G}} ;$$

$$c_S = \frac{k_{HS}^{(a)}v_H + k_{IS}^{(a)}v_I + k_{SQ}^{(a)}v_Q + k_{SF}^{(a)}v_F + k_{SG}^{(a)}v_G}{1 + k_{HS}^{(b)}v_H + k_{IS}^{(b)}v_I + k_{SQ}^{(b)}v_Q + k_{SF}^{(b)}v_F + k_{SG}^{(b)}v_G} \quad;$$

$$c_Q = \frac{k_{HQ}^{(a)}v_H + k_{IQ}^{(a)}v_I + k_{SQ}^{(a)}v_S + k_{QF}^{(a)}v_F + k_{QG}^{(a)}v_G}{1 + k_{HQ}^{(b)}v_H + k_{IQ}^{(b)}v_I + k_{SQ}^{(b)}v_S + k_{QF}^{(b)}v_F + k_{QG}^{(b)}v_G} \quad;$$

$$c_F = \frac{k_{HF}^{(a)}v_H + k_{IF}^{(a)}v_I + k_{SF}^{(a)}v_S + k_{QF}^{(a)}v_Q + k_{FG}^{(a)}v_G}{1 + k_{HF}^{(b)}v_H + k_{IF}^{(b)}v_I + k_{SF}^{(b)}v_S + k_{QF}^{(b)}v_Q + k_{FG}^{(b)}v_G} \quad;$$

$$c_G = \frac{k_{HG}^{(a)}v_H + k_{IG}^{(a)}v_I + k_{SG}^{(a)}v_S + k_{QG}^{(a)}v_Q + k_{FG}^{(a)}v_F}{1 + k_{HG}^{(b)}v_H + k_{IG}^{(b)}v_I + k_{SG}^{(b)}v_S + k_{QG}^{(b)}v_Q + k_{FG}^{(b)}v_F} \quad;$$

wobei **ON** die Oktanzahl des Mischprodukts ist, **HISQFG** eine Molekülsammlung ist, **H ein** Molekülsatz von n-Alkanen ist, **I** ein Molekülsatz von Isoalkanen ist, **S ein** Molekülsatz von Cycloalkanen ist, **Q** ein Molekülsatz von Olefinen ist, **F ein** Molekülsatz von aromatischen Kohlenwasserstoffen ist, **G** ein Molekülsatz von sauerstoffhaltigen Verbindungen ist, $v_i$ der Gehalt jedes Moleküls in dem Mischprodukt ist, $v_H$, $v_I$, $v_S$, $v_Q$, $v_F$ und $v_G$ der Gesamtgehalt an n-Alkanen, der Gesamtgehalt an Isoalkanen, der Gesamtgehalt an Cycloalkanen, der Gesamtgehalt an Olefinen, der Gesamtgehalt an aromatischen Kohlenwasserstoffen und/oder der Gesamtgehalt an sauerstoffhaltigen Verbindungen in dem Mischprodukt ist, $\beta_i$ ein Regressionsparameter jedes Moleküls in dem Mischprodukt ist, $ON_i$ eine Oktanzahl jedes Moleküls in dem Mischprodukt ist, $C_H$ ein Wechselwirkungskoeffizient von n-Alkanen mit anderen Molekülen ist, $C_I$ ein Wechselwirkungskoeffizient von Isoalkanen mit anderen Molekülen ist; $C_S$ ein Wechselwirkungskoeffizient von Cycloalkanen mit anderen Molekülen ist; $C_Q$ ein Wechselwirkungskoeffizient von Olefinen mit anderen Molekülen ist, $C_F$ ein Wechselwirkungskoeffizient von aromatischen Kohlenwasserstoffen mit anderen Molekülen ist, $C_G$ ein Wechselwirkungskoeffizient von sauerstoffhaltigen Verbindungen mit anderen Molekülen ist, $k_{HI}^{(a)}$ ein erster konstanter Koeffizient zwischen n-Alkanen und Isoalkanen ist, $k_{HS}^{(a)}$ ein erster konstanter Koeffizient zwischen n-Alkanen und Cycloalkanen ist, $k_{HQ}^{(a)}$ ein erster konstanter Koeffizient zwischen n-Alkanen und Olefinen ist, $k_{HF}^{(a)}$ ein erster konstanter Koeffizient zwischen n-Alkanen und aromatischen Kohlenwasserstoffen ist, $k_{HG}^{(a)}$ ein erster konstanter Koeffizient zwischen n-Alkanen und sauerstoffhaltigen Verbindungen ist, $k_{IS}^{(a)}$ ein erster konstanter Koeffizient zwischen Isoalkanen und Cycloalkanen ist, $k_{IQ}^{(a)}$ ein erster konstanter Koeffizient zwischen Isoalkanen und Olefinen ist, $k_{IF}^{(a)}$ ein erster konstanter Koeffizient zwischen Isoalkanen und aromatischen Kohlenwasserstoffen ist, $k_{IG}^{(a)}$ ein erster konstanter Koeffizient zwischen Isoalkanen und sauerstoffhaltigen Verbindungen ist, $k_{SQ}^{(a)}$ ein erster konstanter Koeffizient zwischen Cycloalkanen und Olefinen ist, $k_{SF}^{(a)}$ ein erster konstanter Koeffizient zwischen Cycloalkanen und aromatischen Kohlenwasserstoffen ist, $k_{SG}^{(a)}$ ein erster konstanter Koeffizient zwischen Cycloalkanen und sauerstoffhaltigen Verbindungen ist, $k_{QF}^{(a)}$ ein erster

konstanter Koeffizient zwischen Olefinen und aromatischen Kohlenwasserstoffen ist, $k_{QG}^{(a)}$ ein zweiter

konstanter Koeffizient zwischen Olefinen und sauerstoffhaltigen Verbindungen ist, $k_{FG}^{(a)}$ ein erster konstanter

Koeffizient zwischen aromatischen Kohlenwasserstoffen und sauerstoffhaltigen Verbindungen ist, $k_{HI}^{(b)}$ ein

zweiter konstanter Koeffizient zwischen n-Alkanen und Isoalkanen ist, $k_{HS}^{(b)}$ ein zweiter konstanter Koeffizient zwischen n-Alkanen und Cycloalkanen ist, $k_{HQ}^{(b)}$ ein zweiter konstanter Koeffizient zwischen n-Alkanen und Olefinen ist, $k_{HF}^{(b)}$ ein zweiter konstanter Koeffizient zwischen n-Alkanen und aromatischen Kohlenwassers-

toffen ist, $k_{HG}^{(b)}$ ein zweiter konstanter Koeffizient zwischen n-Alkanen und sauerstoffhaltigen Verbindungen

ist, $k_{IS}^{(b)}$ ein zweiter konstanter Koeffizient zwischen Isoalkanen und Cycloalkanen ist, $k_{IQ}^{(b)}$ ein zweiter

konstanter Koeffizient zwischen Isoalkanen und Olefinen ist, $k_{IF}^{(b)}$ ein zweiter konstanter Koeffizient zwischen Isoalkanen und aromatischen Kohlenwasserstoffen ist, $k_{IG}^{(b)}$ ein zweiter konstanter Koeffizient zwischen Isoalkanen und sauerstoffhaltigen Verbindungen ist, $k_{SQ}^{(b)}$ ein zweiter konstanter Koeffizient zwischen Cycloalkanen und Olefinen ist, $k_{SF}^{(b)}$ ein zweiter konstanter Koeffizient zwischen Cycloalkanen und aromatischen Kohlenwasserstoffen ist, $k_{SG}^{(b)}$ ein zweiter konstanter Koeffizient zwischen Cycloalkanen und sauerstoffhaltigen Verbindungen ist, $k_{QF}^{(b)}$ ein zweiter konstanter Koeffizient zwischen Olefinen und aromatischen Kohlenwasserstoffen ist, $k_{QG}^{(b)}$ ein zweiter konstanter Koeffizient zwischen Olefinen und sauerstoffhaltigen Verbindungen ist, und $k_{FG}^{(b)}$ ein zweiter konstanter Koeffizient zwischen aromatischen Kohlenwasserstoffen und sauerstoffhaltigen Verbindungen ist; wobei die Oktanzahl umfasst: eine Forschungsoktanzahl und eine Motoroktanzahl.

22. Verfahren nach Anspruch 1, wobei ein Schritt des Trainierens des Produktvorhersagemodells umfasst:

Erstellen eines Produktvorhersagemodells; wobei das Produktvorhersagemodell umfasst: einen Satz von Reaktionsregeln, der eine Mehrzahl von Reaktionsregeln und einen Reaktionsgeschwindigkeitsalgorithmus umfasst;
Erfassen von Probeneinsatzstoffinformationen für einen Probeneinsatzstoff;
Trainieren des Satzes von Reaktionsregeln unter Verwendung der Probeneinsatzstoffinformationen und Festlegen des Satzes von Reaktionsregeln, der trainiert wurde; und
Trainieren des Reaktionsgeschwindigkeitsalgorithmus unter Verwendung der Probeneinsatzstoffinformationen und Festlegen des trainierten Reaktionsgeschwindigkeitsalgorithmus, um das trainierte Produktvorhersagemodell zu erhalten.

23. Verfahren nach Anspruch 22, wobei die Probeneinsatzstoffinformationen des Probeneinsatzstoffs umfassen: molekulare Zusammensetzung des Probeneinsatzstoffs, molekularer Gehalt jedes Moleküls in dem Probeneinsatzstoff, molekulare Zusammensetzung eines tatsächlichen Produkts, das dem Probeneinsatzstoff entspricht, und tatsächlicher Gehalt jedes Moleküls in dem tatsächlichen Produkt.

24. Verfahren nach Anspruch 23, wobei das Trainieren des Satzes von Reaktionsregeln unter Verwendung der Probeneinsatzstoffinformationen umfasst:

Verarbeiten der molekularen Zusammensetzung des Probeneinsatzstoffs gemäß einem voreingestellten Satz von Reaktionsregeln, um einen Reaktionsweg zu erhalten, der jedem Molekül in der molekularen Zusammensetzung des Probeneinsatzstoffs entspricht;
Erhalten einer ersten molekularen Zusammensetzung eines den Probeneinsatzstoff, ein Zwischenprodukt und ein vorhergesagtes Produkt umfassenden Vorrichtungsausgangsprodukts gemäß dem Reaktionsweg, der jedem Molekül in der molekularen Zusammensetzung des Probeneinsatzstoffs entspricht; in dem Vorrichtungsausgabeprodukt, umfassend: den Probeneinsatzstoff, das Zwischenprodukt und das vorhergesagte Produkt;
Berechnen einer ersten relativen Abweichung gemäß der ersten molekularen Zusammensetzung des Vorrichtungsausgabeprodukts und der zweiten molekularen Zusammensetzung des tatsächlichen Produkts;
wenn die erste relative Abweichung eine voreingestellte Bedingung erfüllt, Festlegen des Satzes von Reaktionsregeln; und
wenn die erste relative Abweichung die voreingestellte Bedingung nicht erfüllt, Anpassen einer Reaktionsregel in dem Satz von Reaktionsregeln, und Neuberechnen der ersten relativen Abweichung gemäß dem angepassten Satz von Reaktionsregeln, bis die erste relative Abweichung die voreingestellte Bedingung erfüllt.

25. Verfahren nach Anspruch 24, wobei das Berechnen einer ersten relativen Abweichung gemäß der ersten molekularen Zusammensetzung des Vorrichtungsausgabeprodukts und der zweiten molekularen Zusammensetzung des tatsächlichen Produkts umfasst:

Erfassen von Spezies von Einzelmolekülen in der ersten molekularen Zusammensetzung, um einen ersten Satz zu bilden;
Erfassen von Spezies von Einzelmolekülen in der zweiten molekularen Zusammensetzung, um eine zweite Gruppe zu bilden;
Bestimmen, ob der zweite Satz eine Teilmenge des ersten Satzes ist;
wenn der zweite Satz keine Teilmenge des ersten Satzes ist, Erhalten eines vorgespeicherten relativen Abweichungswertes, der die voreingestellte Bedingung nicht erfüllt, als die erste relative Abweichung; und
wenn der zweite Satz eine Teilmenge des ersten Satzes ist, Berechnen der ersten relativen Abweichung durch eine Berechnungsformel wie folgt:

$$x_1 = \frac{card((M-M_1-M_2)-M_3)}{card(M-M_1-M_2)} \quad ;$$

wobei $x_1$ die erste relative Abweichung ist, $M$ der erste Satz ist, $M_1$ ein Satz von Spezies von Einzelmolekülen in der molekularen Zusammensetzung des Probeneinsatzstoffs ist, $M_2$ ein Satz von Spezies von Einzelmolekülen in der molekularen Zusammensetzung des Zwischenprodukts ist, $M_3$ der zweite Satz ist und card die Anzahl der Elemente in den Sätzen ist.

26. Verfahren nach Anspruch 23, wobei das Training des Reaktionsgeschwindigkeitsalgorithmus unter Verwendung der Probeneinsatzstoffinformationen umfasst:

Berechnen einer Reaktionsgeschwindigkeit eines Reaktionswegs entsprechend jedem Molekül in der molekularen Zusammensetzung des Probeneinsatzstoffs, jeweils gemäß dem Reaktionsgeschwindigkeitsalgorithmus;
Erhalten eines vorhergesagten Gehalts jedes Moleküls in einem vorhergesagten Produkt, das dem Probeneinsatzstoff entspricht, gemäß dem molekularen Gehalt jedes Moleküls in dem Probeneinsatzstoff und der Reaktionsgeschwindigkeit des dem Molekül entsprechenden Reaktionswegs;
Berechnen einer zweiten relativen Abweichung gemäß dem vorhergesagten Gehalt jedes Moleküls in dem vorhergesagten Produkt und dem tatsächlichen Gehalt jedes Moleküls in dem tatsächlichen Produkt;
wenn die zweite relative Abweichung eine voreingestellte Bedingung erfüllt, Festlegen des Reaktionsgeschwindigkeitsalgorithmus; und
wenn die zweite relative Abweichung die voreingestellte Bedingung nicht erfüllt, Anpassen eines Parameters in dem Reaktionsgeschwindigkeitsalgorithmus und Neuberechnen der zweiten relativen Abweichung gemäß dem angepassten Reaktionsgeschwindigkeitsalgorithmus, bis die zweite relative Abweichung die voreingestellte Bedingung erfüllt.

27. Verfahren nach Anspruch 26, wobei das Berechnen einer Reaktionsgeschwindigkeit eines Reaktionswegs, der jedem Molekül in der molekularen Zusammensetzung des Probeneinsatzstoffs entspricht, gemäß dem Reaktionsgeschwindigkeitsalgorithmus umfasst:

Berechnen einer Reaktionsgeschwindigkeit für jeden Reaktionsweg gemäß einer Reaktionsgeschwindigkeitskonstante in dem Reaktionsgeschwindigkeitsalgorithmus;
wobei die Reaktionsgeschwindigkeitskonstante gemäß einer Berechnungsformel wie folgt bestimmt wird:

$$k = \frac{k_B E}{h} \exp\left(\frac{E\Delta S - \Delta E}{RE}\right) \varphi \times P^\alpha \quad ;$$

wobei $k$ die Reaktionsgeschwindigkeitskonstante ist, $k_B$ die Boltzmann-Konstante ist, $h$ die Planck-Konstante ist, $R$ eine ideale Gaskonstante ist, $E$ ein Temperaturwert der Umgebung ist, in der der Reaktionsweg angeordnet ist, $\exp$ eine Exponentialfunktion mit Basis Naturkonstante ist, $\Delta S$ eine Entropieänderung vor und nach der Reaktion entsprechend der dem Reaktionsweg entsprechenden Reaktionsregel ist, $\Delta E$ eine Reaktionsenergiebarriere entsprechend der dem Reaktionsweg entsprechenden Reaktionsregel ist, $\varphi$ ein Katalysatoraktivitätsfaktor ist, $P$ ein Druckwert der Umgebung ist, in der der Reaktionsweg angeordnet ist, und $\alpha$ ein Druckeinflussfaktor entsprechend der dem Reaktionsweg entsprechenden Reaktionsregel ist.

28. Verfahren nach einem der Ansprüche 1 bis 27, wobei die Arten des vortrainierten Produktvorhersagemodells, die einer Erdölverarbeitungsvorrichtung entsprechen, umfassen:
ein vortrainiertes Produktvorhersagemodell, das einer katalytischen Krackanlage entspricht, ein vortrainiertes Produktvorhersagemodell, das einer verzögerten Verkokungsanlage entspricht, ein vortrainiertes Produktvorhersagemodell, das einer Rückstandshydrobehandlungsanlage entspricht, ein vortrainiertes Produktvorhersagemodell, das einer Hydrokrackanlage entspricht, ein vortrainiertes Produktvorhersagemodell, das einer Diesel-Hydro-Upgrading-Einheit entspricht, ein vortrainiertes Produktvorhersagemodell, das einer Diesel-Hydroraffinierungseinheit entspricht, ein vortrainiertes Produktvorhersagemodell, das einer Benzin-Hydroraffinierungseinheit entspricht, ein vortrainiertes Produktvorhersagemodell, das einer katalytischen Reformierungseinheit und einer Alkylierungseinheit entspricht ein vortrainiertes Produktvorhersagemodell, wobei jede Erdölverarbeitungsvorrichtung einem Satz von Reaktionsregeln entspricht.

29. Vorrichtung für ein Erhalten eines Produktions- und Verarbeitungsschemas der Erdölverarbeitung, wobei die Vorrichtung umfasst:

eine erste Erfassungseinheit, die dazu eingerichtet ist, die molekulare Zusammensetzung des Rohöls zu erfassen;
eine erste Verarbeitungseinheit, die dazu eingerichtet ist, die molekulare Zusammensetzung verschiedener durch Destillation des Rohöls erhaltener Fraktionen gemäß den physikalischen Eigenschaften verschiedener Einzelmoleküle in der molekularen Zusammensetzung des Rohöls zu erfassen;
eine zweite Verarbeitungseinheit, die dazu eingerichtet ist, die entsprechenden Fraktionen als Erdölverarbeitungs-Einsatzstoffe bereitzustellen und jeweils gemäß einem voreingestellten Einsatzstoffverhältnis die molekulare Zusammensetzung der entsprechenden Fraktionen in ein vortrainiertes Produktvorhersagemodell einzugeben, das einer Erdölverarbeitungsvorrichtung entspricht, um eine vorhergesagte molekulare Zusammensetzung eines entsprechenden vorhergesagten Produkts, das von dem vortrainierten Produktvorhersagemodell ausgegeben wird, und einen vorhergesagten molekularen Gehalt jedes Einzelmoleküls in der vorhergesagten molekularen Zusammensetzung zu erhalten;
eine Produktvermischungseinheit, die dazu eingerichtet ist, jedes der vorhergesagten Produkte, das als Produktvermischungseinsatzstoff verwendet wird, gemäß einem voreingestellten Regelsatz zu vermischen, um die molekulare Zusammensetzung einer Mehrzahl von Mischprodukten und den Gehalt jedes Einzelmoleküls in jedem der Mischprodukte zu erhalten, und jeweils eine Produkteigenschaft jedes der Mischprodukte gemäß der molekularen Zusammensetzung jedes der Mischprodukte und des Gehalts jedes Einzelmoleküls in jedem der Mischprodukte zu berechnen, wobei jeder Satz von voreingestellten Regeln in dem voreingestellten Regelsatz die Art und Menge des verwendeten vorhergesagten Produkts umfasst;
eine zweite Erfassungseinheit, die dazu eingerichtet ist, einen voreingestellten Standardsatz für ein voreingestelltes Zielprodukt zu erfassen; und

eine dritte Verarbeitungseinheit, die dazu eingerichtet, zu bestimmen, ob die Produkteigenschaft jedes der Mischprodukte einer voreingestellten Produkteigenschaft eines Zielmischprodukts entspricht, das durch Vermischen jedes Zielprodukts in dem voreingestellten Standardsatz erhalten wird, und, wenn die voreingestellte Produkteigenschaft erfüllt ist:

Ermitteln eines Zielparameters gemäß aller Mischprodukte und Bestimmen, ob der Zielparameter eine voreingestellte Bedingung erfüllt;

wenn der Zielparameter die voreingestellte Bedingung erfüllt, Bestimmen, dass das vorhergesagte Produkt einen voreingestellten Standard für ein Zielprodukt erfüllt, das dem vorhergesagten Produkt in dem voreingestellten Standardsatz entspricht, und Ausgeben des voreingestellten Einsatzstoffverhältnisses, des vortrainierten Produktvorhersagemodells und des voreingestellten Regelsatzes als ein Produktions- und Verarbeitungsschema, und Verwenden des Produktions- und Verarbeitungsschemas, um die Erdölverarbeitung in dem tatsächlichen Produktionsprozess durchzuführen;

wenn der Zielparameter die voreingestellte Bedingung nicht erfüllt, Anpassen des Betriebsparameters in dem vortrainierten Produktvorhersagemodell und einer voreingestellten Regel in dem voreingestellten Regelsatz, um erneut eine Mehrzahl von Mischprodukten zu erhalten, bis die Produkteigenschaft jedes der Mischprodukte die voreingestellte Produkteigenschaft erfüllt und die Zielparameter in allen Mischprodukten die voreingestellte Bedingung erfüllen, wobei der Zielparameter repräsentiert: oder einen Gehalt an Substanzen in dem Produkt, die die Umwelt schädigen; oder einen Anteil von Produkten, die einen voreingestellten Standard erfüllen, in allen Mischprodukten zu allen Mischprodukten, wobei der Betriebsparameter in dem vortrainierten Produktvorhersagemodell eine Temperatur einer Umgebung, in der ein Reaktionsweg in dem vortrainierten Produktvorhersagemodell angeordnet ist, einen Druck einer Umgebung, in der ein Reaktionsweg in dem vortrainierten Produktvorhersagemodell angeordnet ist und die Raumgeschwindigkeit umfasst.

30. System für ein Erhalten eines Produktions- und Verarbeitungsschemas der Erdölverarbeitung, wobei das System zum Erhalten eines Produktions- und Verarbeitungsschemas der Erdölverarbeitung einen Prozessor und einen Speicher umfasst, wobei der Prozessor dazu eingerichtet ist, ein Echtzeit-Optimierungsprogramm der Vorrichtung auf molekularer Ebene auszuführen, das in dem Speicher gespeichert ist, um ein computerimplementiertes Verfahren für ein Erhalten eines Produktions- und Verarbeitungsschemas der Erdölverarbeitung nach einem der Ansprüche 1 bis 28 zu implementieren.

31. Computerlesbares Speichermedium, wobei in dem computerlesbaren Speichermedium ein oder mehrere Programme gespeichert sind, das eine oder die mehreren Programme von einem oder mehreren Prozessoren ausführbar sind, um ein computerimplementiertes Verfahren für ein Erhalten eines Produktions- und Verarbeitungsschemas der Erdölverarbeitung nach einem der Ansprüche 1 bis 28 zu implementieren.

**Revendications**

1. Procédé mis en œuvre par ordinateur pour obtenir un schéma de production et de traitement du traitement du pétrole, dans lequel le procédé comprend :

l'acquisition de la composition moléculaire du pétrole brut ;

l'acquisition de la composition moléculaire de diverses fractions obtenues par distillation du pétrole brut selon les propriétés physiques de diverses molécules uniques dans la composition moléculaire du pétrole brut ;

l'alimentation des fractions correspondantes comme charges d'alimentation de traitement du pétrole, et la fourniture en entrée, respectivement, conformément à un rapport de charge d'alimentation prédéfini, de la composition moléculaire des fractions correspondantes dans un modèle de prédiction de produit pré-entraîné correspondant à un dispositif de traitement de pétrole, pour obtenir une composition moléculaire prédite d'un produit prédit correspondant fourni en sortie par le modèle de prédiction de produit pré-entraîné et une teneur moléculaire prédite de chaque molécule unique dans la composition moléculaire prédite ;

le mélange de chacun des produits prédits, qui est utilisé en tant que charge d'alimentation de mélange de produits selon un ensemble de règles prédéfinies, pour obtenir une composition moléculaire d'une pluralité de produits mixtes et une teneur de chaque molécule unique dans chacun des produits mixtes, dans lequel chaque ensemble de règles prédéfinies dans l'ensemble de règles prédéfinies comporte le type et la quantité du produit prédit utilisé ;

le calcul respectif d'une propriété de produit de chacun des produits mixtes selon la composition moléculaire de chacun des produits mixtes et la teneur de chaque molécule unique dans chacun des produits mixtes ;

l'acquisition d'un ensemble de normes prédéfini pour un produit cible prédéfini ;

le fait de déterminer si la propriété de produit de chacun des produits mixtes satisfait une propriété de produit prédéfinie d'un produit mixte cible obtenu en mélangeant chaque produit cible correspondant dans l'ensemble de normes prédéfini ;

si la propriété prédéfinie du produit est satisfaite :

l'obtention d'un paramètre cible selon tous les produits mixtes et le fait de déterminer si le paramètre cible satisfait une condition prédéfinie, dans laquelle le paramètre cible représente : une teneur en substances dans le produit qui provoquera des dommages à l'environnement ; ou une proportion de produits, qui satisfait une norme prédéfinie, dans tous les produits mixtes pour tous les produits mixtes ;

si le paramètre cible satisfait à la condition prédéfinie, la détermination du fait que le produit prédit satisfait à une norme prédéfinie pour un produit cible correspondant au produit prédit dans l'ensemble de normes prédéfini, et la fourniture en sortie du rapport de charge d'alimentation prédéfini, du modèle de prédiction de produit pré-entraîné et de l'ensemble de règles prédéfini sous forme d'un schéma de production et de traitement, et l'utilisation du schéma de production et de traitement pour mener un traitement de pétrole dans un processus de production réel ; et

si le paramètre cible ne satisfait pas à la condition prédéfinie le réglage **d'un** paramètre de fonctionnement dans le modèle de prédiction de produit pré-entraîné et d'une règle prédéfinie dans l'ensemble de règles prédéfinies, pour obtenir à nouveau une pluralité de produits mixtes jusqu'à ce que la propriété de produit de chacun des produits mixtes satisfasse à la propriété de produit prédéfinie et que les paramètres cibles dans tous les produits mixtes satisfassent à la condition prédéfinie ;

dans lequel le paramètre de fonctionnement dans le modèle de prédiction de produit pré-entraîné comprend : une température d'un environnement dans lequel une voie réactionnelle dans le modèle de prédiction de produit pré-entraîné est située, une pression d'un environnement dans lequel une voie réactionnelle dans le modèle de prédiction de produit pré-entraîné est située, et une vitesse spatiale.

2. Procédé selon la revendication 1, dans lequel le procédé comprend en outre :

l'acquisition d'un flux d'entrée de charges d'alimentation de traitement de pétrole fourni en entrée dans chacun des dispositifs de traitement de pétrole ;

le fait de déterminer si chacun des flux d'entrée satisfait une plage de flux d'entrée prédéfinie du dispositif de traitement de pétrole respectif ; et

le réglage du rapport de charge d'alimentation prédéfini si l'un quelconque des flux d'entrée ne satisfait pas la plage de flux d'entrée prédéfinie du dispositif de traitement de pétrole respectif, et respectivement la fourniture à nouveau en entrée, conformément au rapport de charge d'alimentation prédéfini réglé, des fractions correspondantes dans le modèle de prédiction de produit pré-entraîné du dispositif de traitement de pétrole respectif en tant que charges d'alimentation de traitement de pétrole, jusqu'à ce que chacun des flux d'entrée satisfasse à la plage de flux d'entrée prédéfinie du dispositif de traitement de pétrole respectif.

3. Procédé selon la revendication 1, dans lequel le réglage d'un paramètre de fonctionnement dans le modèle de prédiction de produit pré-entraîné comprend :

le réglage d'une température d'un environnement où une voie réactionnelle correspondant au produit prédit dans le modèle de prédiction de produit pré-entraîné est située ; et

l'obtention à nouveau de la composition moléculaire prédite du produit prédit et de la teneur moléculaire prédite de chaque molécule unique dans chacun des produits prédits selon la température réglée.

4. Procédé selon la revendication 1, dans lequel le réglage d'un paramètre de fonctionnement dans le modèle de prédiction de produit pré-entraîné comprend :

le réglage d'une pression d'un environnement où une voie réactionnelle correspondant au produit prédit dans le modèle de prédiction de produit pré-entraîné est située ; et

l'obtention à nouveau de la composition moléculaire prédite du produit prédit et de la teneur moléculaire prédite de chaque molécule unique dans chacun des produits prédits selon la pression réglée.

5. Procédé selon la revendication 1, dans lequel le calcul respectif d'une propriété de produit de chacun des produits mixtes selon la composition moléculaire de chacun des produits mixtes et la teneur de chaque molécule unique dans chacun des produits mixtes comprend :

l'acquisition de la première composition moléculaire de chaque ensemble des charges d'alimentation de mélange de produits et de la première teneur en composants de chaque molécule unique dans chaque ensemble des charges d'alimentation de mélange de produits ;

sur la base de l'ensemble de règles prédéfinies, l'obtention d'une deuxième composition moléculaire de chacun des produits mixtes et d'une deuxième teneur en composants de chaque molécule unique dans chacun des produits mixtes selon la première composition moléculaire de chaque ensemble des charges d'alimentation de mélange de produits et la première teneur en composant de chaque molécule unique dans chaque ensemble des charges d'alimentation de mélange de produits ;

le calcul d'une propriété physique de chaque molécule unique dans chacun des produits mixtes selon le nombre de groupes de chaque groupe contenu dans chaque molécule unique dans chacun des produits mixtes et une valeur de contribution de chaque groupe à la propriété physique ; et

le calcul d'une propriété de produit de chacun des produits mixtes selon la propriété physique et la teneur en deuxième composant de chaque molécule unique dans chacun des produits mixtes.

6. Procédé selon la revendication 5, dans lequel le calcul de la propriété physique de chaque molécule unique comprend :

pour chaque molécule unique, l'acquisition du nombre de groupes de chaque groupe constituant la molécule unique et d'une valeur de contribution de chaque groupe à la propriété physique ; et

la fourniture en entrée du nombre de groupes de chaque groupe constituant la molécule unique et la valeur de contribution de chaque groupe à la propriété physique dans un modèle de calcul de propriété pré-entraîné, pour acquérir la propriété physique de la molécule unique fournie en sortie par le modèle de calcul de propriété pré-entraîné ;

dans lequel le modèle de calcul de propriété pré-entraîné est utilisé pour calculer la propriété physique de la molécule unique selon le nombre de groupes de chaque groupe contenu dans une molécule unique et une valeur de contribution de chaque groupe à la propriété physique.

7. Procédé selon la revendication 6, dans lequel, avant la fourniture en entrée du nombre de groupes de chaque groupe constituant la molécule unique et de la valeur de contribution de chaque groupe à la propriété physique dans un modèle de calcul de propriété pré-entraîné, le procédé comprend en outre :

la comparaison du nombre de groupes de chaque groupe constituant la molécule unique avec des informations moléculaires d'une molécule unique matrice avec des propriétés physiques connues préalablement stockées dans une base de données, les informations moléculaires comprenant le nombre de groupes de chaque groupe constituant la molécule unique matrice ;

le fait de déterminer s'il y a une même molécule unique matrice comme molécule unique ;

s'il y a une même molécule unique matrice comme molécule unique, la fourniture en sortie des propriétés physiques de la molécule unique matrice en tant que propriété physique de la molécule unique ; et

s'il n'y a pas une même molécule unique matrice comme molécule unique, alors la réalisation de l'étape consistant à fournir en entrée le nombre de groupes de chaque groupe constituant la molécule unique et de la valeur de contribution de chaque groupe à la propriété physique dans un modèle de calcul de propriété pré-entraîné.

8. Procédé selon la revendication 6 ou 7, dans lequel une étape d'entraînement du modèle de calcul de propriété comprend :

la construction d'un modèle de calcul de propriété d'une molécule unique ;

l'acquisition du nombre de groupes de chaque groupe constituant une molécule unique échantillon ; dans lequel la propriété physique de la molécule unique échantillon est connue ;

la fourniture en entrée du nombre de groupes de chaque groupe constituant la molécule unique échantillon dans le modèle de calcul de propriété ;

l'acquisition d'une propriété physique prédite de la molécule unique d'échantillon fournie en sortie par le modèle de calcul de propriété ;

si une valeur d'écart entre la propriété physique prédite et la propriété physique qui est connue est inférieure à un seuil d'écart prédéfini, la détermination du fait que le modèle de calcul de propriété converge, l'acquisition d'une valeur de contribution de chaque groupe à la propriété physique dans le modèle de calcul de propriété qui converge, et le stockage de la valeur de contribution du groupe à la propriété physique ; et

si la valeur d'écart entre la propriété physique prédite et la propriété physique qui est connue est supérieure ou égale au seuil d'écart, le réglage d'une valeur de contribution de chaque groupe à la propriété physique dans le modèle de calcul de propriété jusqu'à ce que le modèle de calcul de propriété converge.

**9.** Procédé selon la revendication 8, dans lequel le modèle de calcul de propriété est établi comme indiqué ci-dessous :

$$f = a + \sum n_i \Delta f_i \quad ;$$

où, **f** est la propriété physique de la molécule unique, **$n_i$** est le nombre de groupes du **i**-ième groupe dans la molécule unique, $\Delta f_i$ est la valeur de contribution du **i**-ième groupe dans la molécule unique à la propriété physique, et **a** est une constante associée.

**10.** Procédé selon la revendication 8, dans lequel l'acquisition du nombre de groupes de chaque groupe constituant une molécule unique échantillon comprend :

la détermination d'un groupe primaire, du nombre de groupes du groupe primaire, d'un groupe à plusieurs étages et du nombre de groupes du groupe à plusieurs étages dans tous les groupes de la molécule unique ;

la prise de tous les groupes constituant la molécule unique comme étant le groupe primaire ; et

la prise de divers groupes qui coexistent et contribuent à une même propriété physique en commun comme étant le groupe à plusieurs étages, et la prise du nombre des divers groupes comme étant un niveau du groupe à plusieurs étages.

**11.** Procédé selon la revendication 10, dans lequel,

le modèle de calcul de propriété est établi comme indiqué ci-dessous :

$$f = a + \sum_i m_{1i} \Delta f_{1i} + \sum_j m_{2j} \Delta f_{2j} \ldots \ldots + \sum_l m_{Nl} \Delta f_{Nl} \quad ;$$

où, **f** est la propriété physique de la molécule unique, **$m_{1i}$** est le nombre de groupes du **i**-ième groupe dans le groupe primaire, $\Delta f_{1i}$ est la valeur de contribution du **i**-ième groupe dans le groupe primaire à la propriété physique, **$m_{2j}$** est le nombre de groupes du **j**-ième groupe dans un groupe secondaire, $\Delta f_{2}j$ est la valeur de contribution du **j**-ième groupe dans le groupe secondaire à la propriété physique, **$m_{Nl}$** est le nombre de groupes du **l**-ième groupe dans un groupe à N étages, $\Delta f_{Nl}$ est la valeur de contribution du **l**-ième groupe dans le groupe à N étages à la propriété physique, **a** est une constante associée, et **N** est un entier positif supérieur ou égal à 2.

**12.** Procédé selon la revendication 7, dans lequel l'acquisition du nombre de groupes de chaque groupe constituant la molécule unique comprend :

la détermination d'un groupe primaire, du nombre de groupes du groupe primaire, d'un groupe à plusieurs étages et du nombre de groupes du groupe à plusieurs étages dans tous les groupes de la molécule unique ;

la prise de tous les groupes constituant la molécule unique comme étant le groupe primaire ; et

la prise de divers groupes qui coexistent et contribuent à une même propriété physique en commun comme étant le groupe à plusieurs étages, et la prise du nombre des divers groupes comme étant un niveau du groupe à plusieurs étages.

**13.** Procédé selon la revendication 12, dans lequel,

la propriété physique de la molécule unique comprend un point d'ébullition d'une molécule unique ;

le calcul de la propriété physique de la molécule unique comprend : le calcul du point d'ébullition de la molécule unique selon un modèle de calcul de propriété comme suit :

$$T = \frac{SOL \times GROUPE_{11} + SOL \times GROUPE_{12} + \ldots + SOL \times GROUPE_{1N}}{(SOL \times N\text{umh})^d + b} + c \quad ;$$

où, **T** est le point d'ébullition de la molécule unique, **SOL** est un vecteur de molécule unique converti selon le nombre de groupes de chaque groupe constituant la molécule unique, $GROUPE_{11}$ est un premier vecteur de valeur de contribution converti selon une valeur de contribution du groupe primaire au point d'ébullition, $GROUPE_{12}$ est un deuxième vecteur de valeur de contribution converti selon une valeur de contribution du groupe secondaire au point d'ébullition, $GROUPE_{1N}$ est un N-ième vecteur de valeur de contribution converti selon une valeur de contribution du groupe à N étages au point d'ébullition, **Numh** est le nombre d'atomes autres que l'atome d'hydrogène dans la molécule unique, $d$ est une première constante prédéfinie, $b$ est une deuxième constante prédéfinie, $c$ est une troisième constante prédéfinie, et $N$ est un entier positif supérieur ou égal à 2.

**14.** Procédé selon la revendication 12, dans lequel,

la propriété physique de la molécule unique comprend une densité d'une molécule unique ;
le calcul de la propriété physique de la molécule unique comprend :

le calcul de la densité de la molécule unique selon un modèle de calcul de propriété comme suit :

$$D = \frac{SOL \times GROUPE_{21}}{(SOL \times GROUPE_{22} + \ldots + SOL \times GROUPE_{2N}) \times e} \quad ;$$

où, $D$ est la densité de la molécule unique, $SOL$ est un vecteur de molécule unique converti selon le nombre de groupes de chaque groupe constituant la molécule unique, $GROUPE_{21}$ est un N+1-ième vecteur de valeur de contribution converti selon une valeur de contribution du groupe primaire à la densité, $GROUPE_{22}$ est un N+2-ième vecteur de valeur de contribution converti selon une valeur de contribution du groupe secondaire à la densité, $GROUPE_{2N}$ est un 2N-ième vecteur de valeur de contribution converti selon une valeur de contribution du groupe à N étages à la densité, $e$ est la quatrième constante prédéfinie ; et $N$ est un entier positif supérieur ou égal à 2.

**15.** Procédé selon la revendication 12, dans lequel,

la propriété physique de la molécule unique comprend un indice d'octane d'une molécule unique ;
le calcul de la propriété physique de la molécule unique comprend :

le calcul de l'indice d'octane de la molécule unique selon un modèle de calcul de propriété comme suit :

$$X = SOL \times GROUPE_{31} + SOL \times GROUPE_{32} + \ldots + SOL \times GROUPE_{3N} + h ;$$

où, $X$ est l'indice d'octane de la molécule unique, $SOL$ est un vecteur de molécule unique converti selon le nombre de groupes de chaque groupe constituant la molécule unique, $GROUPE_{31}$ est un 2N+1-ième vecteur de valeur de contribution converti selon une valeur de contribution du groupe primaire à l'indice d'octane, $GROUPE_{32}$ est un 2N+2-ième vecteur de valeur de contribution converti selon une valeur de contribution du groupe secondaire à l'indice d'octane, $GROUPE_{3N}$ est un 3N-ième vecteur de valeur de contribution converti selon une valeur de contribution du groupe à N étages à l'indice d'octane, N est un entier positif supérieur ou égal à 2 ; et h est la cinquième constante prédéfinie.

**16.** Procédé selon la revendication 1, dans lequel,
la propriété de produit des produits mixtes comprend une densité, un point de trouble, un point d'écoulement, un point d'aniline et un indice d'octane.

**17.** Procédé selon la revendication 16, dans lequel, lorsqu'une propriété de produit du produit mixte est la densité, le calcul de la propriété de produit de chacun des produits mixtes comprend :

le calcul de la densité de chacun des produits mixtes selon une formule de calcul comme suit :

$$densité = \sum (D_i \times x_{i\_volume}) ;$$

où, **densité** est la densité du produit mixte, $D_i$ est la densité de la $i$-ième molécule unique, et $x_{i\_volume}$ est la teneur en deuxième composant de la $i$-ième molécule unique.

**18.** Procédé selon la revendication 16, dans lequel, lorsqu'une propriété de produit du produit mixte est le point de trouble, le calcul de la propriété de produit de chacun des produits mixtes comprend :

pour chaque produit mixte, le calcul d'une valeur de contribution de point de trouble de chaque molécule unique selon la densité et le point d'ébullition de chaque molécule unique dans le produit mixte ; et
le calcul du point de trouble du produit mixte selon les valeurs de contribution de point de trouble de toutes les molécules uniques et la teneur de chaque molécule unique dans le produit mixte.

**19.** Procédé selon la revendication 16, dans lequel, lorsqu'une propriété de produit du produit mixte est le point d'écoulement, le calcul de la propriété de produit de chacun des produits mixtes comprend :

pour chaque produit mixte, le calcul d'une valeur de contribution de point d'écoulement de chaque molécule unique selon la densité et le poids moléculaire de chaque molécule unique dans le produit mixte ; et
le calcul du point d'écoulement du produit mixte selon les valeurs de contribution de point d'écoulement de toutes les molécules uniques et la teneur de chaque molécule unique dans le produit mixte.

**20.** Procédé selon la revendication 16, dans lequel, lorsqu'une propriété de produit du produit mixte est le point d'aniline, le calcul de la propriété de produit de chacun des produits mixtes comprend :

pour chaque produit mixte, le calcul d'une valeur de contribution de point d'aniline de chaque molécule unique selon la densité et le point d'ébullition de chaque molécule unique dans le produit mixte ; et
le calcul du point d'aniline du produit mixte selon les valeurs de contribution de point d'aniline de toutes les molécules uniques et la teneur de chaque molécule unique dans le produit mixte.

**21.** Procédé selon la revendication 16, dans lequel, lorsqu'une propriété de produit du produit mixte est l'indice d'octane, le calcul de la propriété de produit de chacun des produits mixtes comprend :

pour chaque produit mixte, l'acquisition de l'indice d'octane de chaque molécule unique et de la teneur en chaque molécule unique dans le produit mixte ; et
le calcul de l'indice d'octane de chacun des produits mixtes selon une formule de calcul comme suit :

$$\mathrm{ON} = \left( \sum_{i=\mathrm{HISQFG}} v_i \beta_i \mathrm{ON}_i + \mathrm{C_H} \sum_{i=\mathrm{H}} v_i \beta_i \mathrm{ON}_i + \mathrm{C_I} \sum_{i=\mathrm{I}} v_i \beta_i \mathrm{ON}_i + \right.$$

$$\mathrm{C_S} \sum_{i=\mathrm{S}} v_i \beta_i \mathrm{ON}_i + \mathrm{C_Q} \sum_{i=\mathrm{Q}} v_i \beta_i \mathrm{ON}_i + \mathrm{C_F} \sum_{i=\mathrm{F}} v_i \beta_i \mathrm{ON}_i + \mathrm{C_G} \sum_{i=\mathrm{G}} v_i \beta_i \mathrm{ON}_i \left.\right) \div$$

$$\left( \sum_{i=\mathrm{HISQFG}} v_i \beta_i + \mathrm{C_H}(\sum_{i=\mathrm{H}} v_i \beta_i - \sum_{i=\mathrm{H}} v_i) + \mathrm{C_I}(\sum_{i=\mathrm{I}} v_i \beta_i - \sum_{i=\mathrm{I}} v_i) + \right.$$

$$\mathrm{C_S}(\sum_{i=\mathrm{S}} v_i \beta_i - \sum_{i=\mathrm{S}} v_i) + \mathrm{C_Q}\left(\sum_{i=\mathrm{Q}} v_i \beta_i - \sum_{i=\mathrm{Q}} v_i\right) + \mathrm{C_F}(\sum_{i=\mathrm{F}} v_i \beta_i -$$

$$\left. \sum_{i=\mathrm{F}} v_i) + \mathrm{C_G}(\sum_{i=\mathrm{G}} v_i \beta_i - \sum_{i=\mathrm{G}} v_i) \right) \ ;$$

$$c_{\mathrm{H}} = \frac{k_{\mathrm{HI}}^{(a)} v_{\mathrm{I}} + k_{\mathrm{HS}}^{(a)} v_{\mathrm{S}} + k_{\mathrm{HQ}}^{(a)} v_{\mathrm{Q}} + k_{\mathrm{HF}}^{(a)} v_{\mathrm{F}} + k_{\mathrm{HG}}^{(a)} v_{\mathrm{G}}}{1 + k_{\mathrm{HI}}^{(b)} v_{\mathrm{I}} + k_{\mathrm{HS}}^{(b)} v_{\mathrm{S}} + k_{\mathrm{HQ}}^{(b)} v_{\mathrm{Q}} + k_{\mathrm{HF}}^{(b)} v_{\mathrm{F}} + k_{\mathrm{HG}}^{(b)} v_{\mathrm{G}}} \ ;$$

$$c_{\mathrm{I}} = \frac{k_{\mathrm{HI}}^{(a)} v_{\mathrm{H}} + k_{\mathrm{IS}}^{(a)} v_{\mathrm{S}} + k_{\mathrm{IQ}}^{(a)} v_{\mathrm{Q}} + k_{\mathrm{IF}}^{(a)} v_{\mathrm{F}} + k_{\mathrm{IG}}^{(a)} v_{\mathrm{G}}}{1 + k_{\mathrm{HI}}^{(b)} v_{\mathrm{H}} + k_{\mathrm{IS}}^{(b)} v_{\mathrm{S}} + k_{\mathrm{IQ}}^{(b)} v_{\mathrm{Q}} + k_{\mathrm{IF}}^{(b)} v_{\mathrm{F}} + k_{\mathrm{IG}}^{(b)} v_{\mathrm{G}}} \ ;$$

$$c_{\mathrm{S}} = \frac{k_{\mathrm{HS}}^{(a)} v_{\mathrm{H}} + k_{\mathrm{IS}}^{(a)} v_{\mathrm{I}} + k_{\mathrm{SQ}}^{(a)} v_{\mathrm{Q}} + k_{\mathrm{SF}}^{(a)} v_{\mathrm{F}} + k_{\mathrm{SG}}^{(a)} v_{\mathrm{G}}}{1 + k_{\mathrm{HS}}^{(b)} v_{\mathrm{H}} + k_{\mathrm{IS}}^{(b)} v_{\mathrm{I}} + k_{\mathrm{SQ}}^{(b)} v_{\mathrm{Q}} + k_{\mathrm{SF}}^{(b)} v_{\mathrm{F}} + k_{\mathrm{SG}}^{(b)} v_{\mathrm{G}}} \ ;$$

$$c_Q = \frac{k_{HQ}^{(a)}v_H + k_{IQ}^{(a)}v_I + k_{SQ}^{(a)}v_S + k_{QF}^{(a)}v_F + k_{QG}^{(a)}v_G}{1 + k_{HQ}^{(b)}v_H + k_{IQ}^{(b)}v_I + k_{SQ}^{(b)}v_S + k_{QF}^{(b)}v_F + k_{QG}^{(b)}v_G} \quad ;$$

$$c_F = \frac{k_{HF}^{(a)}v_H + k_{IF}^{(a)}v_I + k_{SF}^{(a)}v_S + k_{QF}^{(a)}v_Q + k_{FG}^{(a)}v_G}{1 + k_{HF}^{(b)}v_H + k_{IF}^{(b)}v_I + k_{SF}^{(b)}v_S + k_{QF}^{(b)}v_Q + k_{FG}^{(b)}v_G} \quad ;$$

$$c_G = \frac{k_{HG}^{(a)}v_H + k_{IG}^{(a)}v_I + k_{SG}^{(a)}v_S + k_{QG}^{(a)}v_Q + k_{FG}^{(a)}v_F}{1 + k_{HG}^{(b)}v_H + k_{IG}^{(b)}v_I + k_{SG}^{(b)}v_S + k_{QG}^{(b)}v_Q + k_{FG}^{(b)}v_F} \quad ;$$

où, **ON** est l'indice d'octane du produit mixte, **HISQFG** est une collection moléculaire, **H** est un ensemble moléculaire de n-alcanes, **I** est un ensemble moléculaire d'isoalcanes, **S** est un ensemble moléculaire de cycloalcanes, **Q** est un ensemble moléculaire d'oléfines, **F** est un ensemble moléculaire d'hydrocarbures aromatiques, **G** est un ensemble moléculaire de composés oxygénés, $v_i$ est la teneur de chaque molécule dans le produit mixte, $v_H$, $v_I$, $v_S$, $v_Q$ $v_F$ et $v_G$ sont la teneur totale en n-alcanes, la teneur totale en isoalcanes, la teneur totale en cycloalcanes, la teneur totale en oléfines, la teneur totale en hydrocarbures aromatiques et la teneur totale d'un composé de composés oxygénés dans le produit mixte, respectivement, $\beta_i$ est un paramètre de régression de chaque molécule dans le produit mixte, $ON_i$ est un indice d'octane de chaque molécule dans le produit mixte, $C_H$ est un coefficient d'interaction des n-alcanes avec d'autres molécules, $C_I$ est un coefficient d'interaction des isoalcanes avec d'autres molécules ; $C_S$ est un coefficient d'interaction des cycloalcanes avec d'autres molécules ; $C_Q$ est un coefficient d'interaction des oléfines avec d'autres molécules, $C_F$ est un coefficient d'interaction des hydrocarbures aromatiques avec d'autres molécules, $C_G$ est un coefficient d'interaction des composés oxygénés avec d'autres molécules, $k_{HI}^{(a)}$ est un premier coefficient constant entre les n-alcanes et les isoalcanes, $k_{HS}^{(a)}$ est un premier coefficient constant entre les n-alcanes et les cycloalcanes, $k_{HQ}^{(a)}$ est un premier coefficient constant entre les n-alcanes et les oléfines, $k_{HF}^{(a)}$ est un premier coefficient constant entre les n-alcanes et les hydrocarbures aromatiques, $k_{HG}^{(a)}$ est un premier coefficient constant entre les n-alcanes et les composés oxygénés, $k_{IS}^{(a)}$ est un premier coefficient constant entre les isoalcanes et les cycloalcanes, $k_{IQ}^{(a)}$ est un premier coefficient constant entre les isoalcanes et les oléfines, $k_{IF}^{(a)}$ est un premier coefficient constant entre les isoalcanes et les hydrocarbures aromatiques, $k_{IG}^{(a)}$ est un premier coefficient constant entre les isoalcanes et les composés oxygénés, $k_{SQ}^{(a)}$ est un premier coefficient constant entre les cycloalcanes et les oléfines, $k_{SF}^{(a)}$ est un premier coefficient constant entre les cycloalcanes et les hydro-carbures aromatiques, $k_{SG}^{(a)}$ est un premier coefficient constant entre les cycloalcanes et les composés oxygénés, $k_{QF}^{(a)}$ est un premier coefficient constant entre les oléfines et les hydrocarbures aromatiques, $k_{QG}^{(a)}$ est un deuxième coefficient constant entre les oléfines et les composés oxygénés, $k_{FG}^{(a)}$ est un premier

coefficient constant entre les hydrocarbures aromatiques et les composés oxygénés, $k_{HI}^{(b)}$ est un deuxième coefficient constant entre les n-alcanes et les isoalcanes, $k_{HS}^{(b)}$ est un deuxième coefficient constant entre les n-alcanes et les cycloalcanes, $k_{HQ}^{(b)}$ est un deuxième coefficient constant entre les n-alcanes et les oléfines, $k_{HF}^{(b)}$ est un deuxième coefficient constant entre les n-alcanes et les hydrocarbures aromatiques, $k_{HG}^{(b)}$ est un deuxième coefficient constant entre les n-alcanes et les composés oxygénés, $k_{IS}^{(b)}$ est un deuxième coefficient constant entre les isoalcanes et les cycloalcanes, $k_{IQ}^{(b)}$ est un deuxième coefficient constant entre les isoalcanes et les oléfines, $k_{IF}^{(b)}$ est un deuxième coefficient constant entre les isoalcanes et les hydrocarbures aromatiques, $k_{IG}^{(b)}$ est un deuxième coefficient constant entre les isoalcanes et les composés oxygénés, $k_{SQ}^{(b)}$ est un deuxième coefficient constant entre les cycloalcanes et les oléfines, $k_{SF}^{(b)}$ est un deuxième coefficient constant entre les cycloalcanes et les hydrocarbures aromatiques, $k_{SG}^{(b)}$ est un deuxième coefficient constant entre les cycloalcanes et le composé oxygéné, $k_{QF}^{(b)}$ est un deuxième coefficient constant entre les oléfines et les hydrocarbures aromatiques, $k_{QG}^{(b)}$ est un deuxième coefficient constant entre les oléfines et le composé oxygéné, et $k_{FG}^{(b)}$ est un deuxième coefficient constant entre les hydrocarbures aromatiques et le composé oxygéné ; dans lequel l'indice d'octane comprend : un indice d'octane de recherche et un indice d'octane de moteur.

22. Procédé selon la revendication 1, dans lequel une étape d'entraînement du modèle de prédiction de produit comprend :

l'établissement d'un modèle de prédiction de produit ; dans lequel le modèle de prédiction de produit comprend : un ensemble de règles de réaction comprenant une pluralité de règles de réaction et un algorithme de vitesse de réaction ;
l'acquisition d'informations de charge d'alimentation d'échantillon pour une charge d'alimentation d'échantillon ;
l'entraînement de l'ensemble de règles de réaction en utilisant les informations de charge d'alimentation d'échantillon, et la correction de l'ensemble de règles de réaction qui a été entraîné ; et
l'entraînement de l'algorithme de vitesse de réaction en utilisant les informations de charge d'alimentation d'échantillon, et la fixation de l'algorithme de vitesse de réaction qui a été entraîné, pour obtenir le modèle de prédiction de produit qui a été entraîné.

23. Procédé selon la revendication 22, dans lequel les informations de charge d'alimentation d'échantillon de la charge d'alimentation d'échantillon comprennent : la composition moléculaire de la charge d'alimentation d'échantillon, la teneur moléculaire de chaque molécule dans la charge d'alimentation d'échantillon, la composition moléculaire d'un produit réel correspondant à la charge d'alimentation d'échantillon, et la teneur réelle de chaque molécule dans le produit réel.

24. Procédé selon la revendication 23, dans lequel l'entraînement de l'ensemble de règles de réaction en utilisant les informations de charge d'alimentation d'échantillon comprend :

le traitement de la composition moléculaire de la charge d'alimentation d'échantillon selon un ensemble prédéfini de règles de réaction, pour obtenir une voie de réaction correspondant à chaque molécule dans la composition moléculaire de la charge d'alimentation d'échantillon ;

l'obtention de la première composition moléculaire d'un produit de sortie de dispositif comprenant la charge d'alimentation d'échantillon, un produit intermédiaire, et un produit prédit selon la voie réactionnelle correspondant à chaque molécule dans la composition moléculaire de la charge d'alimentation d'échantillon ; dans le produit de sortie de dispositif, comprenant : la charge d'alimentation d'échantillon, le produit intermédiaire et le produit prédit ;

le calcul d'un premier écart relatif selon la première composition moléculaire du produit de sortie du dispositif et la deuxième composition moléculaire du produit réel ;

si le premier écart relatif satisfait une condition prédéfinie, la correction de l'ensemble de règles de réaction ; et

si le premier écart relatif ne satisfait pas à la condition prédéfinie, le réglage d'une règle de réaction dans l'ensemble de règles de réaction, et le nouveau calcul du premier écart relatif selon l'ensemble réglé de règles de réaction jusqu'à ce que le premier écart relatif satisfasse à la condition prédéfinie.

25. Procédé selon la revendication 24, dans lequel le calcul d'un premier écart relatif selon la première composition moléculaire du produit de sortie du dispositif et la deuxième composition moléculaire du produit réel comprend :

l'acquisition d'espèces de molécules uniques dans la première composition de molécules, pour constituer un premier ensemble ;

l'acquisition d'espèces de molécules uniques dans la deuxième composition de molécules, pour constituer un deuxième ensemble ;

le fait de déterminer si le deuxième ensemble est un sous-ensemble du premier ensemble ;

si le deuxième ensemble n'est pas un sous-ensemble du premier ensemble, l'obtention d'une valeur d'écart relatif préstockée qui ne satisfait pas la condition prédéfinie en tant que premier écart relatif ; et

si le deuxième ensemble est un sous-ensemble du premier ensemble, le calcul du premier écart relatif par une formule de calcul comme suit :

$$x_1 = \frac{card((M - M_1 - M_2) - M_3)}{card(M - M_1 - M_2)} \quad ;$$

où, $x_1$ est le premier écart relatif, $M$ est le premier ensemble, $M_1$ est un ensemble d'espèces de molécules uniques dans la composition moléculaire de la charge d'alimentation d'échantillon, $M_2$ est un ensemble d'espèces de molécules uniques dans la composition moléculaire du produit intermédiaire, $M_3$ est le deuxième ensemble, et **card** est le nombre d'éléments dans les ensembles.

26. Procédé selon la revendication 23, dans lequel l'entraînement de l'algorithme de vitesse de réaction en utilisant les informations de charge d'alimentation d'échantillon comprend :

le calcul d'une vitesse de réaction d'une voie réactionnelle correspondant à chaque molécule dans la composition moléculaire de la charge d'alimentation d'échantillon, respectivement, selon l'algorithme de vitesse de réaction ;

l'obtention d'une teneur prédite de chaque molécule dans un produit prédit correspondant à la charge d'alimentation d'échantillon selon la teneur moléculaire de chaque molécule dans la charge d'alimentation d'échantillon et la vitesse de réaction de la voie réactionnelle correspondant à la molécule ;

le calcul d'un deuxième écart relatif selon la teneur prédite de chaque molécule dans le produit prédit et la teneur réelle de chaque molécule dans le produit réel ;

si le deuxième écart relatif satisfait une condition prédéfinie, la correction de l'algorithme de vitesse de réaction ; et

si le deuxième écart relatif ne satisfait pas à la condition prédéfinie, le réglage d'un paramètre dans l'algorithme de vitesse de réaction, et le nouveau calcul du deuxième écart relatif selon l'algorithme de vitesse de réaction réglé jusqu'à ce que le deuxième écart relatif satisfasse à la condition prédéfinie.

27. Procédé selon la revendication 26, dans lequel le calcul d'une vitesse de réaction d'une voie réactionnelle correspondant à chaque molécule dans la composition moléculaire de la charge d'alimentation d'échantillon, respectivement, selon l'algorithme de vitesse de réaction comprend :

le calcul d'une vitesse de réaction de chaque voie réactionnelle selon une constante de vitesse de réaction dans l'algorithme de vitesse de réaction ;

dans lequel la constante de vitesse de réaction est déterminée selon une formule de calcul comme suit :

$$k = \frac{k_B E}{h} \exp\left(\frac{E\Delta S - \Delta E}{RE}\right)\varphi \times P^{\alpha} \quad ;$$

où, $k$ est la constante de vitesse de réaction, $k_B$ est la constante de Boltzmann, $h$ est la constante de Planck, $R$ est une constante des gaz parfaits, $E$ est une valeur de température de l'environnement dans lequel se trouve la voie réactionnelle, $exp$ est une fonction exponentielle à base de constante naturelle, $\Delta S$ est un changement d'entropie avant et après la réaction correspondant à la règle de réaction correspondant à la voie réactionnelle, $\Delta E$ est une barrière d'énergie de réaction correspondant à la règle de réaction correspondant à la voie réactionnelle, $\varphi$ est un facteur d'activité de catalyseur, $P$ est une valeur de pression de l'environnement dans lequel se trouve la voie réactionnelle, et $\alpha$ est un facteur d'influence de pression correspondant à la règle de réaction correspondant à la voie réactionnelle.

28. Procédé selon l'une quelconque des revendications 1 à 27, dans lequel les types du modèle de prédiction de produit pré-entraîné correspondant à un dispositif de traitement de pétrole comprennent :
un modèle de prédiction de produit pré-entraîné correspondant à une unité de craquage catalytique, un modèle de prédiction de produit pré-entraîné correspondant à une unité de cokéfaction retardée, un modèle de prédiction de produit pré-entraîné correspondant à une unité d'hydroraffinage de résidu, un modèle de prédiction de produit pré-entraîné correspondant à une unité d'hydrocraquage, un modèle de prédiction de produit pré-entraîné correspondant à une unité d'hydro-amélioration diesel, un modèle de prédiction de produit pré-entraîné correspondant à une unité de raffinage par hydrogénation de diesel, un modèle de prédiction de produit pré-entraîné correspondant à une unité de raffinage par hydrogénation d'essence, un modèle de prédiction de produit pré-entraîné correspondant à une unité de reformage catalytique et une unité d'alkylation ; un modèle de prédiction de produit pré-entraîné correspondant à dans lequel chaque dispositif de traitement de pétrole correspond à un ensemble de règles de réaction.

29. Appareil pour obtenir un schéma de production et de traitement de traitement de pétrole, dans lequel l'appareil comprend :

une première unité d'acquisition configurée pour acquérir une composition moléculaire de pétrole brut ;
une première unité de traitement configurée pour acquérir la composition moléculaire de diverses fractions obtenues par distillation du pétrole brut selon les propriétés physiques de diverses molécules uniques dans la composition moléculaire du pétrole brut ;
une deuxième unité de traitement configurée pour alimenter les fractions correspondantes comme charges d'alimentation de traitement du pétrole, et fournir en entrée, respectivement, conformément à un rapport de charge d'alimentation prédéfini, la composition moléculaire des fractions correspondantes dans un modèle de prédiction de produit pré-entraîné correspondant à un dispositif de traitement de pétrole, pour obtenir une composition moléculaire prédite d'un produit prédit correspondant fourni en sortie par le modèle de prédiction de produit pré-entraîné et une teneur moléculaire prédite de chaque molécule unique dans la composition moléculaire prédite ;
une unité de mélange de produits configurée pour mélanger chacun des produits prédits qui est utilisé comme une charge d'alimentation de mélange de produits selon un ensemble de règles prédéfinies, pour obtenir une composition moléculaire d'une pluralité de produits mixtes et une teneur de chaque molécule unique dans chacun des produits mixtes, et calculer respectivement une propriété de produit de chacun des produits mixtes selon la composition moléculaire de chacun des produits mixtes et la teneur de chaque molécule unique dans chacun des produits mixtes, dans lequel chaque ensemble de règles prédéfinies dans l'ensemble de règles prédéfinies comporte le type et la quantité du produit prédit utilisé ;
une deuxième unité d'acquisition configurée pour acquérir un ensemble de normes prédéfini pour un produit cible prédéfini ; et
une troisième unité de traitement configurée pour déterminer si la propriété de produit de chacun des produits mixtes satisfait une propriété de produit prédéfinie d'un produit mixte cible obtenu en mélangeant chaque produit cible correspondant dans l'ensemble de normes prédéfini, et, si la propriété de produit prédéfinie est satisfaite :

l'obtention d'un paramètre cible selon tous les produits mixtes et le fait de déterminer si le paramètre cible satisfait une condition prédéfinie ;
si le paramètre cible satisfait à la condition prédéfinie, la détermination du fait que le produit prédit satisfait à une norme prédéfinie pour un produit cible correspondant au produit prédit dans l'ensemble de normes prédéfini, et la fourniture en sortie du rapport de charge d'alimentation prédéfini, du modèle de prédiction de produit pré-entraîné et de l'ensemble de règles prédéfini sous forme d'un schéma de production et de traitement, et l'utilisation du schéma de production et de traitement pour mener un traitement de pétrole dans

EP 4 089 680 B1

le processus de production réel ;

si le paramètre cible ne satisfait pas la condition prédéfinie, le réglage du paramètre de fonctionnement dans le modèle de prédiction de produit pré-entraîné et d'une règle prédéfinie dans l'ensemble de règles prédéfini, pour obtenir à nouveau une pluralité de produits mixtes, jusqu'à ce que la propriété de produit de chacun des produits mixtes satisfasse la propriété de produit prédéfinie et que les paramètres cibles dans tous les produits mixtes satisfassent la condition prédéfinie, dans lequel le paramètre cible représente : ou une teneur en substances dans le produit qui provoqueront des dommages à l'environnement ; ou une proportion de produits, qui satisfont une norme prédéfinie, dans tous les produits mixtes à tous les produits mixtes, dans lequel le paramètre de fonctionnement dans le modèle de prédiction de produit pré-entraîné comprend une température d'un environnement dans lequel une voie réactionnelle dans le modèle de prédiction de produit pré-entraîné est située, une pression d'un environnement dans lequel une voie réactionnelle dans le modèle de prédiction de produit pré-entraîné est située, et la vitesse spatiale.

30. Système pour obtenir un schéma de production et de traitement de traitement de pétrole, dans lequel le système pour obtenir un schéma de production et de traitement de traitement de pétrole comprend un processeur et une mémoire, dans lequel le processeur est configuré pour exécuter un programme d'optimisation en temps réel du dispositif de niveau moléculaire stocké dans la mémoire pour mettre en œuvre un procédé mis en œuvre par ordinateur pour obtenir un schéma de production et de traitement de traitement de pétrole selon l'une quelconque des revendications 1 à 28.

31. Support de stockage lisible par ordinateur, dans lequel un ou plusieurs programmes sont stockés dans le support de stockage lisible par ordinateur, les un ou plusieurs programmes étant exécutables par un ou plusieurs processeurs pour mettre en œuvre un procédé mis en œuvre par ordinateur pour obtenir un schéma de production et de traitement de traitement de pétrole selon l'une quelconque des revendications 1 à 28.

68

molecular composition of petroleum processing feedstocks is determined ⌇— S101

the molecular composition of petroleum processing feedstocks is input into a pre-trained product prediction model corresponding to a petroleum processing device, to obtain predicted molecular composition of a corresponding predicted product output by the pre-trained product prediction model and predicted molecular content of each single molecule in the predicted molecular composition ⌇— S102

a preset standard set for a preset target product is acquired ⌇— S103

S104

it is determined whether the predicted product meets a preset standard for a target product corresponding to the predicted product in the preset standard set according to the predicted molecular composition of the predicted product and the predicted molecular content of each single molecule in the predicted molecular composition

yes → production is performed directly by utilizing the petroleum processing feedstocks ⌇— S105

no

an operation parameter in the pre-trained product prediction model is adjusted, to re-obtain predicted molecular composition of the predicted product and predicted molecular content of each single molecule in the predicted molecular composition, until the predicted product meets the preset standard for the target product corresponding to the predicted product in the preset standard set ⌇— S106

FIG. 1

| first acquisition unit | — | first processing unit | — | second processing unit |

11

12

13

| third processing unit | — | second acquisition unit |

15

14

FIG. 2

| processor 210 | — | memory 211 |

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LI J. et al.** Data-driven mathematical modeling and global optimization framework for entire petrochemical planning operations. *AICHE JOURNAL*, 2016, vol. 62 (9), 3020-3040
- **KHOR C. S. et al.** Petroleum refinery optimization. *OPTIMIZATION AND ENGINEERING*, 2016, vol. 18 (4), 943-989
- **GUEDDART T. et al.** Disaggregatio-naggregation based model reduction for refinery-wide optimization. *COMPUTERS & CHEMICAL ENGINEERING*, 2011, vol. 35 (9), 1838-1856
- **CHERNYSHEVA E. A. et al.** Enhancing the Efficiency of Refinery Crude Oil Distillation Process by Optimized Preliminary Feedstock Blending (Review). *PETROLEUM CHEMISTRY*, 2020, vol. 60 (1), 1-15
- **REN Y. et al.** Molecular reconstruction: Recent progress toward composition modeling of petroleum fractions. *CHEMICAL ENGENEERING JOURNAL*, 2018, vol. 357, 761-775